# EUROPEAN PATENT APPLICATION

(11) **EP 2 607 366 A1**
(43) Date of publication of application: **26.06.2013**
(21) Application number: 11010060.9
(22) Date of filing: 21.12.2011
(51) Int. Cl.: C07F 9/09, C07F 9/10, C07C 69/612, C07C 69/88, C07C 229/08, A61K 31/661, A61P 35/00, A61K 31/223, A61K 31/216, A61K 31/235

(54) **Glycerol phenyl butyrate esters**

(71) Applicant: Lunamed AG, 7000 Chur (CH)
(72) Inventor: Truog, Peter, 7000 Chur (CH); Buschmann, Helmut H., 52076 Aachen (Walheim) (DE)
(74) Representative: Peters, Hajo

(57) **Abstract**

The present invention refers to Glycerol Phenyl Butyrate Esters, including esters and amides, their stereoisomers, enantiomers, racemates as well as the acids, bases or salts thereof, medicaments comprising them, as well as their use in cancer therapy and other pharmaceutical applications. 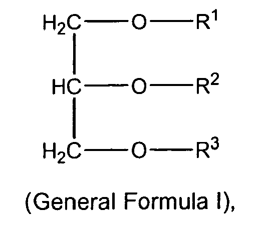 wherein
one of R¹, R² or R³ is

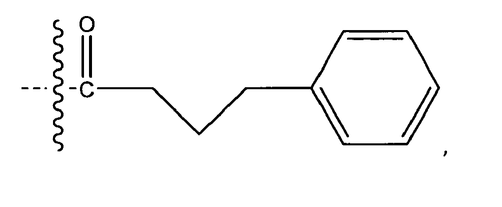

## Description

### Field of the invention

The present invention refers to Glycerol Phenyl Butyrate Esters, including esters and amides, their stereoisomers, enatiomers, racemates as well as the acids, bases or salts thereof, medicaments comprising them, as well as their use in cancer therapy and other pharmaceutical applications.

### Background of the invention

Phenylbutyric acid is a well known compound and is marketed in form of its sodium salt as a drug in the USA and European Union. Sodium phenylbutyrate is an orphan drug, marketed by Ucyclyd Pharma (Hunt Valley, USA) under the trade name Buphenyl and by Swedish Orphan International (Sweden) as Ammonaps for the treatment of urea cycle disorders.

In addition, 4-phenylbutyric acid sodium salt (sodium 4-phenylbutyrate) has also been described in patents and scientific literature in a number of medical uses. These uses encompass the treatment of a variety of illnesses, such as benign prostate hyperplasy, cancer, cystic fibrosis, HIV, kidney and liver failures, and thalasemia. For example, WO 93/0786, WO 9510271 or EP 725635 (Samid) disclose compositions and methods using phenylacetic acid derivatives for therapy and prevention of a number of pathologies, including cancer, AIDS, anemia, and severe beta-chain hemoglobinopathies.

One of the major drawbacks of 4-Phenylbutyric acid is that it is relatively fast metabolized in the human body to phenylacetic acid. This fact influences and challenges treatment schedules and thus leads to problems in applying a proper treatment with 4-Phenylbutyric acid and its salts.

Accordingly, it would be desirable to have a 4-phenylbutyrate derivative like Glycerol Phenyl Butyrate Esters that avoids the problems associated with application of 4-phenylbutyric acid or its salts. It would be even more preferable to find a 4-phenylbutyrate derivative whose activity is enhanced by an additional factor resting in the molecular structure of the derivative. Thus, it was the goal of this invention to identify alternative or improved derivatives of phenylbutyric acid.

It has now been found that certain selected Glycerol Phenyl Butyrate Esters as described below would solve this problem showing very advantageous attributes.

Accordingly, the invention is drawn to Glycerol Phenyl Butyrate Esters of General Formula I 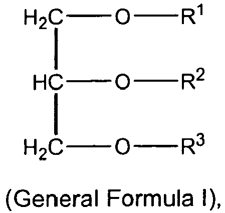 wherein
one of R¹, R² or R³ is 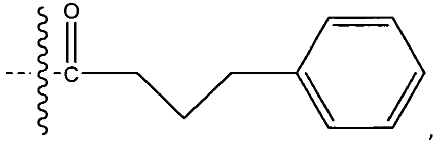 while the two others are independently of one another selected from
hydrogen, a radical of an amino acid, a radical of a D-amino acid, or any of the following structures, which may optionally be substituted by one or more of F, Cl, Br, 1, NH₂, OH, SH, CH₃, C₂H₅, C₃H₇, C₄H₉, CH₂Cl, CHCl₂, CF₃, CHF₂, CH₂F: 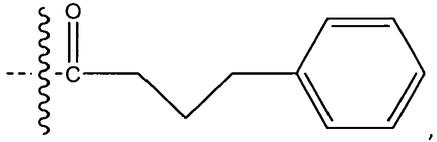 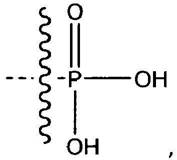 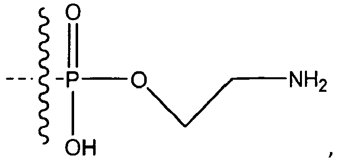 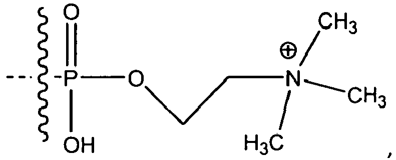 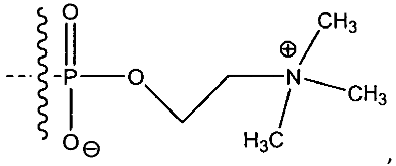 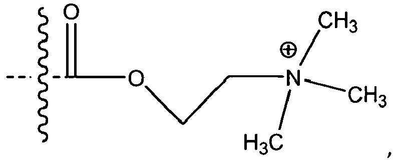 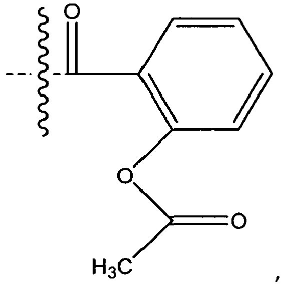 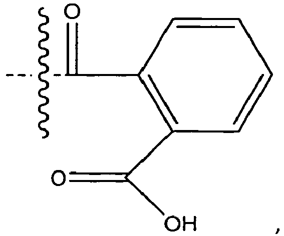 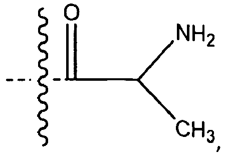 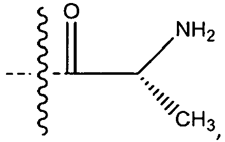 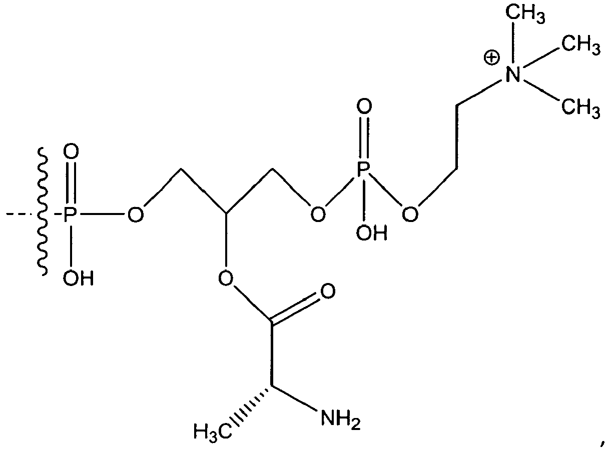 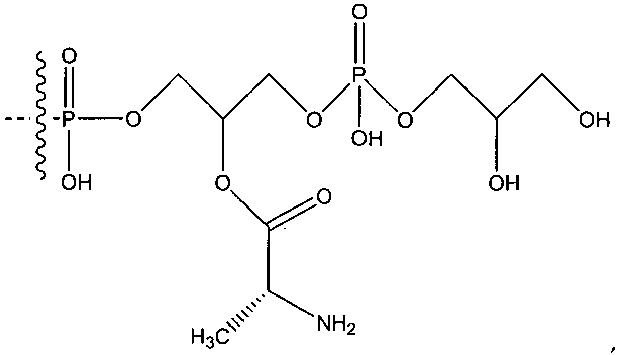 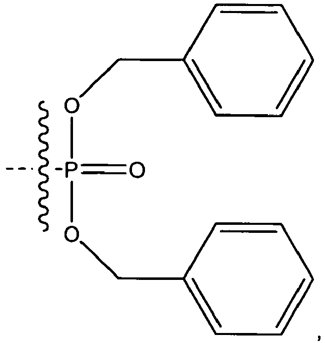 or 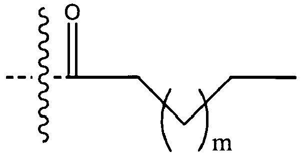 with m being 6, 7, 8, 9, 10, 11, 12, 13, 14, or 15, optionally unsaturated,
optionally in form of one of its stereoisomers, enantiomers or diastereomers, its racemate or in form of a mixture of at least two of its stereoisomers in any mixing ratio;
in form of the free acid, free base or salt.

In a preferred embodiment the proviso applies that not all three of R¹, R² and R³ may be

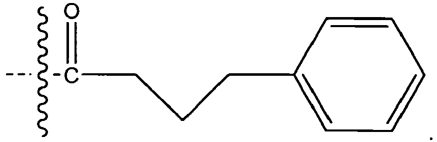

"Amino acid" according to the invention is defined as any of the naturally occurring amino acids as well as any synthetic amino acid, preferably is defined as naturally occurring amino acids. Accordingly "radical of an amino acid" is a radical/substituent derived from an amino acid as defined above.

Any use of "(D-Alanine)" in the description hereinafter is to indicate that the alanine substituent/radical in the respective compound is in the D-configuration. This also applies if used besides the formula of a compound example below. Thus, it also indicates that the alanine substituent/radical in the respective compound is in the D-configuration.

In an embodiment of the Glycerol Phenyl Butyrate Esters of General Formula I according to the invention
one of R¹, R² or R³ is 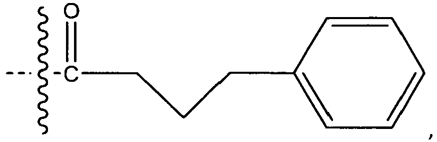 while the two others are independently of one another selected from hydrogen, 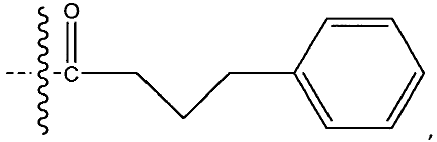 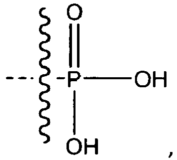 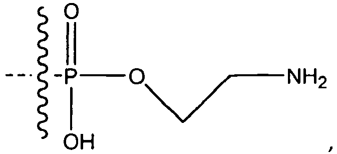 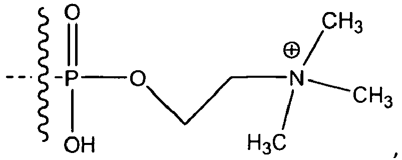 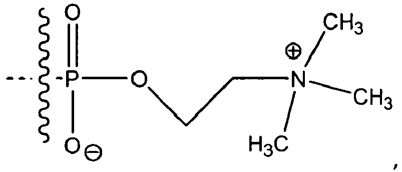 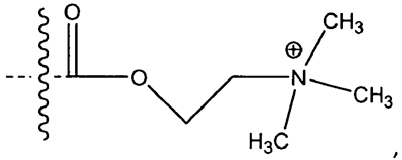 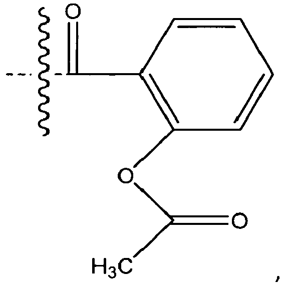 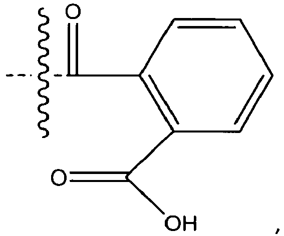 a radical of an amino acid, 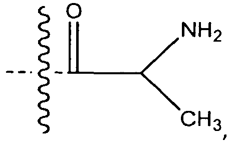 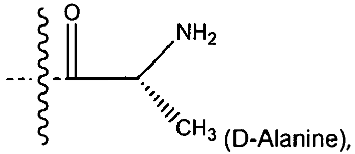 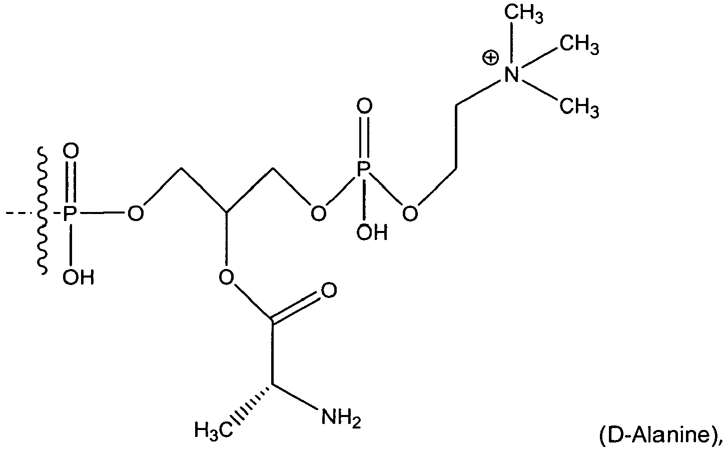 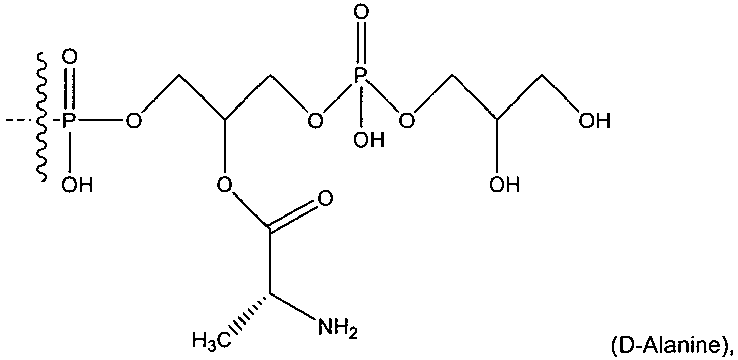 or 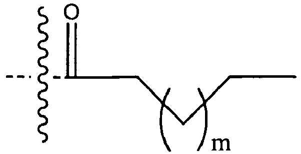 with m being 6, 7, 8, 9, 10. 11, 12, 13, 14, or 15, optionally unsaturated.

In another embodiment of the Glycerol Phenyl Butyrate Esters of General Formula I according to the invention,
one of R¹, R² or R³ is 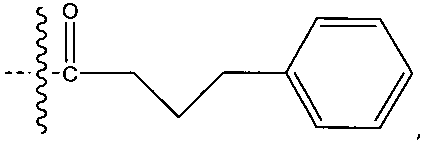 while the two others are independently of one another selected from hydrogen, 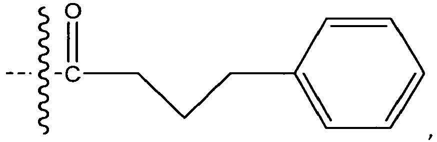 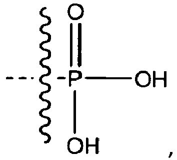 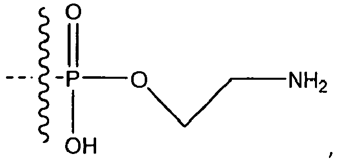 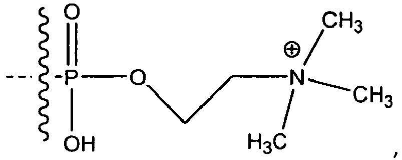 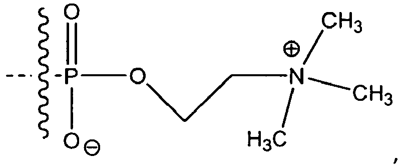 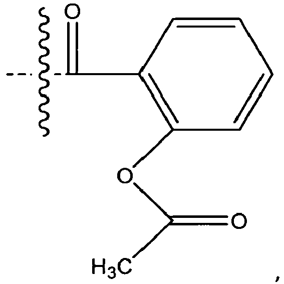 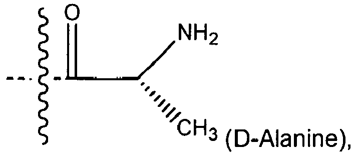 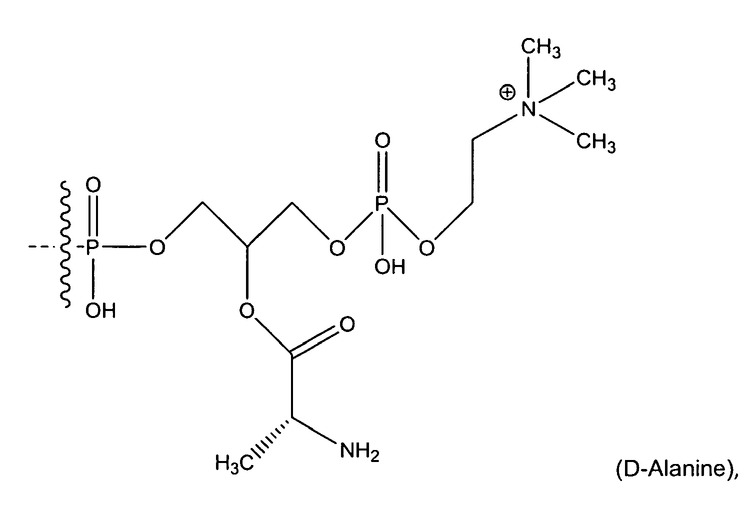 or

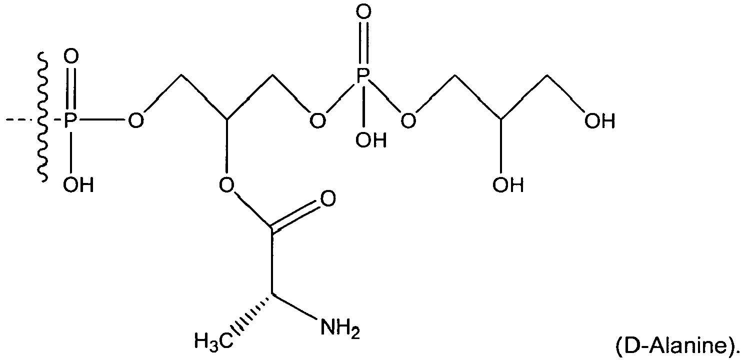

In another embodiment of the Glycerol Phenyl Butyrate Esters of General Formula I according to the invention

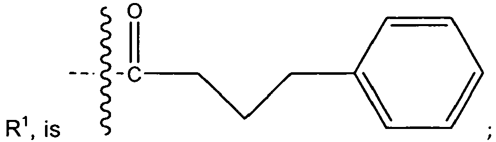

R² and R³ are independently of one another selected from hydrogen or any of the following structures, which may optionally be substituted by one or more of F, Cl, Br, 1, NH₂, OH, SH, CH₃, C₂H₅, C₃H₇, C₄H₉, CH₂Cl, CHCl₂, CF₃, CHF₂, CH₂F: 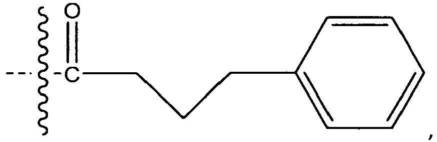 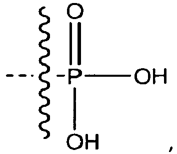 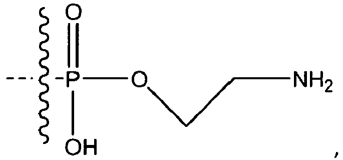 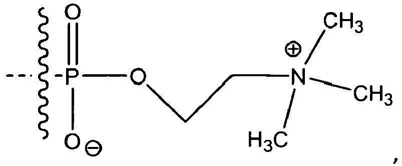 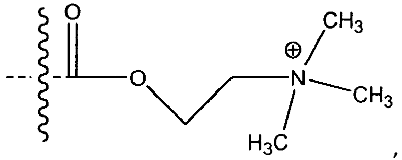 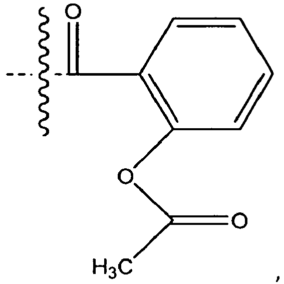 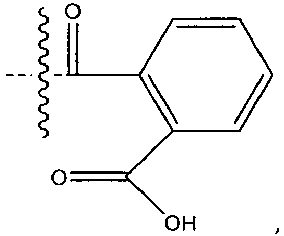 a radical of an amino acid, 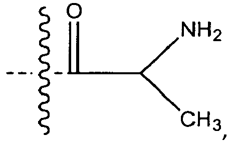 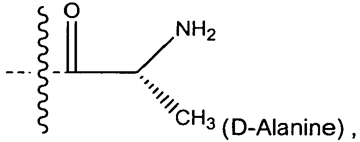 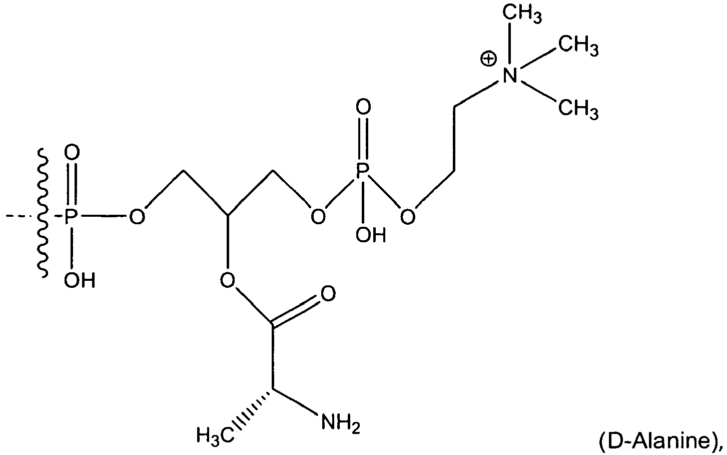 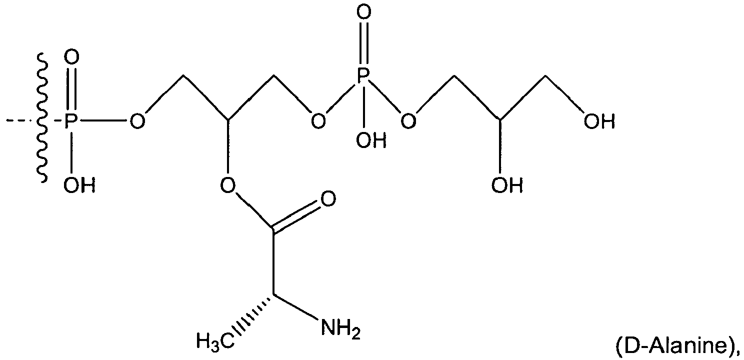 or 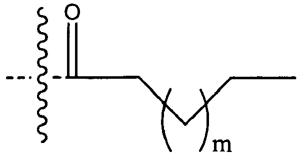 with m being 6, 7, 8, 9, 10, 11, 12, 13, 14, or 15, optionally unsaturated.

In another embodiment of the Glycerol Phenyl Butyrate Esters of General Formula I according to the invention two of R¹, R² and R³ are 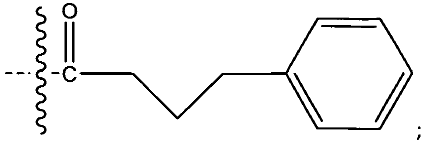 and the remaining is selected from
hydrogen or any of the following structures, which may optionally be substituted by one or more of F, Cl, Br, I, NH₂, OH, SH, CH₃, C₂H₅, C₃H₇, C₄H₉, CH₂Cl, CHCl₂, CF₃, CHF₂, CH₂F: 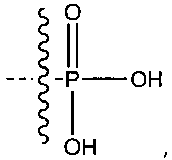 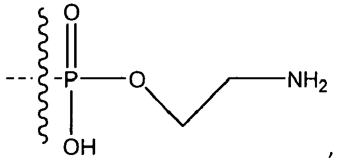 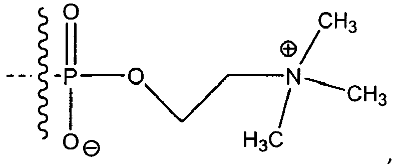 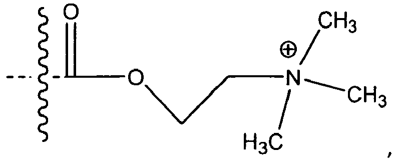 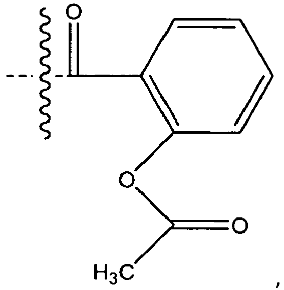 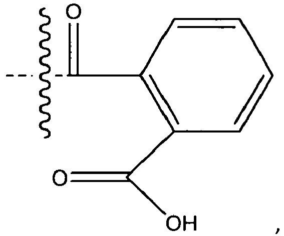 a radical of an amino acid, 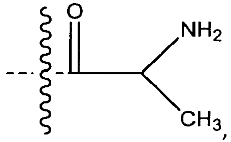 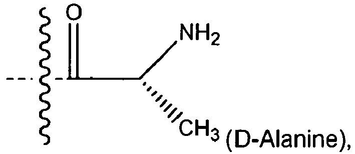 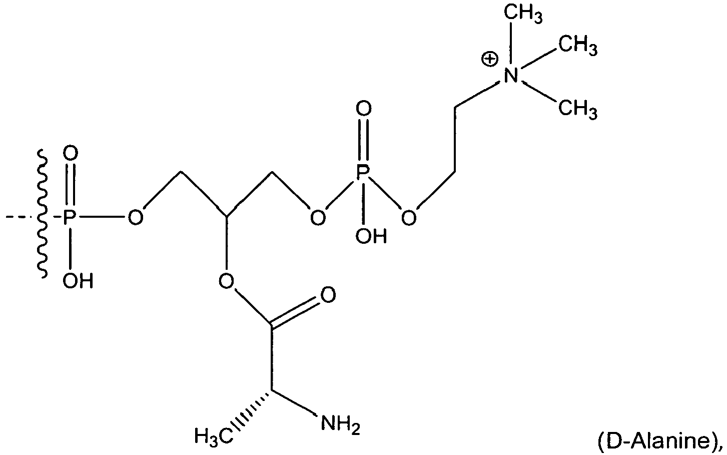 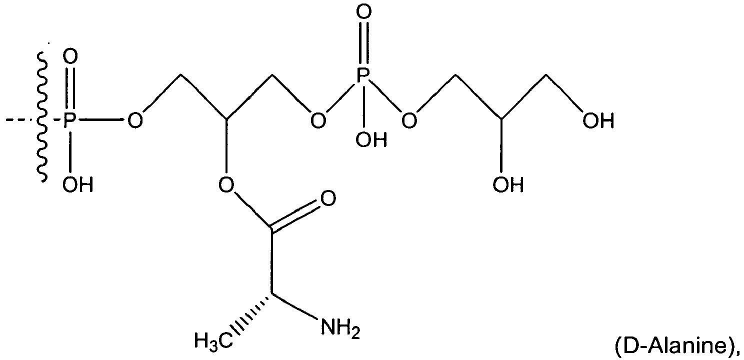 or 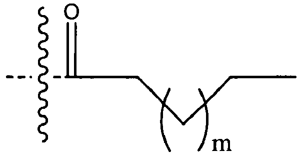 with m being 6, 7, 8, 9, 10, 11, 12, 13, 14, or 15, optionally unsaturated.

In another embodiment of the Glycerol Phenyl Butyrate Esters of General Formula I according to the invention
R¹, is 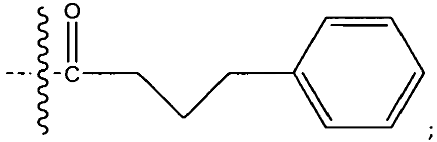 R² and R³ are independently of one another selected from
hydrogen or any of the following structures, which may optionally be substituted by one or more of F, Cl, Br, I, NH₂, OH, SH, CH₃, C₂H₅, C₃H₇, C₄H₉, CH₂Cl, CHCl₂, CF₃, CHF₂, CH₂F: 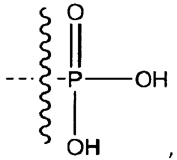 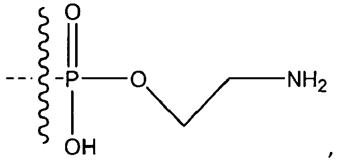 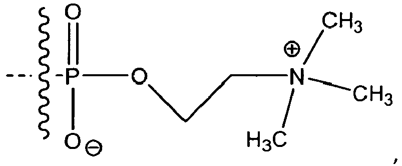 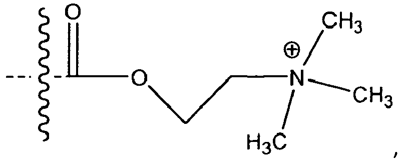 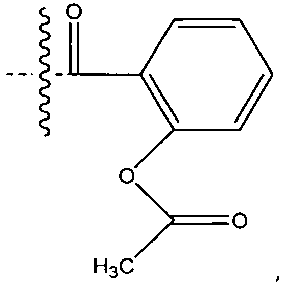 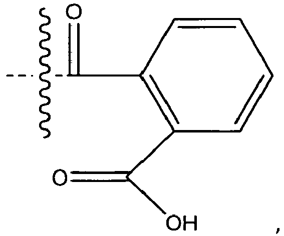 a radical of an amino acid, 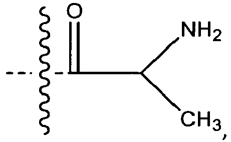 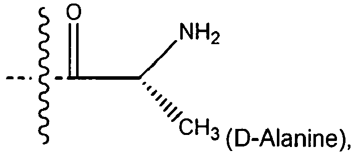 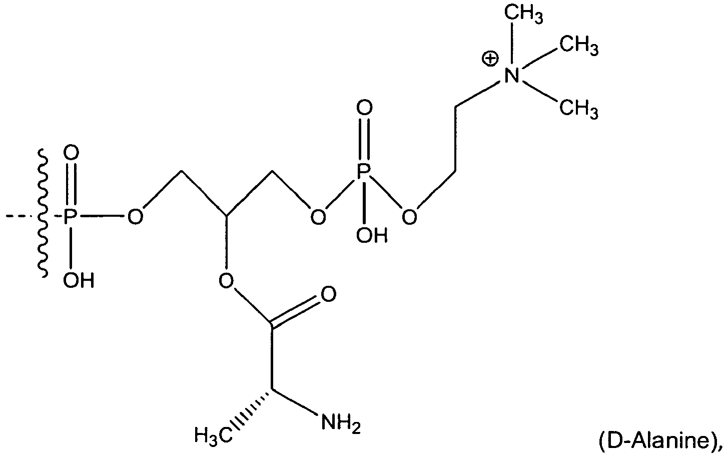 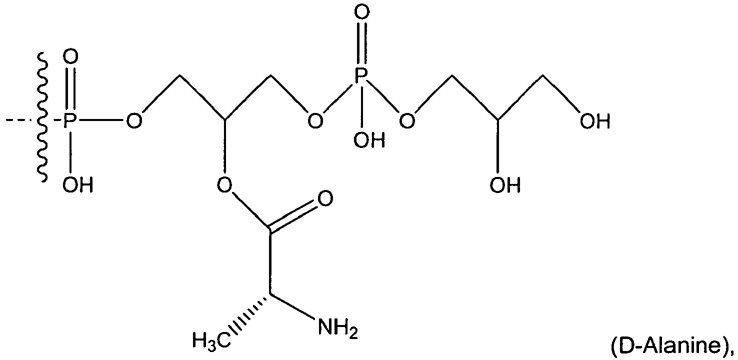 or 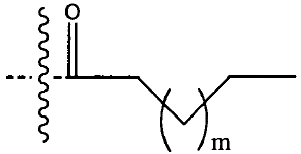 with m being 6, 7, 8, 9, 10, 11, 12, 13, 14, or 15, optionally unsaturated.

In another embodiment of the Glycerol Phenyl Butyrate Esters of General Formula I according to the invention
R² is selected from
hydrogen or any of the following structures: 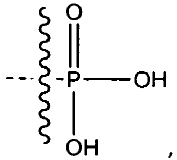 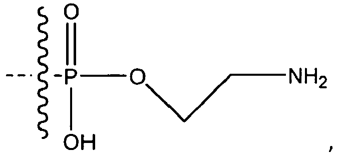 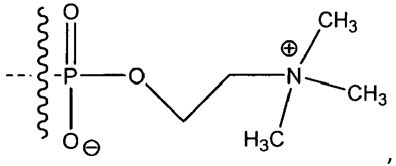 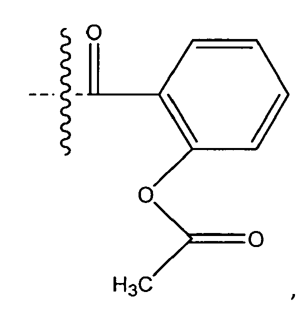 or 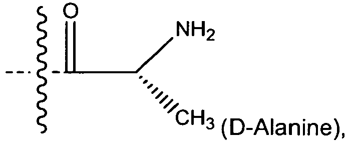 and
R³ is selected from 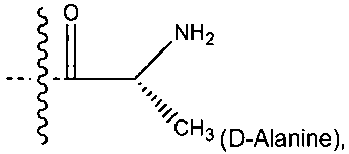 Preferably wherein
R² is hydrogen
and R³ is 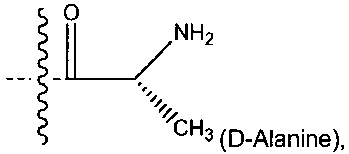 or
R² is 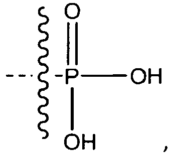 and R³ is 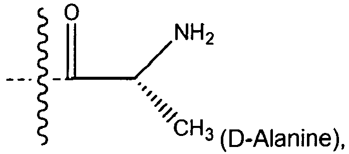 or
R² is 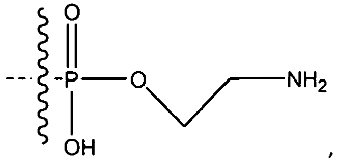 and R³ is... 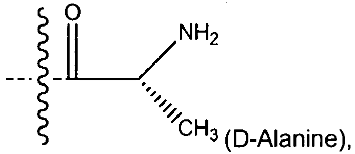 or
R² is 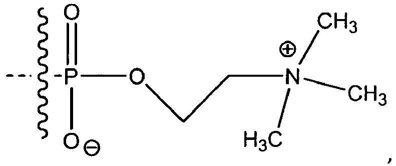 and R³ is... 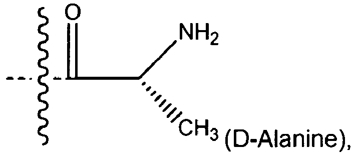 or
R² is 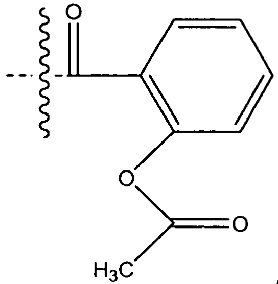 or
and R³ is... 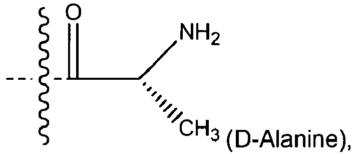 or
R² is 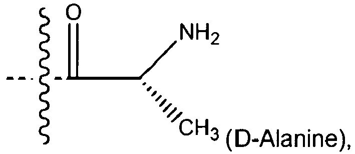 and R³ is...

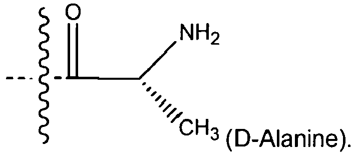

In another embodiment of the Glycerol Phenyl Butyrate Esters of General Formula I according to the invention
R¹ and R³ are 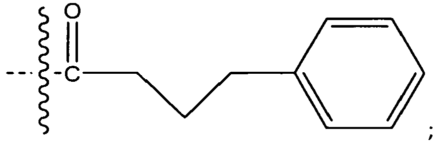 and R² is selected from
hydrogen or any of the following structures, which may optionally be substituted by one or more of F, Cl, Br, I, NH₂, OH, SH, CH₃, C₂H₅, C₃H₇, C₄H₉, CH₂Cl, CHCl₂, CF₃, CHF₂, CH₂F: 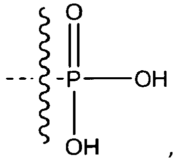 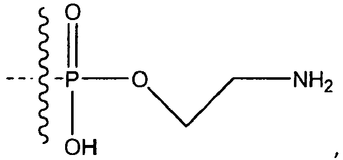 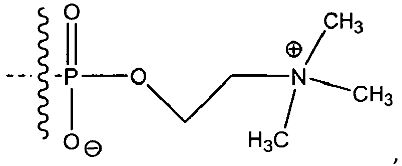 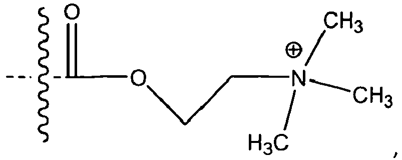 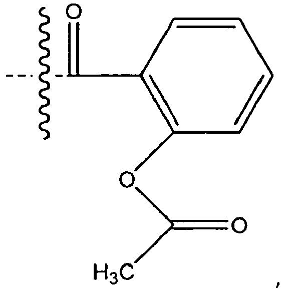 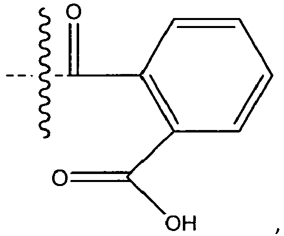 a radical of an amino acid, 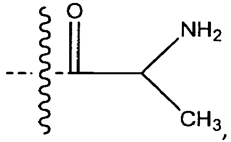 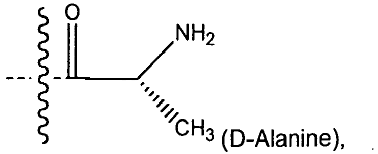 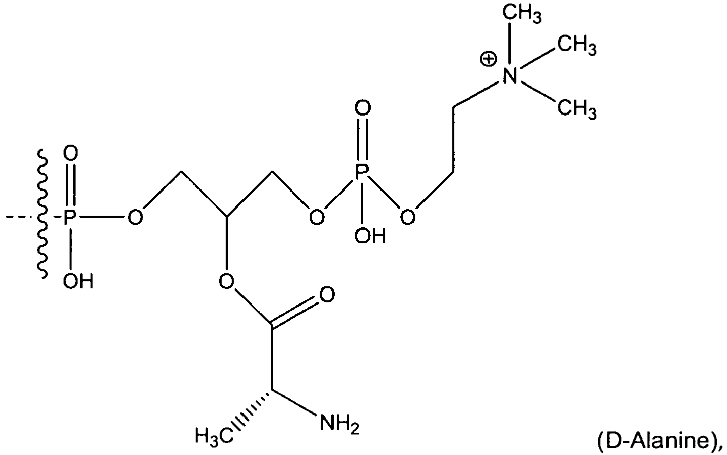 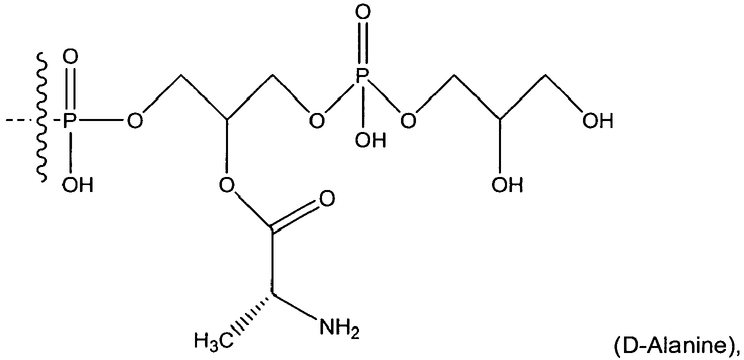 or 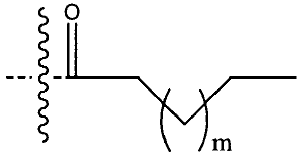 with m being 6, 7, 8, 9, 10, 11, 12, 13, 14, or 15, optionally unsaturated.

In another embodiment of the Glycerol Phenyl Butyrate Esters of General Formula I according to the invention
R² is selected from hydrogen or any of the following structures, which may optionally be substituted by one or more of F, Cl, Br, I, NH₂, OH, SH, CH₃, C₂H₅, C₃H₇, C₄H₉, CH₂Cl, CHCl₂, CF₃, CHF₂, CH₂F: 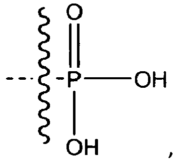 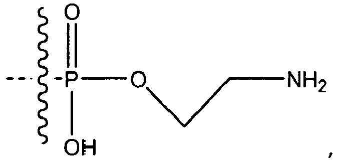 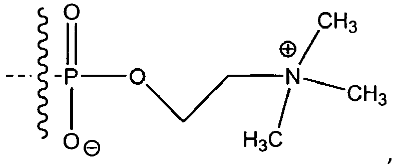 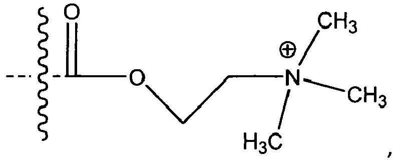 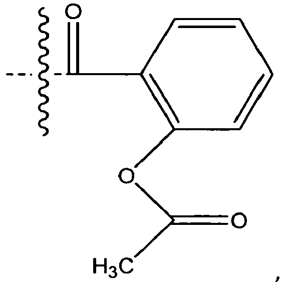 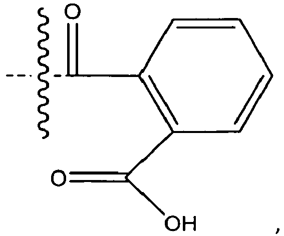 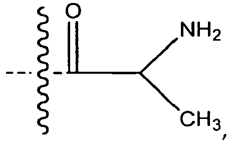 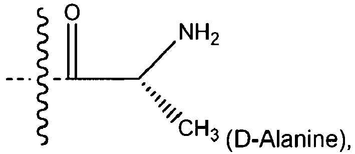 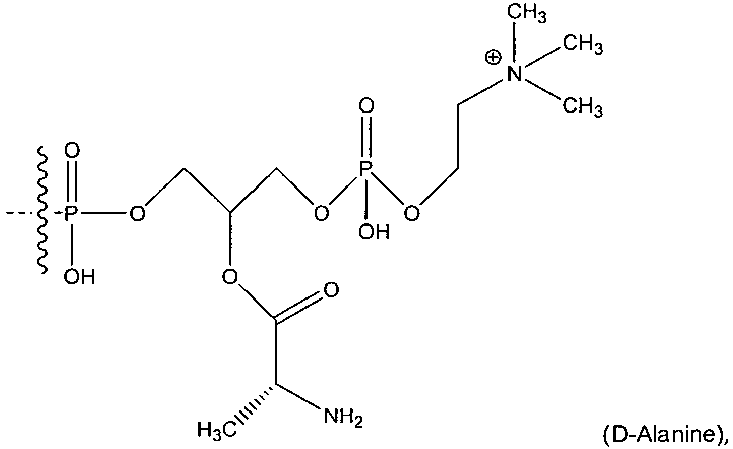 or

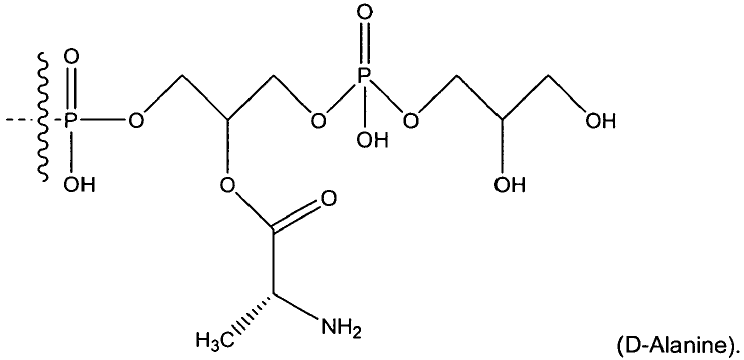

In another embodiment of the Glycerol Phenyl Butyrate Esters of General Formula I according to the invention
R² is 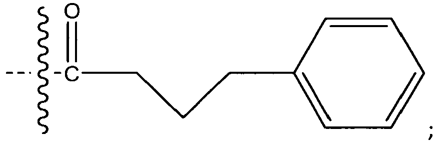 and R¹ and R³ independently from one another are selected from
hydrogen or any of the following structures, which may optionally be substituted by one or more of F, Cl, Br, l, NH₂, OH, SH, CH₃, C₂H₅, C₃H₇, C₄H₉, CH₂Cl, CHCl₂, CF₃, CHF₂, CH₂F: 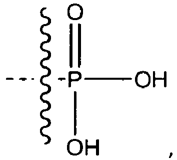 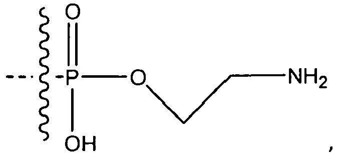 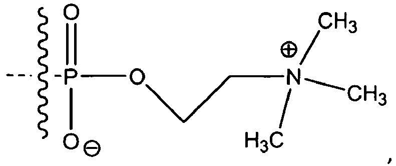 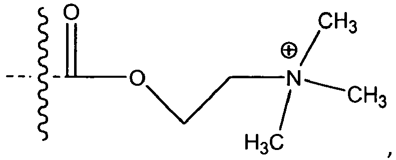 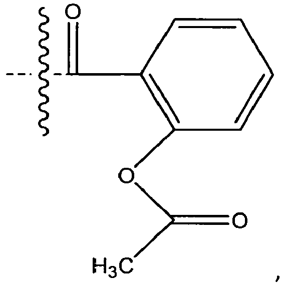 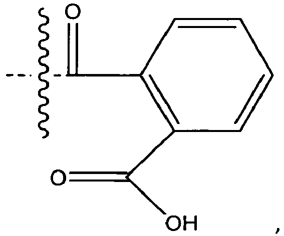 a radical of an amino acid, 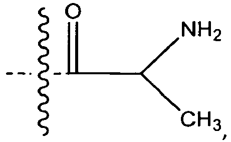 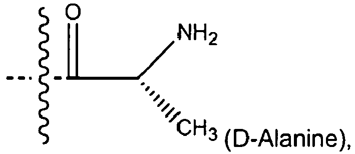 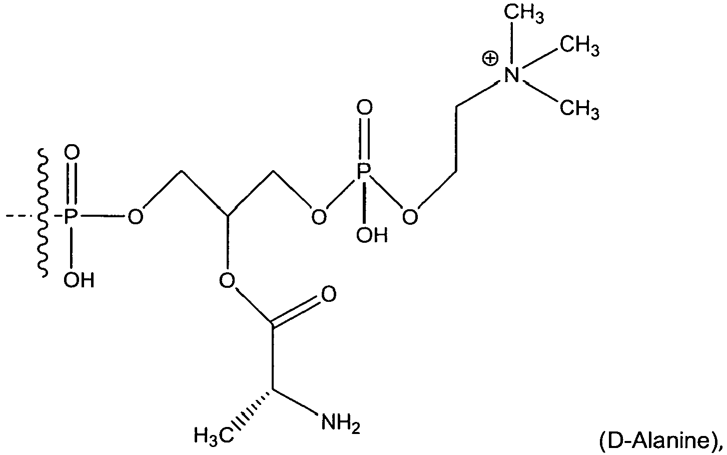 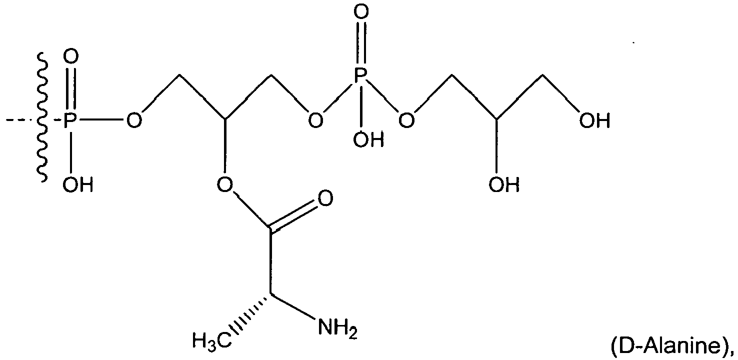 or 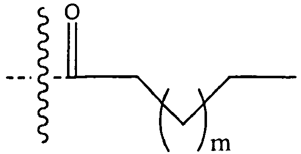 with m being 6, 7, 8, 9, 10, 11, 12, 13, 14, or 15, optionally unsaturated.

In another embodiment of the Glycerol Phenyl Butyrate Esters of General Formula I according to the invention
R¹ and R³ independently from one another are selected from
hydrogen or any of the following structures, which may optionally be substituted by one or more of F, Cl, Br, I, NH₂, OH, SH, CH₃, C₂H₅, C₃H₇, C₄H₉, CH₂Cl, CHCl₂, CF₃, CHF₂, CH₂F: 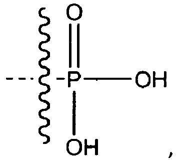 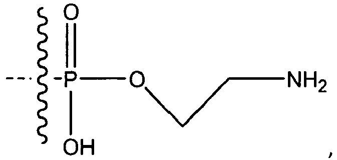 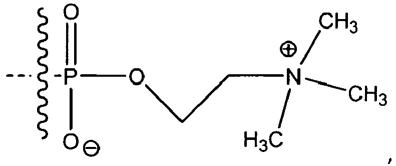 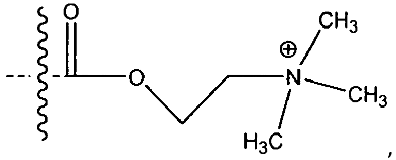 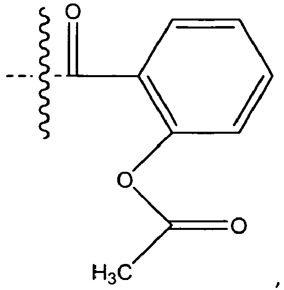 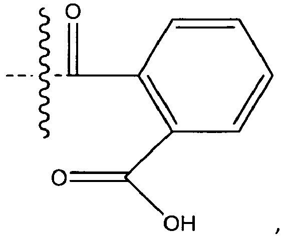 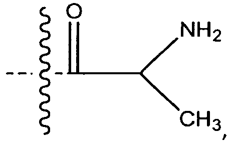 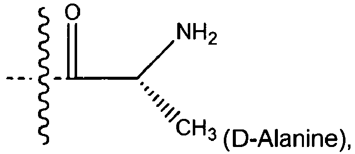 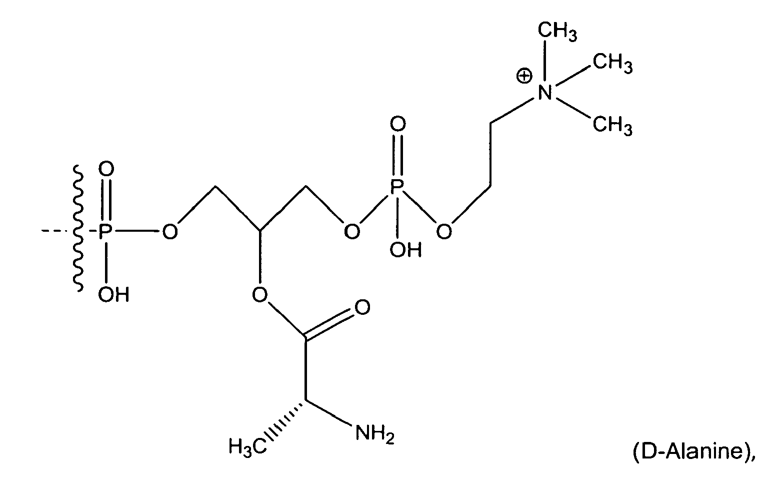 or

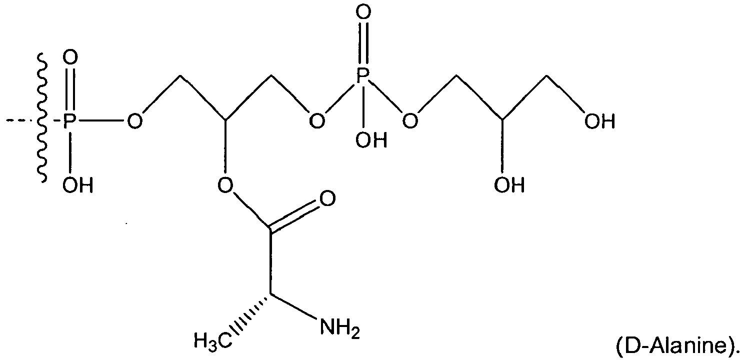

In another embodiment the Glycerol Phenyl Butyrate Ester of General Formula I according to the invention is selected from 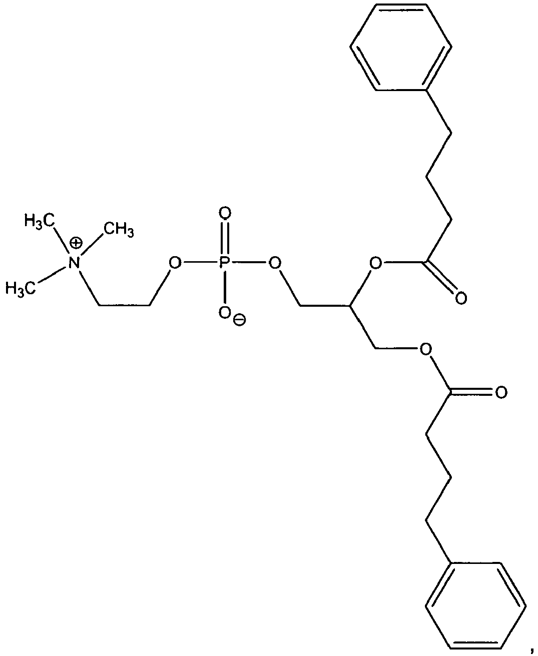 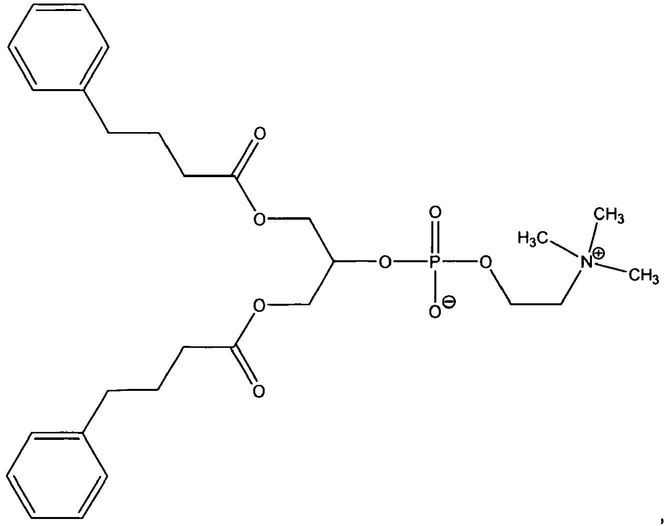 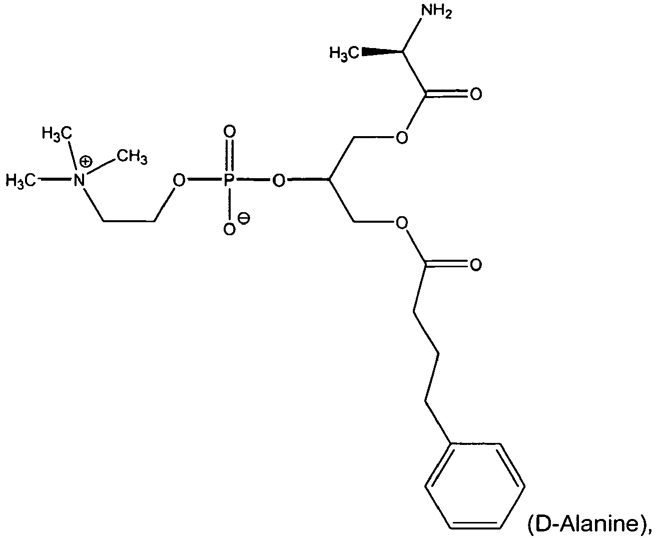 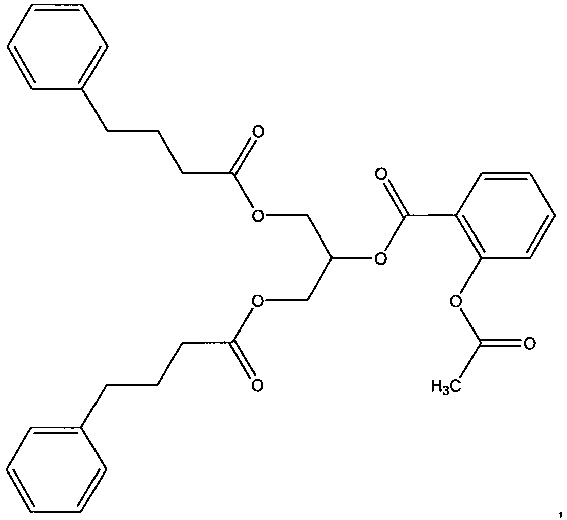 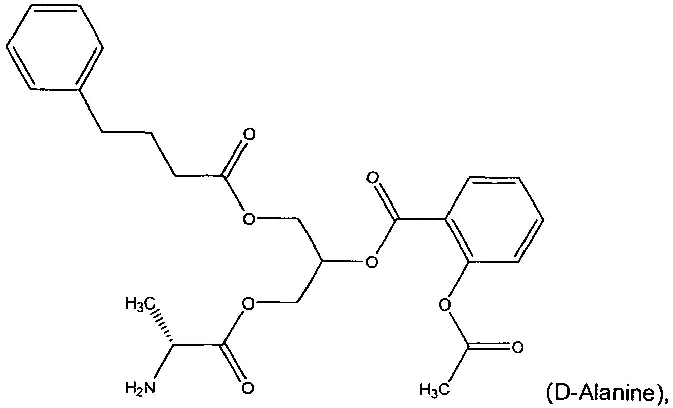 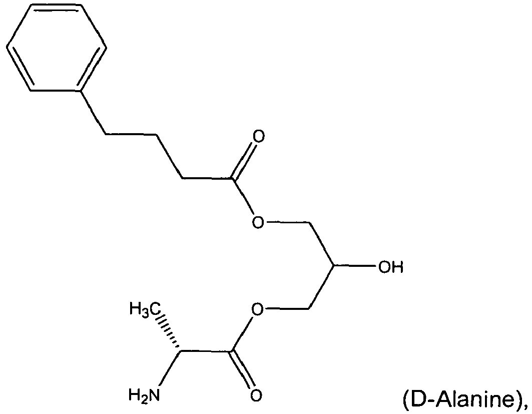 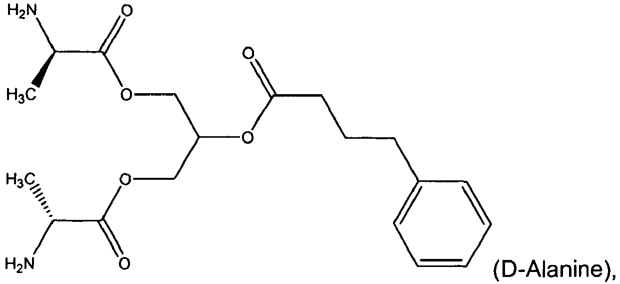 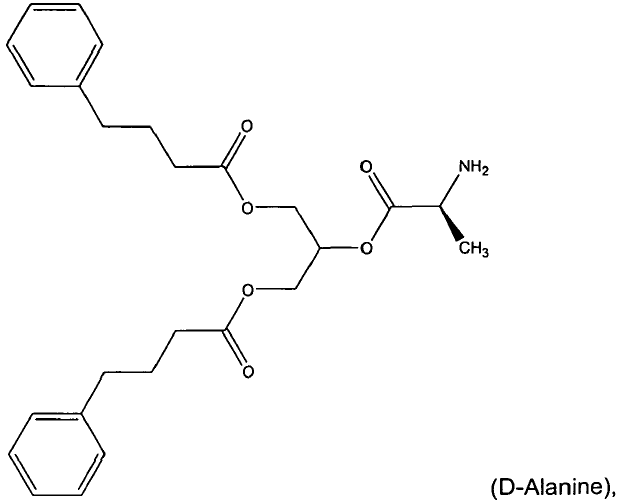 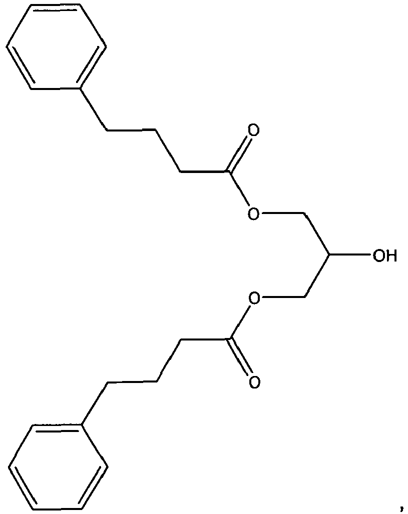 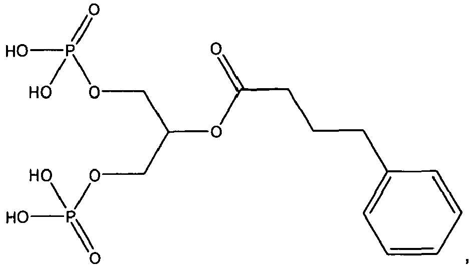 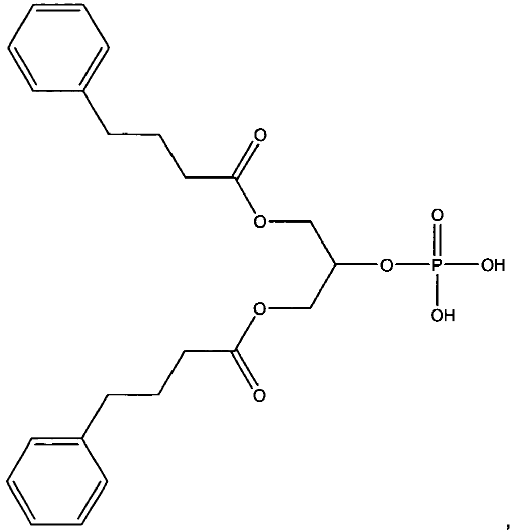 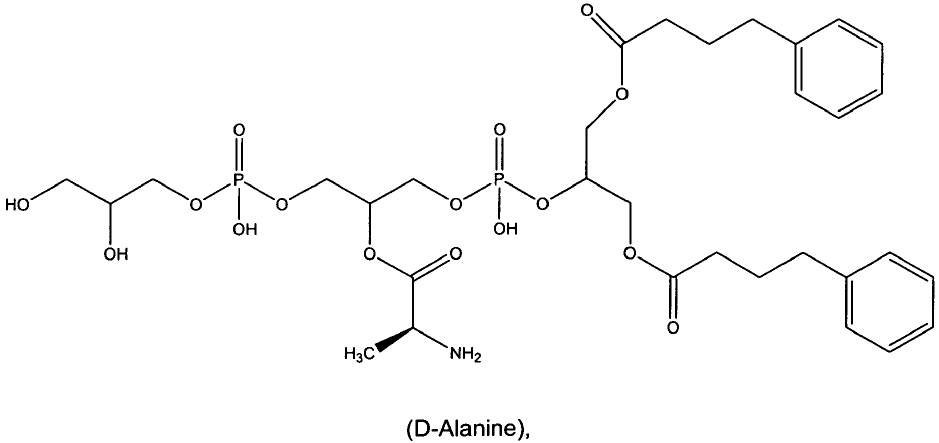 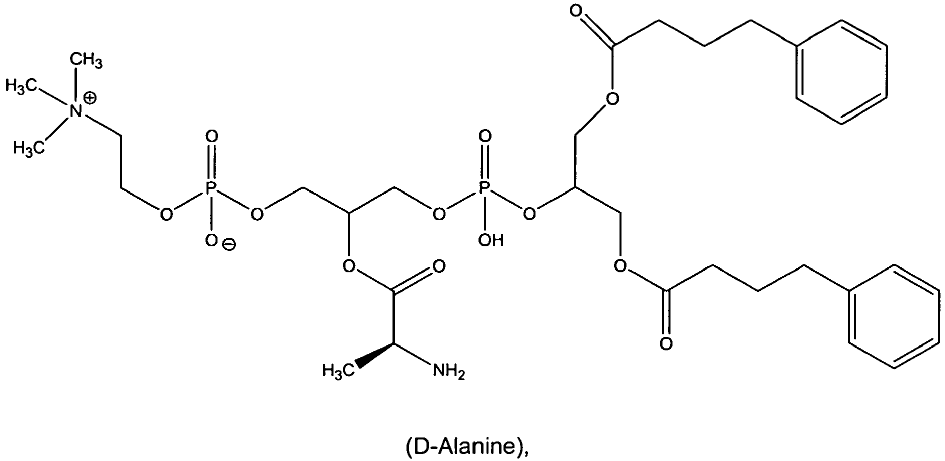 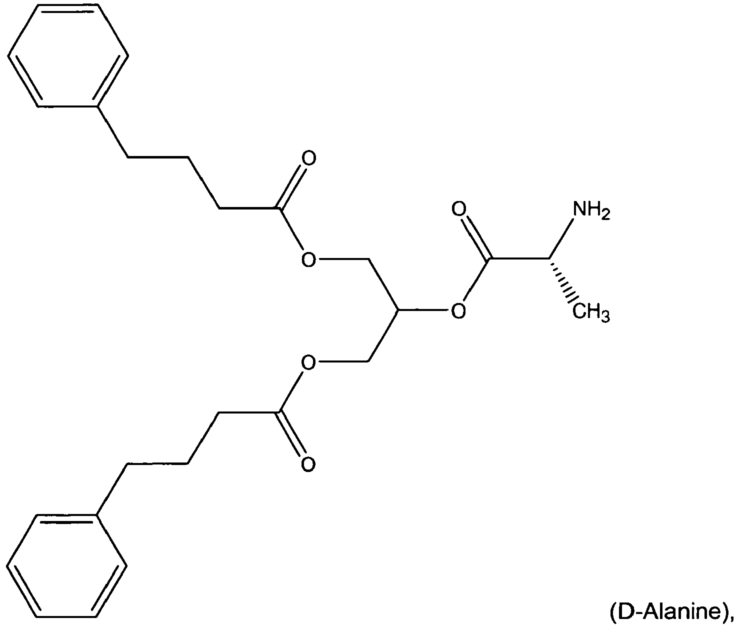 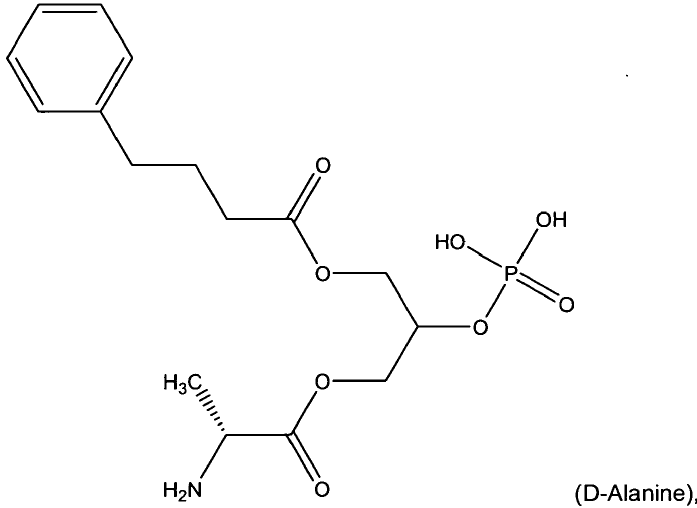 or 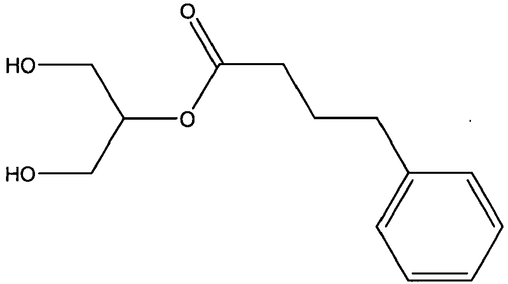 wherein the compound may optionally be substituted by one or more of F, Cl, Br, I, NH₂, OH, SH, CH₃, C₂H₅, C₃H₇, C₄H₉, CH₂Cl, CHCl₂, CF₃, CHF₂, CH₂F, preferably is unsubstituted;
optionally in form of one of its stereoisomers, enantiomers or diastereomers, its racemate or in form of a mixture of at least two of its stereoisomers in any mixing ratio;
in form of the free acid, free base or salt.

Another aspect of the invention refers to a Process for the production of a Glycerol Phenyl Butyrate Ester according to the invention wherein in a first step a compound according to formula II 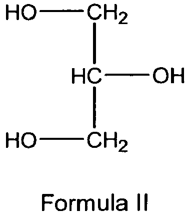 is reacted with at least one of R¹', R²' or R³' being compounds identical in structure to the substituents R¹, R² and R³ respectively as defined above except for being either the free acid, or base or salt thereof, or comprising one or more leaving groups LG like a halogen, like Br or Cl, and optionally one or more protecting groups.

Glycerol Phenyl Butyrate Esters according to the invention are physiologically well-tolerated. Accordingly, a further aspect of the invention refers to a Pharmaceutical formulation comprising at least one Glycerol Phenyl Butyrate Ester according to the invention and optionally at least one physiologically acceptable excipient.

A further aspect of the invention refers to a Glycerol Phenyl Butyrate Ester according to the invention for use in the treatment of cancer, mucositis, amyotropic lateral sclerosis (ALS), skin/mucosal itching, degenerative diseases of the eye, neurodegenerative disorders, Huntington's disease, eating disorders, obesity, drug induced weight gain, pruritis, alcoholism, promyelocytic leukemia, especially acute promyelocytic leukemia, cardiac injury, especially adriamycin induced cardiac injury, epithelial damage, connective tissue damage, desmosis, Parkinson Disease, myelodysplastic syndrome, urea cycle disorder, fibrotic lung diseases, hepatic encephalopathies, thioredoxin mediated diseases, human papilloma virus (HPV) infections, autoimmune diseases, thalassemia, genetic disorders; or in the regulation of HDAC6 activity, the modulation of stem cells, the prevention of grey hair, the promotion of neuronal growth, the prevention of bone and joint diseases, or as adjuvant in cancer therapy.

A parallel further aspect of the invention refers to the use of a Glycerol Phenyl Butyrate Ester according to the invention in the manufacture of a medicament for the treatment of cancer, mucositis, amyotropic lateral sclerosis (ALS), skin/mucosal itching, degenerative diseases of the eye, neurodegenerative disorders, Huntington's disease, eating disorders, obesity, drug induced weight gain, pruritis, alcoholism, promyelocytic leukemia, especially acute promyelocytic leukemia, cardiac injury, especially adriamycin induced cardiac injury, epithelial damage, connective tissue damage, desmosis, Parkinson Disease, myelodysplastic syndrome, urea cycle disorder, fibrotic lung diseases, hepatic encephalopathies, thioredoxin mediated diseases, human papilloma virus (HPV) infections, autoimmune diseases, thalassemia, genetic disorders; or for the regulation of HDAC6 activity, the modulation of stem cells, the prevention of grey hair, the promotion of neuronal growth, the prevention of bone and joint diseases, or for use as adjuvant in cancer therapy.

A parallel further aspect of the invention refers to a method of treating a being (human or animal) suffering from cancer, mucositis, amyotropic lateral sclerosis (ALS), skin/mucosal itching, degenerative diseases of the eye, neurodegenerative disorders, Huntington's disease, eating disorders, obesity, drug induced weight gain, pruritis, alcoholism, promyelocytic leukemia, especially acute promyelocytic leukemia, cardiac injury, especially adriamycin induced cardiac injury, epithelial damage, connective tissue damage, desmosis, Parkinson Disease, myelodysplastic syndrome, urea cycle disorder, fibrotic lung diseases, hepatic encephalopathies, thioredoxin mediated diseases, human papilloma virus (HPV) infections, autoimmune diseases, thalassemia, genetic disorders; or from a dis-regulation of HDAC6 activity, the dis-modulation of stem cells, having or threatened by grey hair, the lack of neuronal growth, having or threatened by bone and joint diseases, or subject to cancer therapy by applying a suitable amount of at least one a Glycerol Phenyl Butyrate Ester according to the invention to the being. Preferably this application is done in form of a medicament or pharmaceutical formulation.

For the medicament or pharmaceutical formulation according to the present invention or for a medicament or pharmaceutical formulation used according to the invention the medicament or pharmaceutical formulation can be of any form suitable for the application to humans and/or animals, preferably humans including infants, children and adults and can be produced by standard procedures known to those skilled in the art. The medicament or pharmaceutical formulation can be produced by standard procedures known to those skilled in the art, e.g. from the table of contents of "Pharmaceutics: The Science of Dosage Forms", Second Edition, Aulton, M.E. (ED. Churchill Livingstone, Edinburgh (2002); "Encyclopedia of Pharmaceutical Technology", Second Edition, Swarbrick, J. and Boylan J.C. (Eds.), Marcel Dekker, Inc. New York (2002); "Modern Pharmaceutics", Fourth Edition, Banker G.S. and Rhodes C.T. (Eds.) Marcel Dekker, Inc. New York 2002 y "The Theory and Practice of Industrial Pharmacy", Lachman L., Lieberman H. And Kanig J. (Eds.), Lea & Febiger, Philadelphia (1986). The respective descriptions are hereby incorporated by reference and form part of the disclosure. The composition of the medicament may vary depending on the route of administration.

The medicament or pharmaceutical formulation of the present invention may for example be administered parentally in combination with conventional injectable liquid carriers, such as water or suitable alcohols. Conventional pharmaceutical excipients for injection, such as stabilizing agents, solubilizing agents, and buffers, may be included in such injectable compositions. These medicaments or pharmaceutical formulation may for example be injected intramuscularly, intraperitoneally, or intravenously.

Medicaments or pharmaceutical formulation according to the present invention may also be formulated into orally administrable compositions containing one or more physiologically compatible carriers or excipients, in solid or liquid form. These compositions may contain conventional ingredients such as binding agents, fillers, lubricants, and acceptable wetting agents. The compositions may take any convenient form, such as tablets, pellets, granules, capsules, lozenges, aqueous or oily solutions, suspensions, emulsions, or dry powdered forms suitable for reconstitution with water or other suitable liquid medium before use, for immediate or modified or controlled release. The multiparticulate forms, such as pellets or granules, may e.g. be filled into a capsule, compressed into tablets or suspended in a suitable liquid.

Suitable controlled release formulations, materials and methods for their preparation are known from the prior art, e.g. from the table of contents of "Modified-Release Drug Delivery Technology", Rathbone, M.J. Hadgraft, J. and Roberts, M.S. (Eds.), Marcel Dekker, Inc., New York (2002); "Handbook of Pharmaceutical Controlled Release Technology", Wise, D.L. (Ed.), Marcel Dekker, Inc. New York, (2000); "Controlled Drug Delivery", Vol, I, Basic Concepts, Bruck, S.D. (Ed.), CRD Press Inc., Boca Raton (1983) y de Takada, K. and Yoshikawa, H., "Oral Drug Delivery", Encyclopedia of Controlled Drug Delivery, Mathiowitz, E. (Ed.), John Wiley & Sons, Inc., New York (1999), Vol. 2, 728-742; Fix, J., "Oral drug delivery, small intestine and colon", Encyclopedia of Controlled Drug Delivery, Mathiowitz, E. (Ed.), John Wiley & Sons, Inc., New York (1999), Vol. 2, 698-728. The respective descriptions are hereby incorporated by reference and form part of the disclosure.

Medicaments or pharmaceutical formulation according to the present invention may also comprise an enteric coating, so that their dissolution is dependent on pH-value. Due to said coating the medicament may pass the stomach undissolved and the respective Glycerol Phenyl Butyrate Ester according to the invention is liberated in the intestinal tract. Preferably the enteric coating is soluble at a pH value of 5 to 7.5. Suitable materials and methods for the preparation are known from the prior art.

Typically, the medicaments according to the present invention may contain 1-60 % by weight of one or more of the Glycerol Phenyl Butyrate Ester according to the invention and 40-99 % by weight of one or more auxiliary substances (additives/excipients).

The joint mixture of the present invention may also be administered topically or via a suppository.

The daily dosage for humans and animals may vary depending on factors that have their basis in the respective species or other factors, such as age, sex, weight or degree of illness and so forth. The daily dosage for humans preferably is in the range of 10 to 2000 milligrams of active substance (Glycerol Phenyl Butyrate Ester according to the invention) to be administered during one or several intakes per day.

In a very preferred embodiment the medicament or pharmaceutical formulation of the invention is an oral pharmaceutical composition. Very suitable oral pharmaceutical compositions comprising 4-phenyl-butyric acid or a physiologically acceptable salt thereof are disclosed in EP1427396 (WO2003/22253), the content of which forming herewith part of the description/disclosure of the current invention. Accordingly, it is very preferred if such an oral pharmaceutical composition comprising a Glycerol Phenyl Butyrate Ester according to the invention is formulated in an analoguous way to the formulation presented in EP1427396 (WO2003/22253). It is thus further very preferred if an oral medicament according to the invention especially a pharmaceutical composition comprises 100 to 2000 mg of the Glycerol Phenyl Butyrate Ester according to the invention, preferably 200 to 1000 mg, most preferably approximately 500 mg or 250 mg of the Glycerol Phenyl Butyrate Ester according to the invention.

In another alternative embodiment the Glycerol Phenyl Butyrate Ester according to the invention is formulated in a medicament or pharmaceutical formulation in form of a tablet wherein each gram of granulate contains 100 to 2000 mg of the Glycerol Phenyl Butyrate Ester according to the invention, preferably 500 to 1000 mg of the Glycerol Phenyl Butyrate Ester according to the invention. Preferably the granulate does further comprise microcrystalline cellulose, magnesium stearate and/or colloidal anhydrous silica.

In another alternative embodiment the Glycerol Phenyl Butyrate Ester according to the invention is formulated in a medicament or pharmaceutical formulation in form of a granulate containing 100 to 2000 mg of the Glycerol Phenyl Butyrate Ester according to the invention, preferably 500 to 1000 mg of the Glycerol Phenyl Butyrate Ester according to the invention. Preferably, the granulate does further comprise calcium stearate and/or colloidal anhydrous silica.

The present invention is illustrated below with the help of figures and examples. These illustrations are given solely by way of example and do not limit the invention.

### FIGURES:

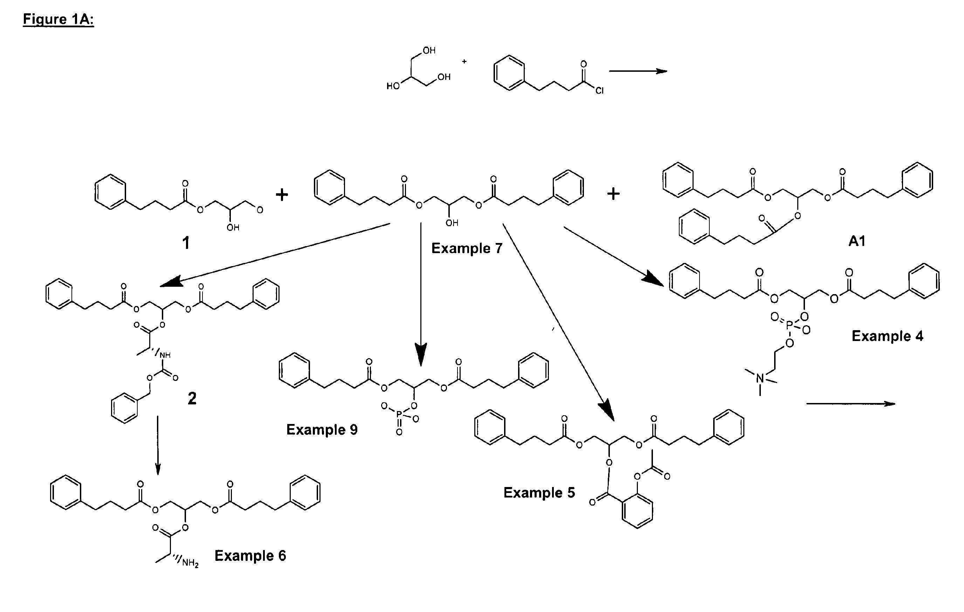

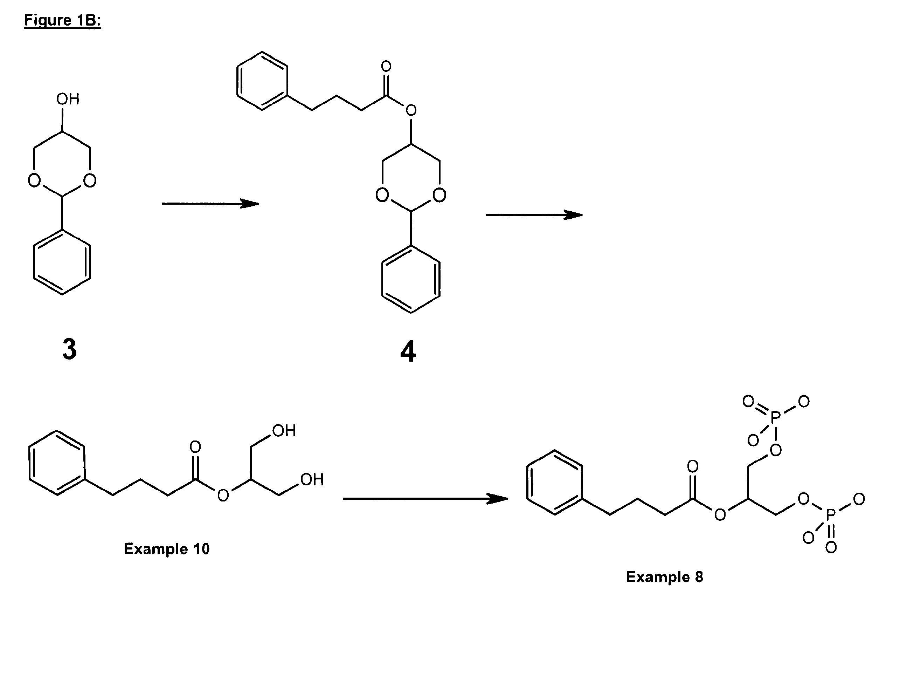

**Figure 1****:** Synthesis Scheme covering the Synthesis of Examples 4, 5, 6, 7, 8, 9 and 10.

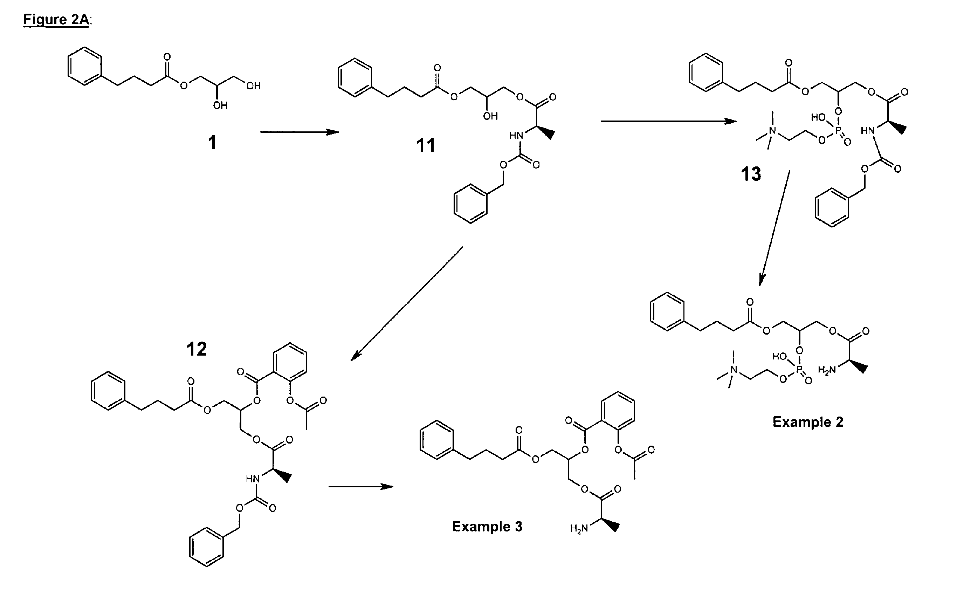

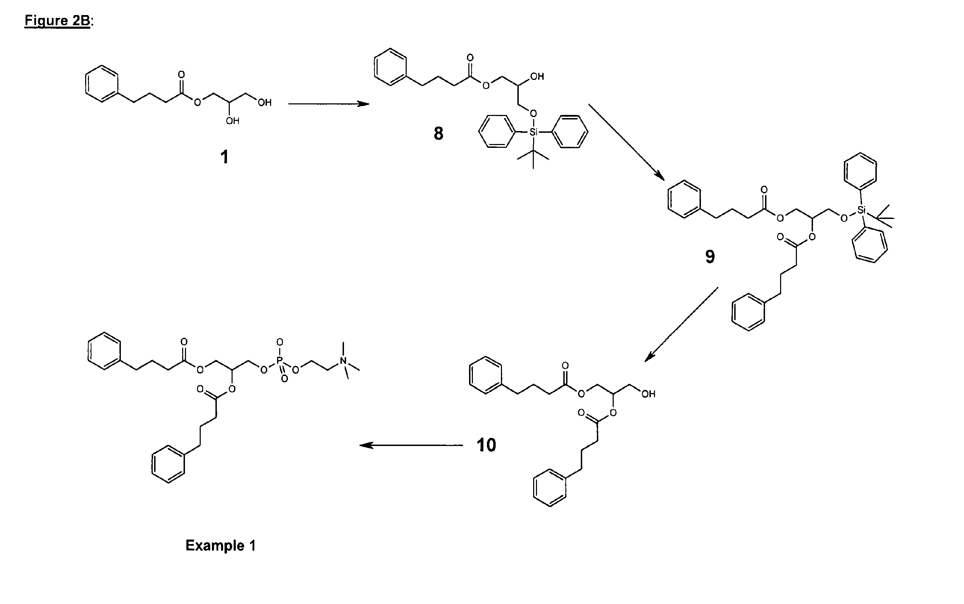

**Figure 2****:** Synthesis Scheme covering the Synthesis of Examples 1, 2, and 3.

### EXAMPLES:

### Example 1: Synthesis of 4-Phenyl-butyric acid 2-[(2-dimethylamino-ethoxy)-hydroxy-phosphoryloxyl-l-(4-phenyl-butyryloxymethyl)-ethyl ester (Example 1)

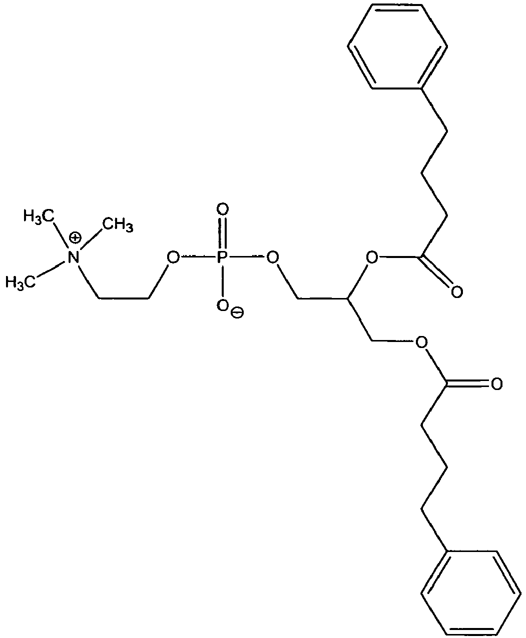

### Example 2: Synthesis of 4-Phenyl-butyric acid 3-(2-amino-propionyloxy)-2-[(2-dimethylamino-ethoxy)-hydroxy-phosphoryloxyl-propyl ester (Example 2)

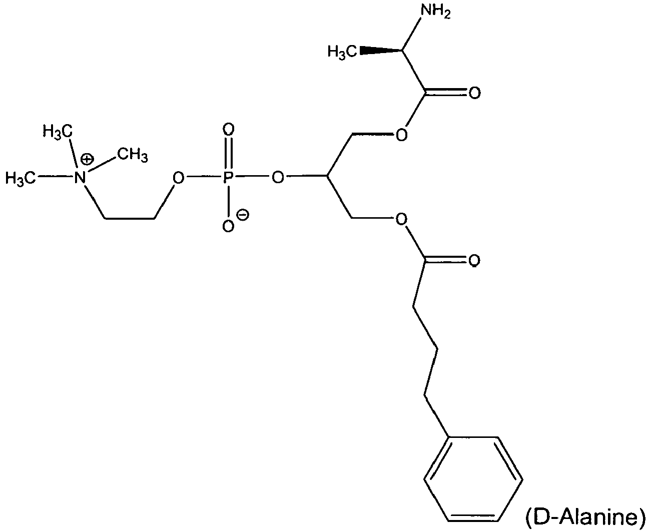

### Example 3: Synthesis of 2-Acetoxy-benzoic acid 2-(2-amino-propionyloxy)-l-(4-phenyl-butyryloxymethyl)-ethyl ester (Example 3)

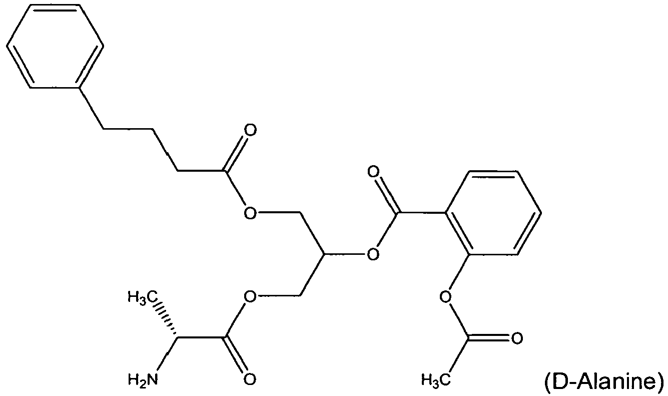

### Synthesis of Examples 1 to 3:

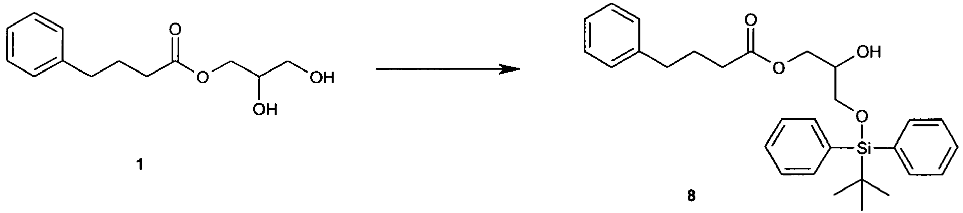

To a solution of **1** (40g) and imidazole (17.3g) in DMF (430ml) was added 4-tert.Butyldiphenylsilylchlorid (47.3ml) drop wise at 0°. The mixture was stirred for 18 h at 0° to room temperature. After dilution with MTBE the organic Phase was washed with satd. Na2CO3 solution and satd. NaCl solution. The organic layer was dried with NaSO4 and concentrated under reduced pressure. **8** (84g, 0.176mol, 105%) was obtained as yellow oil.
1HNMR in CDCl3
7.60-7.66 (m,4H), 7.35-7.15(m, 11H,Ph), 4.19 (m,2H), 3.95 (m,1H), 3.75 (m,2H), ), 2.65, (m,2H), 2.39 (m,2H),1.95(m,2H), 1.12(s, 9H). M+H= 456, M+Na= 499.

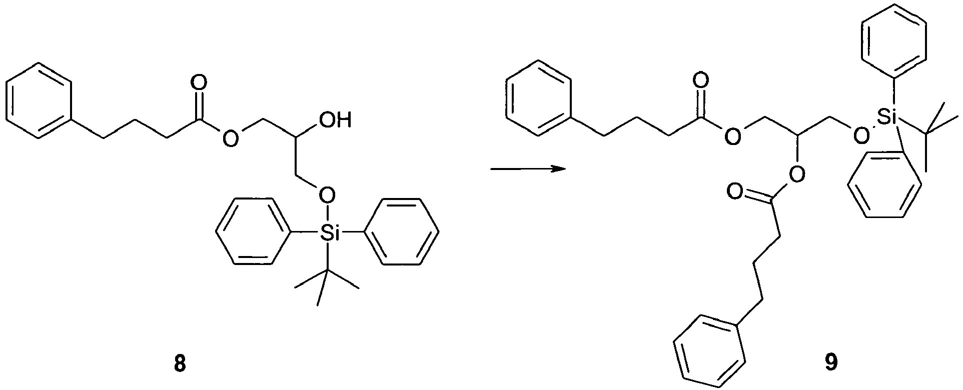

To a solution of **8** (15g) in pyridine (150ml) was added4-Phenylbutanoylchloride (6.5g) dissolved in 12 ml THF at room temperature. The mixture was stirred for 16 h at rt. The solvent was removed under reduced pressure and the residue was dissolved in MTBE and washed with 1 N NaOH and 1 N. HCl. The organic layer was dried with NaSO4 and concentrated under reduced pressure. **9** (21g, 33.7mmol, 107%) was obtained as yellow oil.
1 HNMR in CDCl3
8.62 (m, 4H), 7.72-7.15(m, 16H), 5.15 (m, 1H), 4.41 (dd,j= 3.8,11.8, 1H), 4.22 (dd, j=6.1,11.8, 1H) 3.75 (m, 2H), ), 2.65 (m, 4H), 2.39 (m, 4H), 1.95(m, 4H), 1.12 (s, 9H).M+Na= 645.

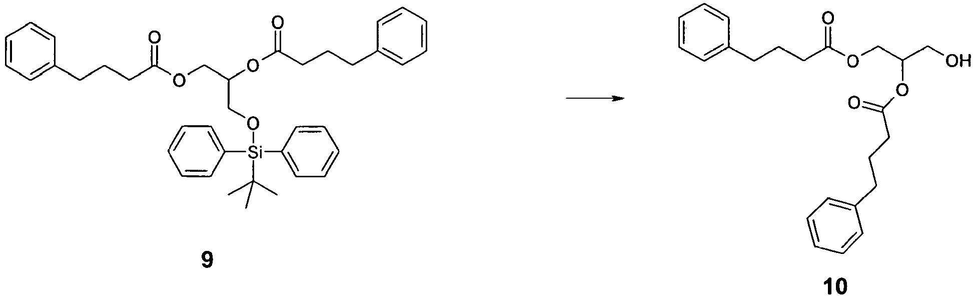

To a solution of **9** (20g) in THF (200ml) was added 1 M TBAF in THF (48.2ml), which was neutralized with AcOH. The mixture was stirred for 24 h at rt.EE was added and the resulting solution was washed with 1 N HCl. The organic layer was dried with NaS04 and concentrated under reduced pressure. The crude product was purified by chromatography (Tol/EE 10-1 → Tol/EE 9-1) to give **10** (1.2g, 2.2mmol, 28%) as colorless oil.
1 HNMR in CDCl3
7.35-7.15(m,10H,Ph), 5.15 (m,1H), 4.33 (dd,j= 4.4,11.9, 1H), 4.22 (dd, j=5.6,11.9, 1H) 2.65, (m,4H), 2.33 (m,4H), 1.95(m,4H). M+Na= 407.

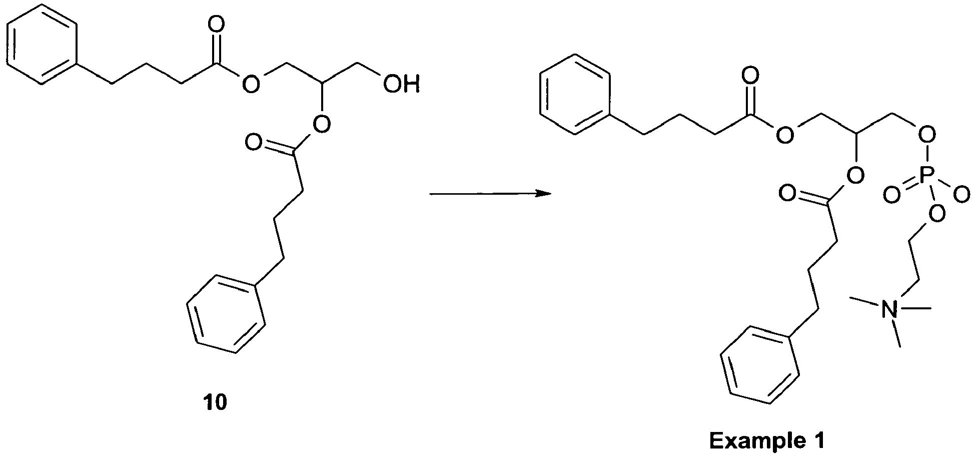

To a solution of 2-Bromethyldichlorphosphate (11.75g) in 450ml THF was added 68.8ml pyridine dissolved in 100 ml THF drop wise at 0°. To the resulting mixture was added 10 (7g) dissolved in 100ml THF drop wise at 0°. Stirring was continued for 16 h. Hydrolysis was obtained by addition of 150 ml ice water. Then the solvent was removed by evaporation. The residue was dissolved in DCM and extracted with water. The organic layer was dried with NaSO4 and concentrated under reduced pressure. The crude product was used without further purification. It was dissolved in 18 ml pyridine and 50 ml Trimethylamin solution in water (44%) was added and stirred at room temperature for 18h. After dilution with DCM the organic phase was washed with water. The organic layer was dried with NaSO4 and concentrated under reduced pressure. The crude product was purified by chromatography (Tol/MeOH/NH3 = 4/1/0.05) to give **Example 1** (4.09g, 7.3mmol, 43%) as colorless oil.
1H NMR in CDCl3:
7.35-7.15(m,10H), 5.21 (m, 1H), 4.41 (dd,j=3.1,12.0, 1H),4.22 (m, 2H), 4.15 (dd, j= 7.0, 12.0), 3.90 (m, 2H), 3.23 (s, 9H), 2.60, (m, 4H), 2.38 (m,4H), 1.85 (m, 4H). M⁺= 551.

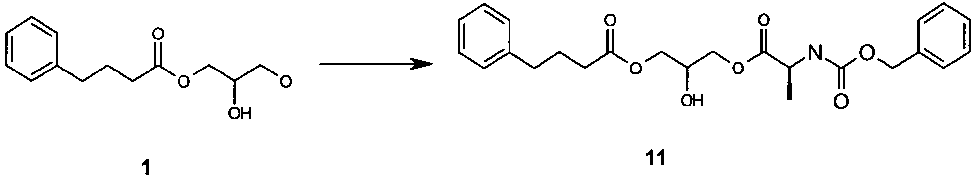

A solution of 1 (50g), Z-D-Ala-OH (46.84g), HOBT (28.64 g), EDCxHCl (40.63g) and DMAP (2.59g) in DCM (500ml) was stirred for 24h.The organic phase was washed with satd. NaHCO3 The organic layer was dried with NaSO4 and concentrated under reduced pressure.
The crude product was purified by chromatography (Toluol → Toluol/EE 4-1) to give 11 (48.7g, 0.11 mol, 52 %) as colorless oil
1HNMR in CDCl3:
7.35-7.15(m, 10H), 5.25 (d,j=6.7, 1H), 5.10 (m, 2H), 4.41-4.02 (m, 6H), 2.60, (m, 4H), 2.38 (m, 4H), 1.85 (m, 4H). 2.62, (m, 2H), 2.38 (m, 2H), 1.88 (m, 2H), 1.42 (d, j=7.2, 3H). M+Na=467.

### Example 4: Synthesis of 4-Phenyl-butyric acid 2-[(2-dimethylamino-ethoxy)-hydroxy-phosphoryloxy]-3-(4-phenyl-butyryloxy)-propyl ester (Example 4)

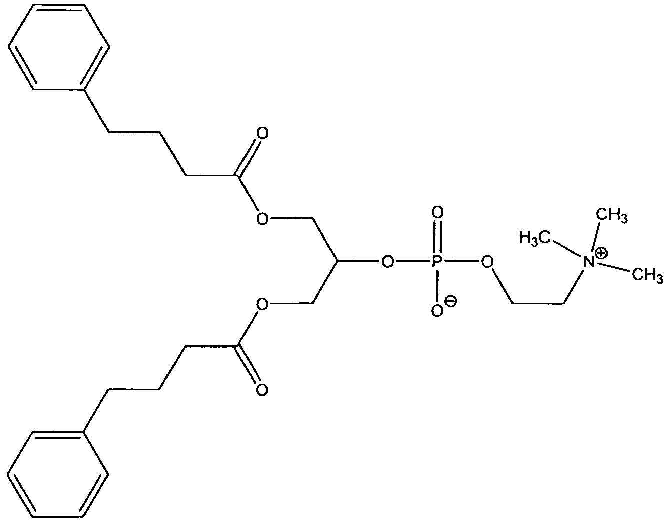

### Example 5: Synthesis of 2-Acetoxy-benzoic acid 2-(4-phenyl-butyryloxy)-1-(4-phenyl-butyryloxymethyl)-ethyl ester (Example 5)

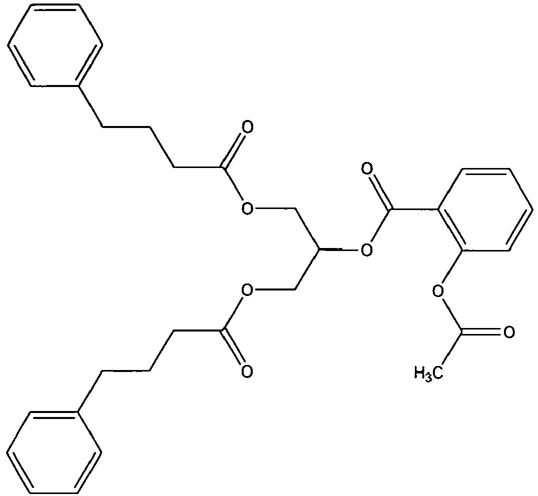

### Example 6: Synthesis of 4-Phenyl-butyric acid 2-(2-amino-propionyloxy)-3-(4-phenyl-butyryloxy)-propyl ester (Example 6)

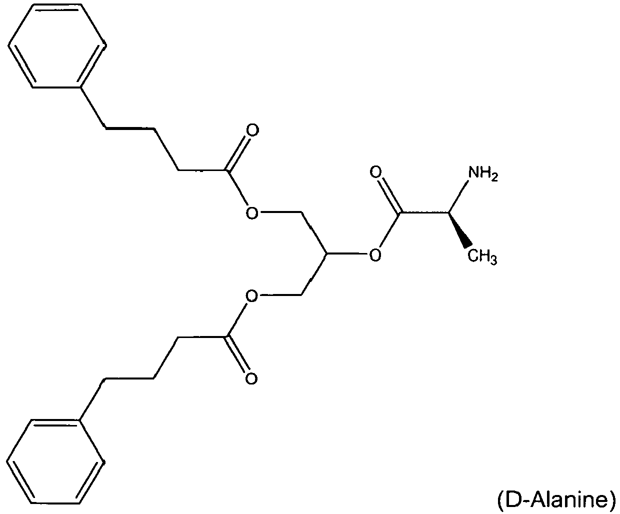

### Example 7: Synthesis of 4-Phenyl-butyric acid 2-hydroxy-3-(4-phenyl-butyryloxy)-propyl ester (Example 7)

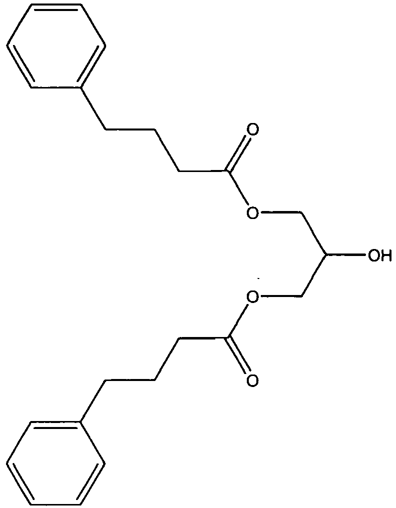

### Example 8: Synthesis of 4-Phenyl-butyric acid 2-phosphonooxy-1-phosphonooxymethyl-ethyl ester (Example 8)

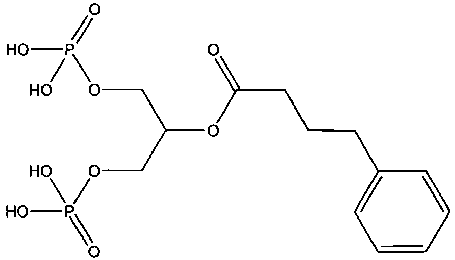

### Example 9: Synthesis of 4-Phenyl-butyric acid 3-(4-phenyl-butyryloxy)-2-phosphonooxy-propyl ester (Example 9)

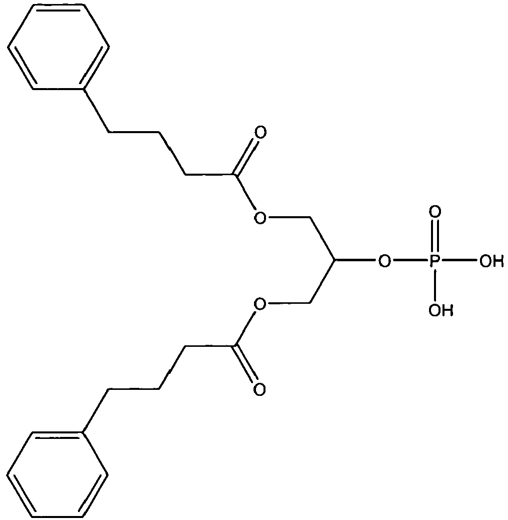

### Example 10: Synthesis of 4-Phenyl-butyric acid 2-hydroxy-1-hydroxymethyl-ethyl ester (Example 10)

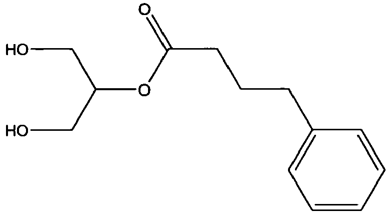

### Synthesis of Examples 4 to 10:

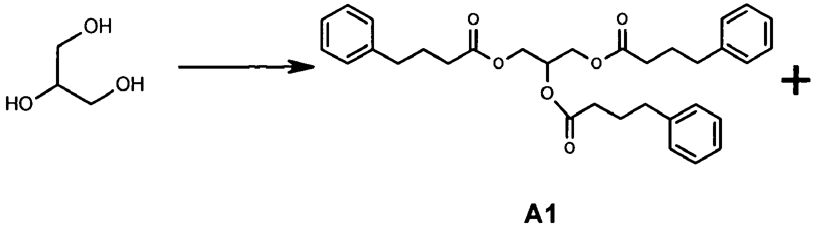

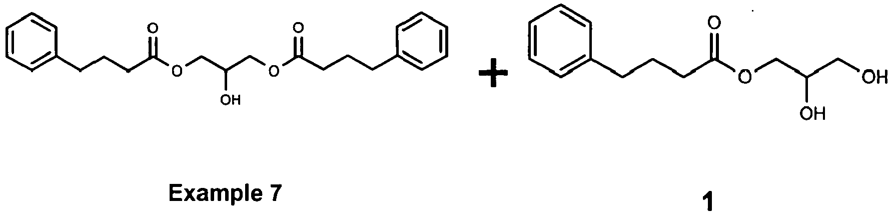

To a solution of glycerol (79.4g) in THF (1530ml) and NEt3 (132ml) was added 4-Phenylbutanoylchloride (155.9g) dissolved in 500 ml THF at 0°. The mixture was stirred for 4 h this temperature. 2.51 satd. NaHCO3-solution is added and the mixture was extracted with 2.51 MTBE. The organic layer was dried with NaSO4 and concentrated under reduced pressure. The residue was purified by chromatography (Tol/EE = 20/1→Tol/EE=1/1) to give A1 (3.4g, 6.4mmol, 1.7%), **Example 7** (43.8g, 0.11mol, 22%) and 1 (51g, 0.21, 25%).
1HNMR in CDCl3:
**A1:** 7.35-7.15(m, 5H), 5.27(m, 1H), 4.3(dd, 2H, j=4.2, 11.9), 4.11 (dd, 2H,j=5.9,11.9), 2.65, (m,6H), 2.31 (m,6H), 1.95(m,6H). M+Na= 553.

### Example 7: 7.35-7.15(m, 10H, 4.22-3.95 (m, 5H), 2.65, (m,4H), 2.31 (m,4H), 1.95 (m,4H). M+Na= 407.

1: 7.35-7.15(m,5H), 4.25-4.15(m,2H), 3.95 (m,1H), 3.70-3.55(m, 2H), 2.65, (m,2H), 2.31 (m,2H), 1.95 (m,2H). M+Na= 261.

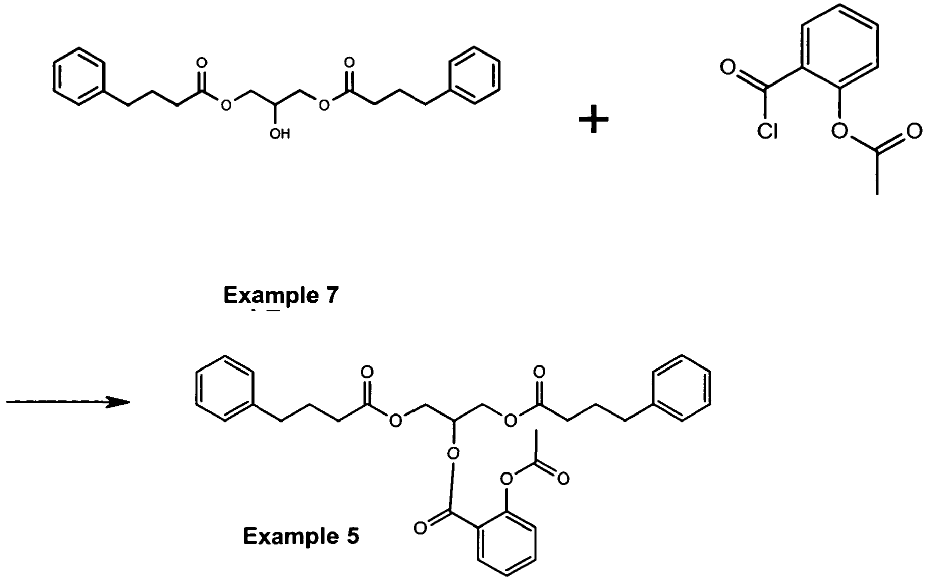

To a solution of **Example 7** (3g) in pyridine (30ml) was added Acetylsalicyloylchloride (1.7g) dissolved in 2 ml THF at room temperature. The mixture was stirred for 4 h at rt. The solvent was removed under reduced pressure and the residue was dissolved in TMBE and washed with 10% KHSO4, satd. NaHCO3 and satd. NaCl. The organic layer was dried with NaSO4 and concentrated under reduced pressure. The crude product was purified by chromatography (Heptane/EE = 4/1) to give **Example 5** (1.2g, 2.2mmol, 28%) as colorless oil.
1HNMR in CDCl3:
8.0 (m, 1H), 7.55 (m. 1H), 7.35-7.15(m, 12H), 5.47 (m, 1H), 4.42-4.25 (m, 4H), 2.65, (m, 4H), 2.31 (m, 4H), 1.95 (m, 4H). M+Na= 569.

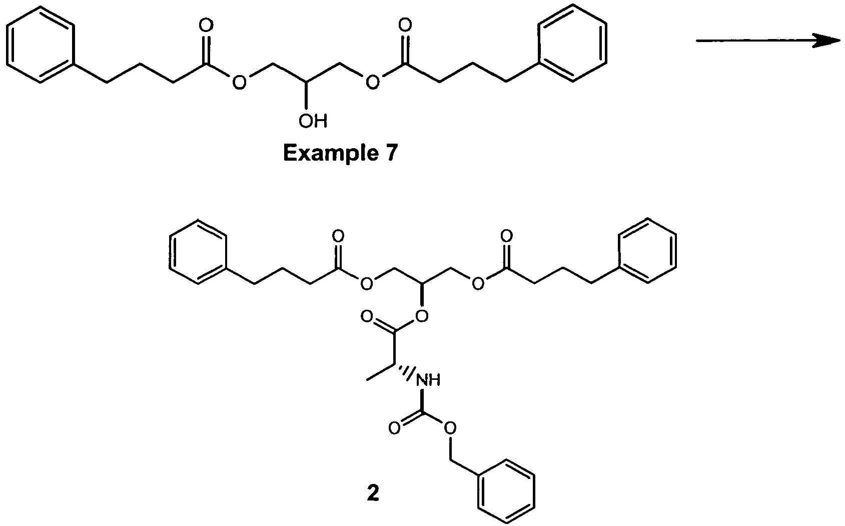

A solution of **Example 7** (10g), Z-D-Ala-OH (5.6g), HOBT (6.8g), EDCl (5.6g) and DMAP (0.6g) in DCM was stirred for 24h.The organic phase was washed with 1 N NaOH, 1 N HCI and satd. NaHCO3. The organic layer was dried with NaSO4 and concentrated under reduced pressure.

The crude product was purified by chromatography (Heptane/EE = 4/1) to give 2 (2.44g, 4.1 mmol, 12 %) as colorless oil
1HNMR in CDCI3:
7.75 (d, 1H, j=7.0), 7.35-7.15(m, 15H), 5.35 (m, 1H), 5.0 (m2H), 4.28-4.00 m, 5H), 2.65, (m, 4H), 2.31 (m, 4H), 1.95 (m, 4H), 1.12 (d, 3H, j=7.0). M+Na= 612.

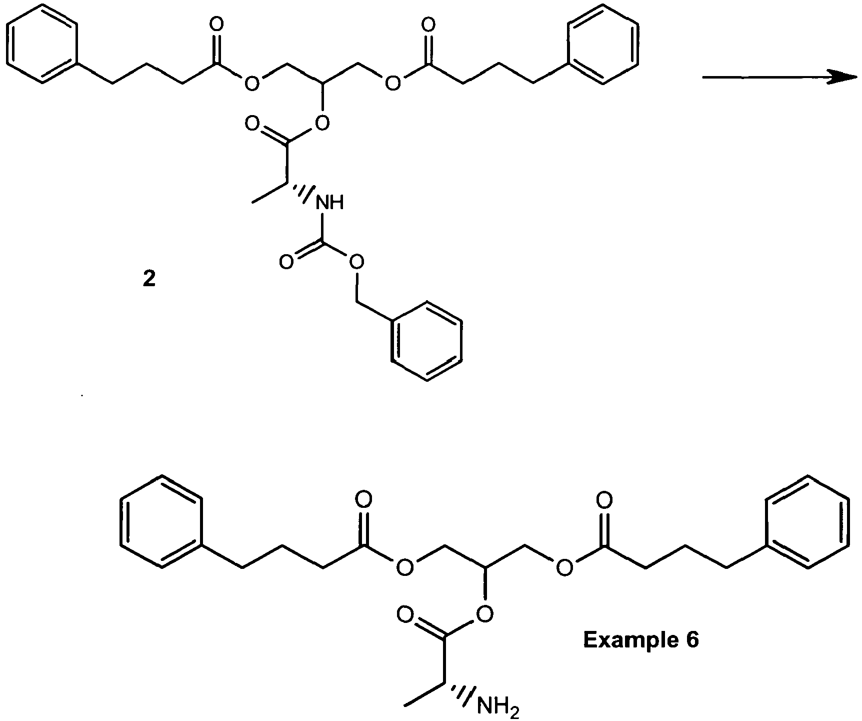

A suspension of 2 (1.96g) and Pd/C (5%Pd) (196mg) in THF (19.6m1) was stirred under H2-atmosphere for 4h. After completion of the reaction, the Pd/C was removed by filtration and was concentrated under reduced pressure. **Example 6** (1.4g, 3.1 mmol, 100%) was obtained as colorless oil.
1HNMR in CDCl3
7.35-7.15(m, 10H), 5.22 (m, 1H), 4.35-4.16 (m, 4H), 3.41 (m, 1H), ), 2.65, (m, 4H), 2.31 (m, 4H), 1.85-1.68 (m, 6H), 1.10 (d, 3H, j=7.0). M+H= 456, M+Na= 478.

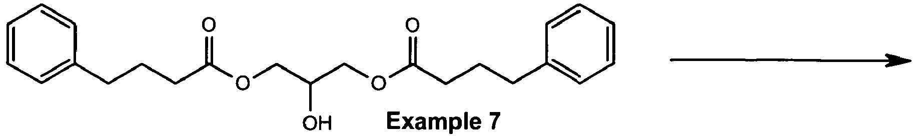

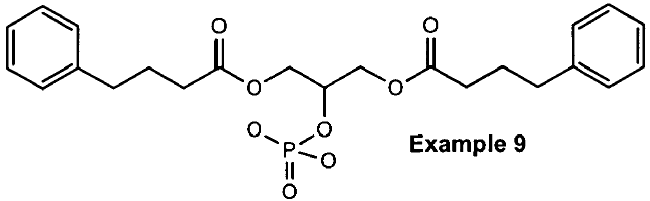

To a solution of POCl3 (1ml) in 19 ml THF was added pyridine (3.78ml) dissolved in 47 ml THF at 0°. To the resulting mixture was added Example 7 (3g) dissolved in 56mi THF drop wise at 0°. After 1 h the cooling bath was removed, and the reaction mixture was stirred for 16h at room temperature. After dilution with DCM the organic Phase was washed with water. The organic layer was dried with NaSO4 and concentrated under reduced pressure. The crude product was purified by chromatography (CHCl3/MeOH/H2O = 6.5/3.5/0.5) to give Example 9 (1.6g, 3.4mmol, 46%) as colorless wax.
1HNMR in DMSO:
7.35-7.15(m, 10H), 4.35 (m, 1H), 4.19-4.01 (m, 4H), 2.58, (m, 4H), 2.31 (m, 4H), 1.90 (m, 4H). (M-H2P04)⁺= 367.

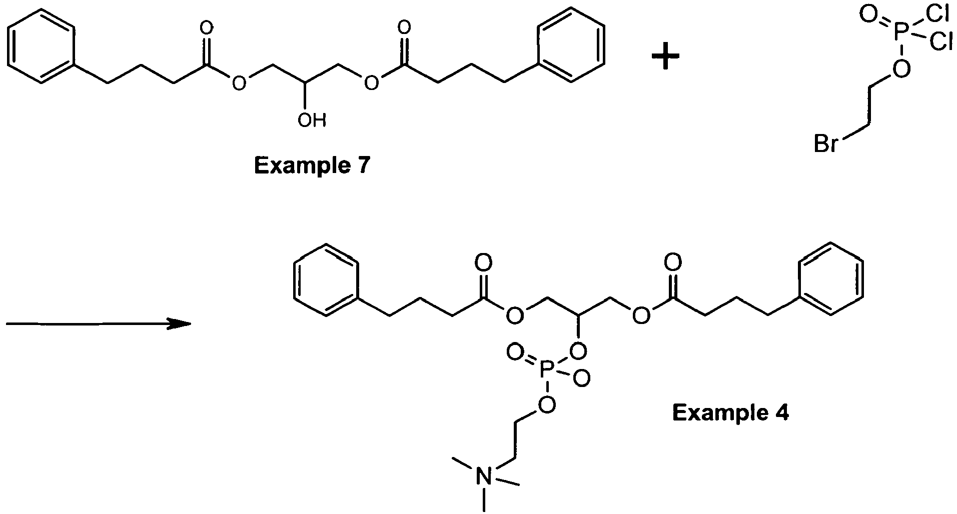

To a solution of 2-Bromethyldichlorphosphate (5.3g) in 200ml THF was added 30ml pyridine dissolved in 40 ml THF drop wise at 0°. To the resulting mixture was added Example 7 (3g) dissolved in 40ml THF drop wise at 0°. Stirring was continued for 16 h at rt. Hydrolysis was obtained by addition of 78 ml ice water. Then the solvent was removed by evaporation. The residue was dissolved in DCM and extracted with water. The organic layer was dried with NaSO4 and concentrated under reduced pressure. The crude product was used without further purification. It was dissolved in 10 ml pyridine and 25 ml Trimethylamin solution in water (44%) was added and stirred at room temperature for 18h. After dilution with DCM the organic Phase was washed with water. The organic layer was dried with NaSO4 and concentrated under reduced pressure. The crude product was purified by chromatography (CHCl3/MeOH/H2O = 6.5/3.5/0.5) to give **Example 4** (0.65g, 1.2mmol, 15%) as colorless oil.
1H NMR in DMSO:
7.35-7.15(m, 10H), 4.35 (m, 1H), 4.19-4.01 (m, 6H),3.55 (m, 2H), 3.15 (s, 9H), 2.60, (m, 4H), 2.35 (m, 4H), 1.90 (m, 4H). M⁺= 551.

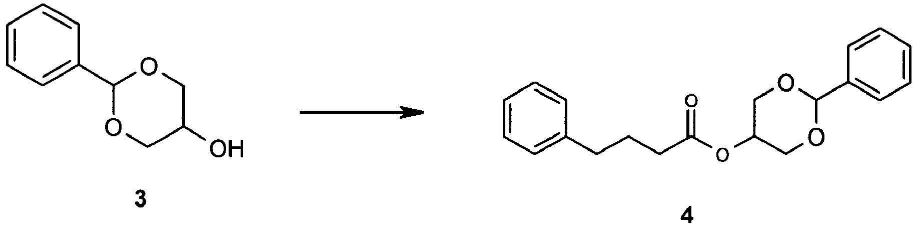

To a solution of **3** (11.2g) in pyridine (115ml) was added 4-Phenylbutanoylchloride (12.5g) dissolved in 12 ml THF at room temperature. The mixture was stirred for 16 h at rt. The solvent was removed under reduced pressure and the residue was dissolved in MTBE and washed with water, satd. NaHCO3 and satd. NaCl. The organic layer was dried with NaSO4 and concentrated under reduced pressure. The crude product was purified by chromatography (Heptane/EE = 9/1) to give **4** (9.7g, 29.8mmol, 46%) as colorless oil.
1H-NMR in CDCl3:
7.50-7.15(m, 10H), 5.43 (s, 1H), 5.00 (m, 1H), 4.41 (m, 2H), 3.68 (m, 2H), 2.63, (m, 2H), 2.38 (m, 2H), 2.00 (m, 2H). M+Na = 349.

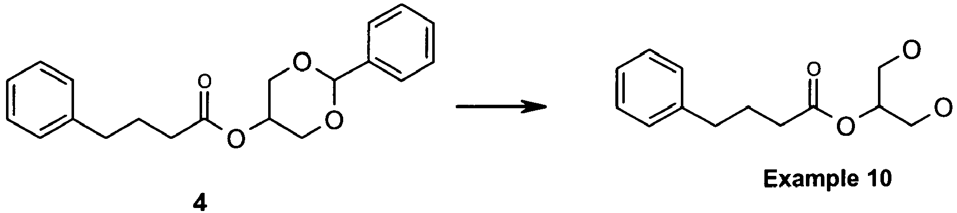

A suspension of **4** (3.93g) and Pd/C (5%Pd) (396mg) in THF (39.3ml) was stirred under H2-atmosphere. After completion of the reaction, the Pd/C was removed by filtration and was concentrated under reduced pressure. **Example 10** (2.41g, 10.1 mmol, 91%) was obtained as colorless oil.
1HNMR in CDCl3
7.35-7.15 (m, 5H), 4.95 (m, 1H), 3.81 (m, 4H), 2.66 (m, 2H), 2.40 (m, 4H), 1.99 (m, 2H).M+Na= 261.

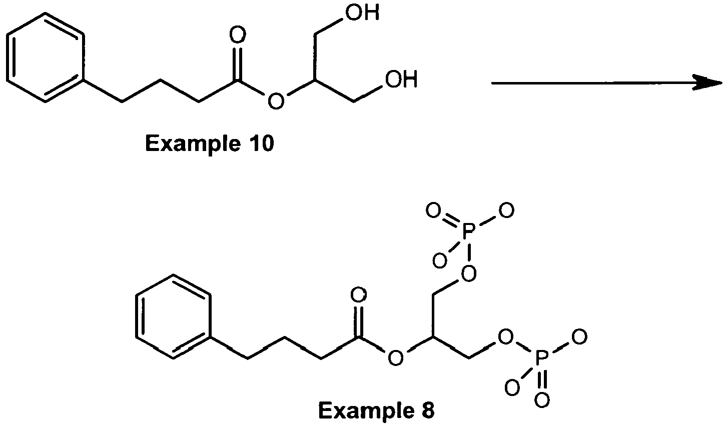

To a solution of **Example 10** (3.17g) and Dibenzyl N,N-diisopropylphosphoramidite (11.62g) was added Pyridinium-trifluoroacetat (6.3g) portion wise in 5 min. The solution was stirred for 2h at room temperature. After the starting material was consumed, the solution was cooled to -60°. 3-Chloroperbenzoic acid (14.9g) dissolved in DCM was added drop wise to the solution (20min). After 15 min at this temperature the cooling bath was replaced by an ice bath and the suspension was stirred for 2 h at 0°. The reaction was quenched with 10% Na2S2O3-Solution. The organic layer was separated and washed with 5% NaHCO3, water and satd. NaCl. The organic layer was dried with Na2SO4 and concentrated under reduced pressure. The crude product was purified by chromatography (Tol/EE = 2/1) to give the intermediate benzylester (9.5g, 12.5 mmol, 94%) as colorless oil. 1H-NMR inCDCl3: 7.05-7.30 (m, 25H), 5.11-4.99 (m, 9H), 4.12-3.95 (m, 4H), 2.60 (m, 2H), 2.32 (m, 2H), 1.85 (m, 2H). M+H= 759.

The intermediate (500mg) was dissolved in 2 ml THF and 2 ml MeOH and 50 mg Pd/C (5%) was added. After 1 h the reaction was finished. The catalyst was filtered off and the solvent was removed under reduced pressure. The residue was dissolved in 0.1 N ammoniumacetate solution and was extracted with DCM. The aqueous layer was lyophilized to give the tetra-ammonium salt of **Example 8** (233mg, 0.5mmol, 76%) as colorless oil.
1HNMR in DMSO:
7.35-7.18 (m, 5 H), 5.0 (m, 1H), 3.95 (m, 4 H), 2.60 (m, 2 H), 2.38 (m, 2 H), 1.80 (m, 2 H). (M-P04)⁺= 300, M+4H+Na=421.

### Example 11: Synthesis of 4-Phenyl-butyric acid 3-(2-amino-propionyloxy)-2-hydroxypropyl ester (Example 11)

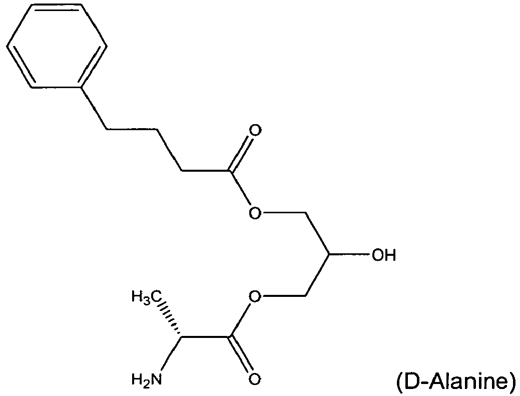

### Example 12: Synthesis of 4-Phenyl-butyric acid 2-(2-amino-propionyloxy)-1-(2-amino-propionyloxymethyl)-ethyl ester (Example 12)

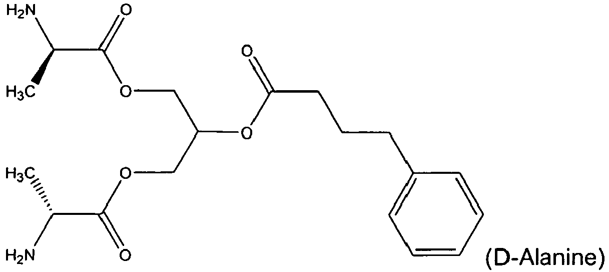

### Example 13: Synthesis of 4-Phenyl-butyric acid 2-({2-(2-amino-propionyloxy)-3-[(2,3-dihydroxy-propoxy)-hydroxy-phosphoryloxy]-propoxy}-hydroxy-phosphoryloxy)-3-(4-phenyl-butyryloxy)-propyl ester (Example 13)

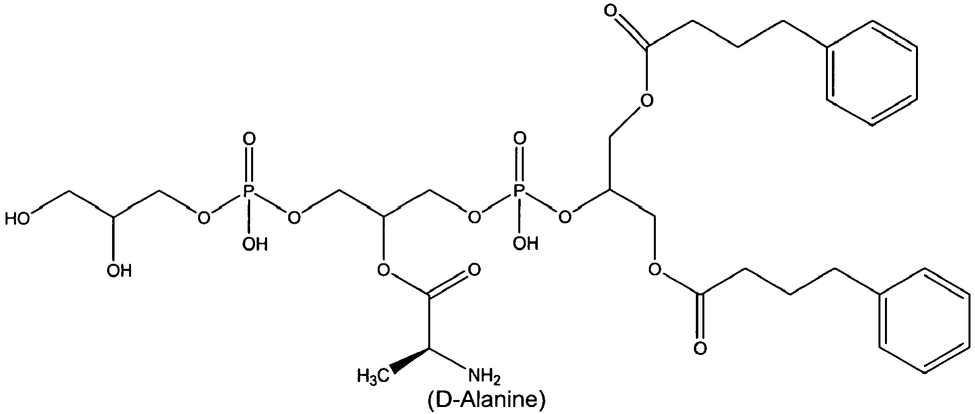

### Example 14: Synthesis of 4-Phenyl-butyric acid 2-({2-(2-amino-propionyloxy)-3-[(2-dimethylamino-ethoxy)-hydroxy-phosphoryloxy]-propoxy}-hydroxy-phosphoryloxy)-3-(4-phenyl-butyryloxy)-propyl ester (Example 14)

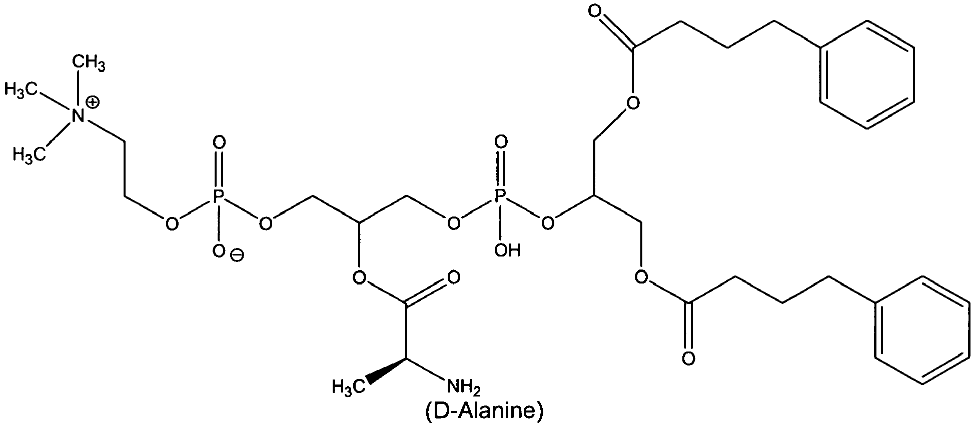

### Example 15: Synthesis of 4-Phenyl-butyric acid 2-(2-amino-propionyloxy)-3-(4-phenyl-butyryloxy)-propyl ester (Example 15)

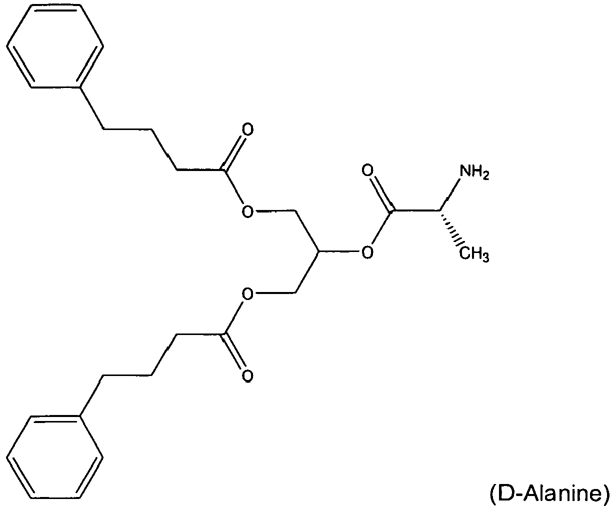

### Example 16: Synthesis of 4-Phenyl-butyric acid 3-(2-amino-propionyloxy)-2-phosphonooxy-propyl ester (Example 16)

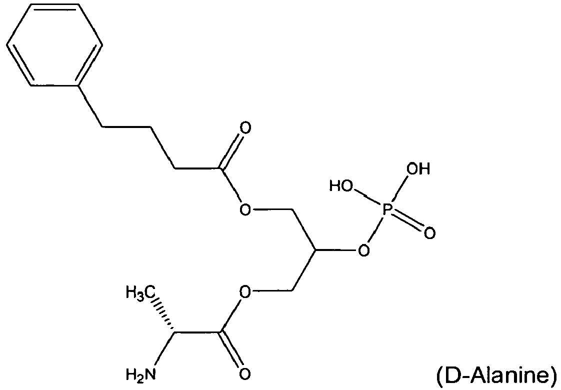

## Claims

1. Glycerol Phenyl Butyrate Ester of General Formula I 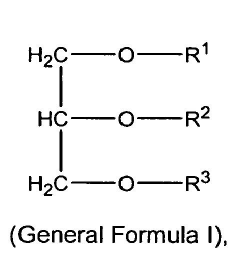 wherein
one of R¹, R² or R³ is 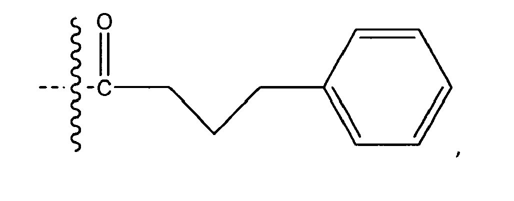 while the two others are independently of one another selected from
hydrogen, a radical of an amino acid, a radical of a D-amino acid, or any of the following structures, which may optionally be substituted by one or more of F, Cl, Br, I, NH₂, OH, SH, CH₃, C₂H₅, C₃H₇, C₄H₉, CH₂Cl, CHCl₂, CF₃, CHF₂, CH₂F: 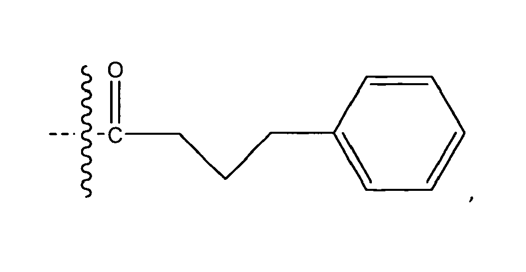 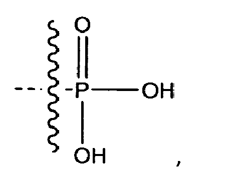 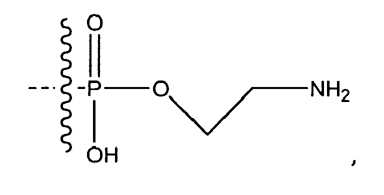 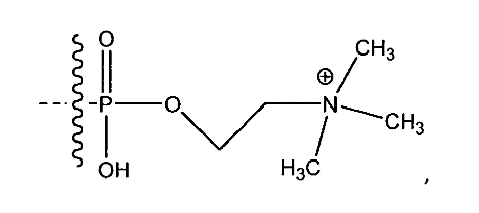 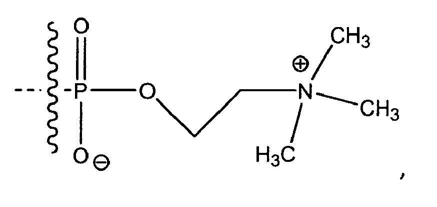 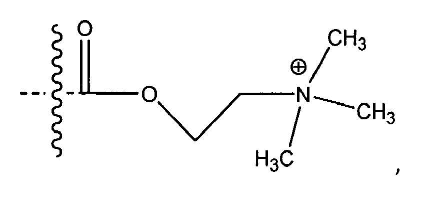 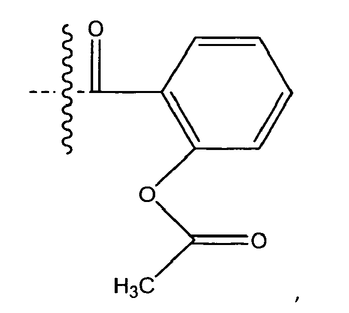 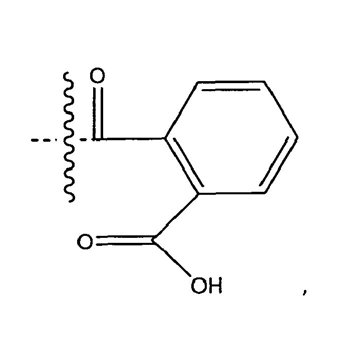 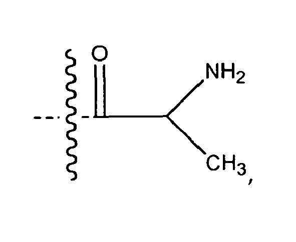 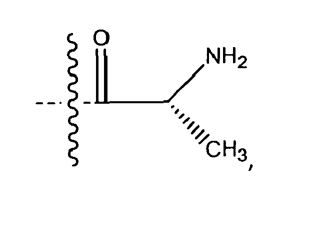 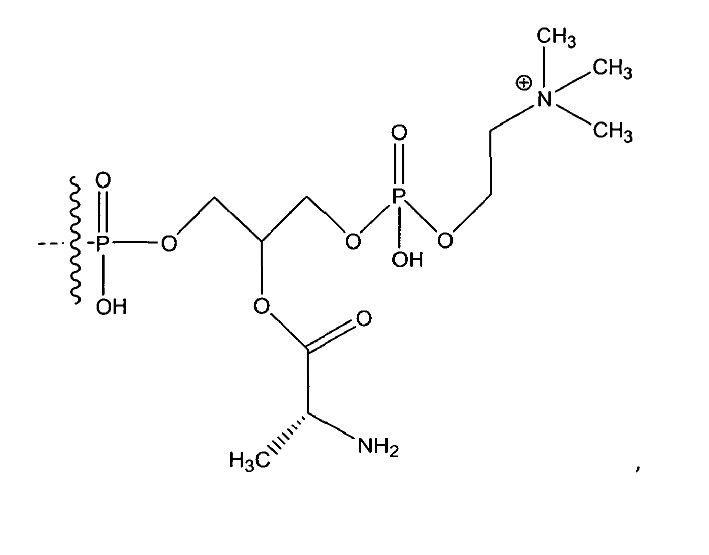 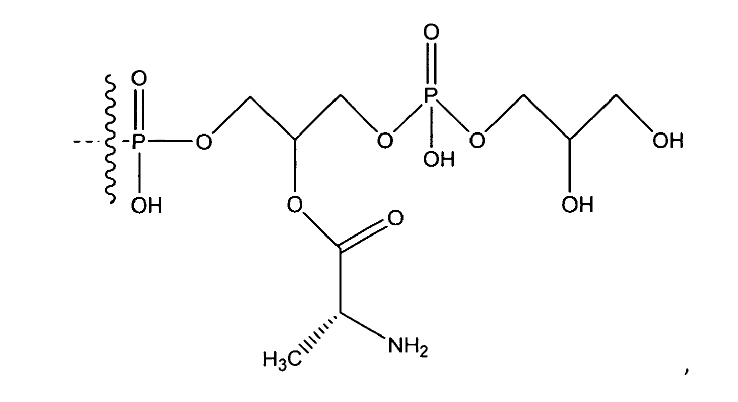 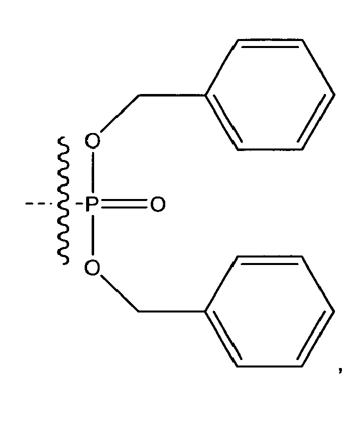 or 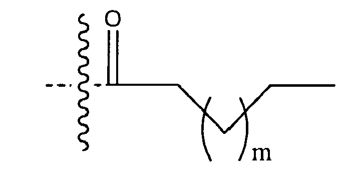 with m being 6, 7, 8, 9, 10, 11, 12, 13, 14, or 15, optionally unsaturated,
optionally in form of one of its stereoisomers, enantiomers or diastereomers, its racemate or in form of a mixture of at least two of its stereoisomers in any mixing ratio;
in form of the free acid, free base or salt;
with the proviso that not all three of R¹, R² and R³ may be

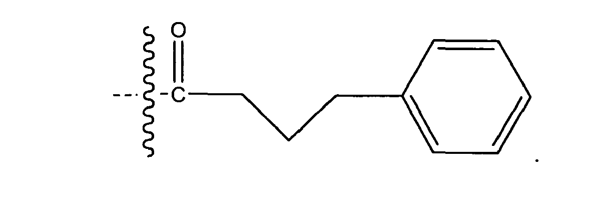

2. Glycerol Phenyl Butyrate Ester of General Formula I according to claim 1, wherein
one of R¹, R² or R³ is 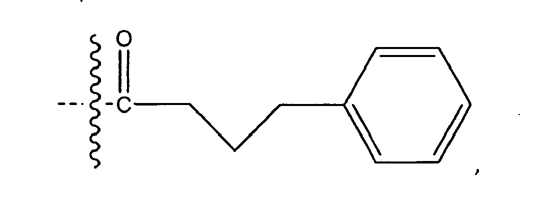 while the two others are independently of one another selected from
hydrogen, 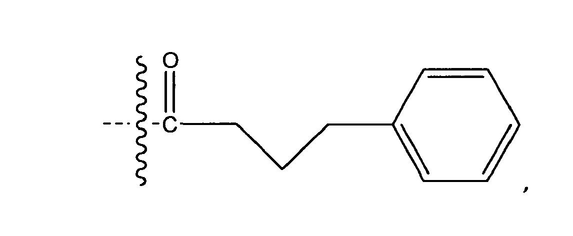 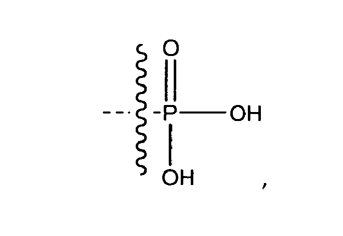 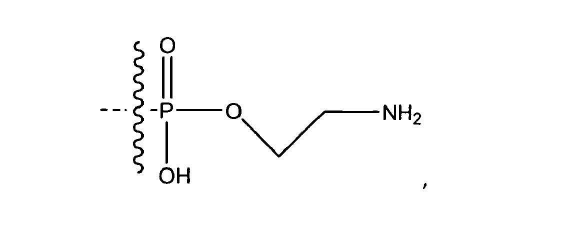 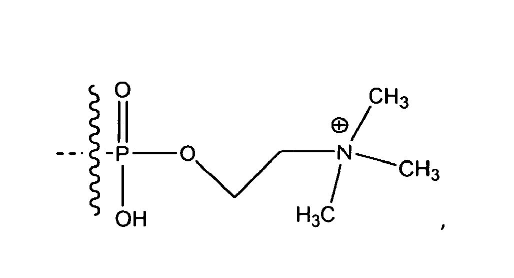 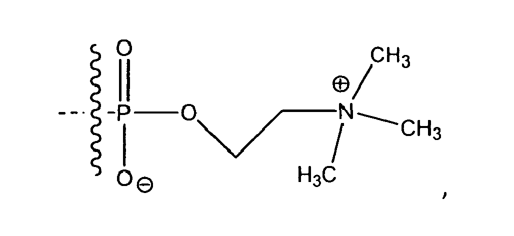 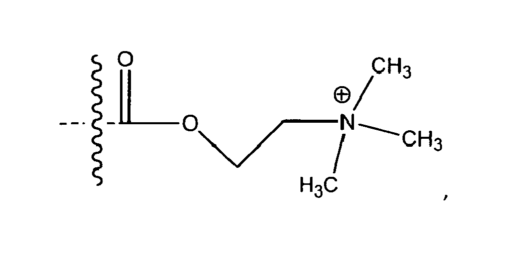 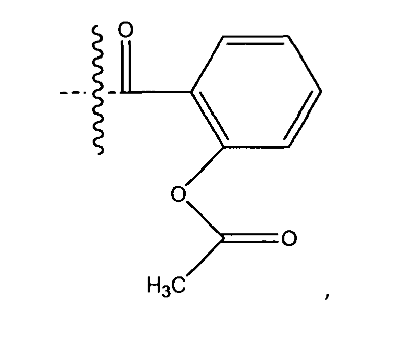 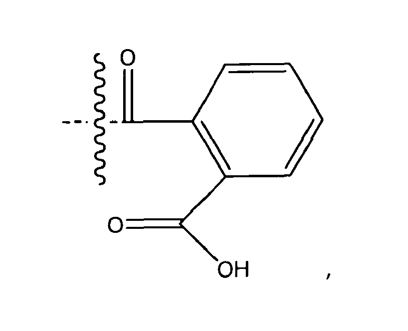 a radical of an amino acid, 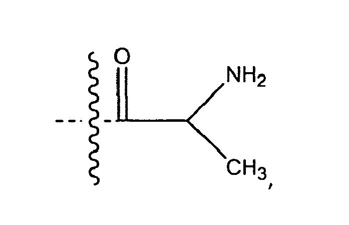 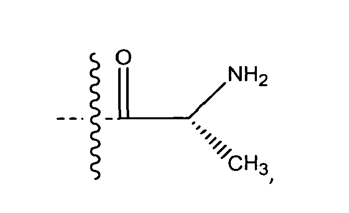 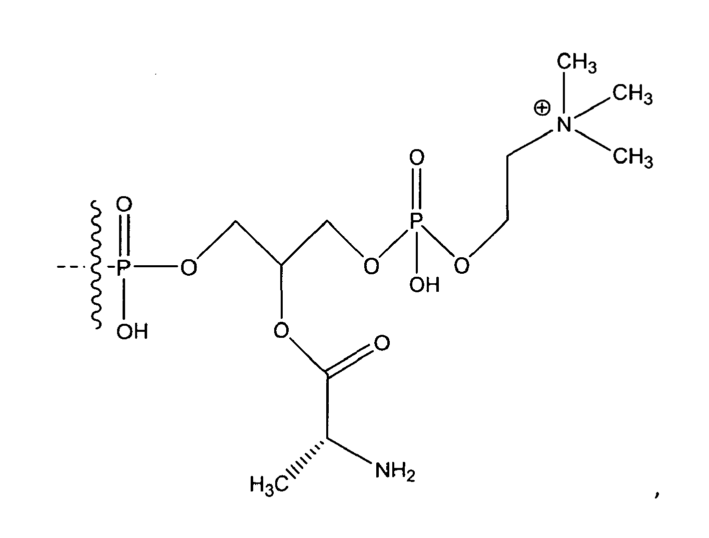 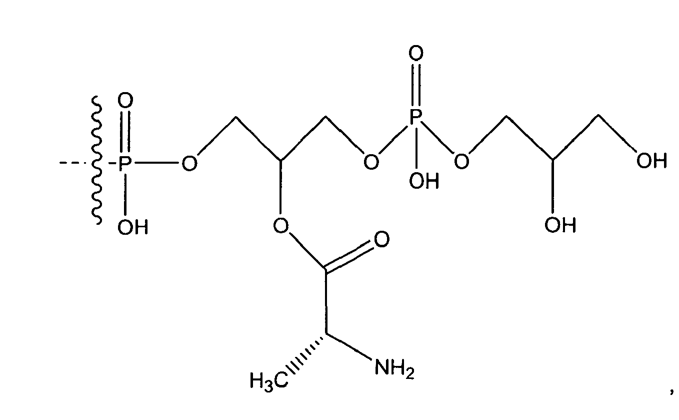 or 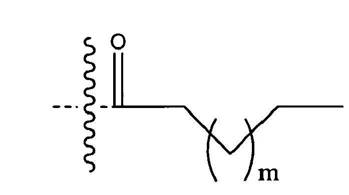 with m being 6, 7, 8, 9, 10, 11, 12, 13, 14, or 15, optionally unsaturated.

3. Glycerol Phenyl Butyrate Ester of General Formula I according to claim 1, wherein one of R¹, R² or R³ is 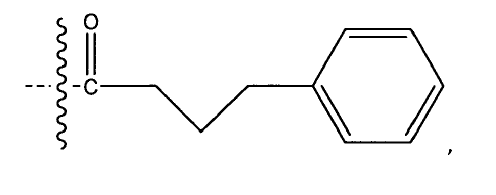 while the two others are independently of one another selected from
hydrogen, 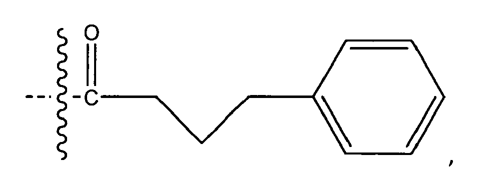 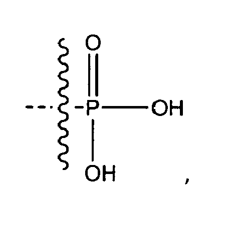 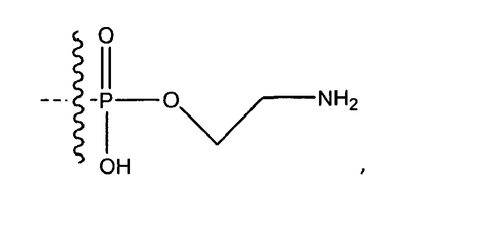 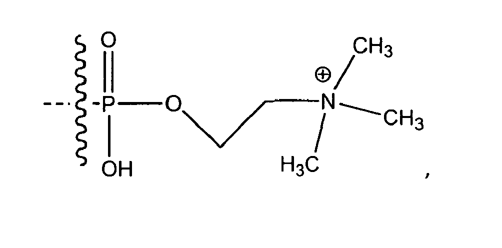 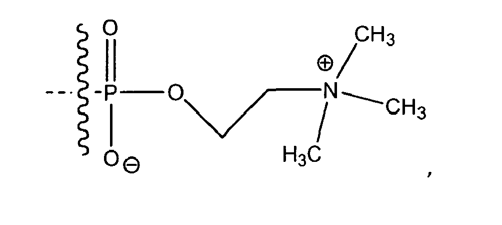 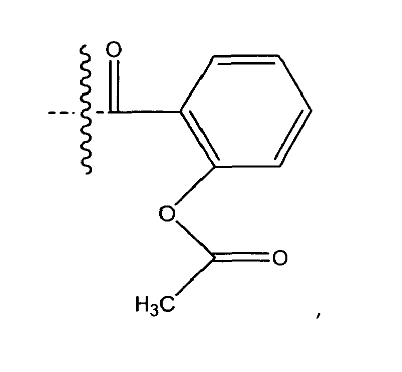 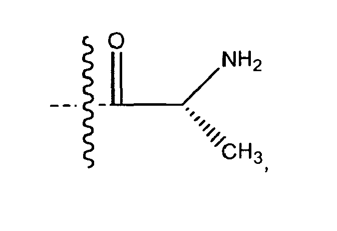 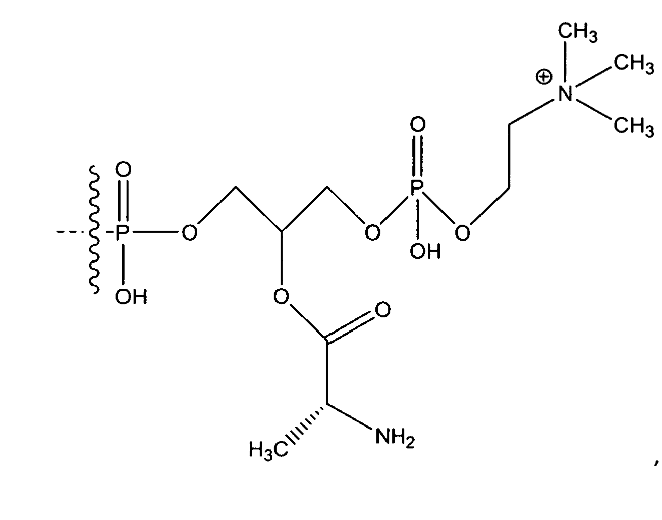 or

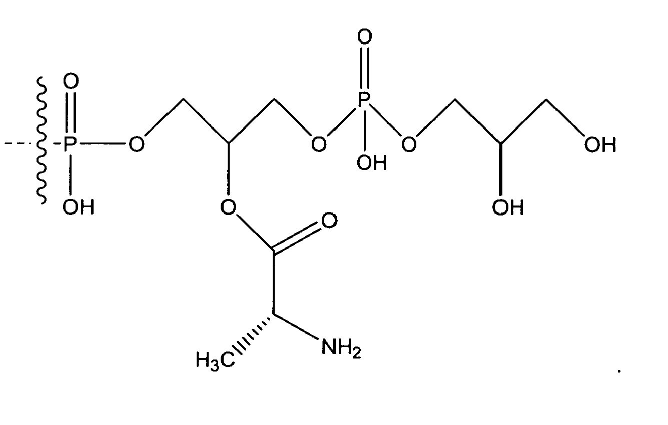

4. Glycerol Phenyl Butyrate Ester of General Formula I according to any of claims 1 to 3, wherein 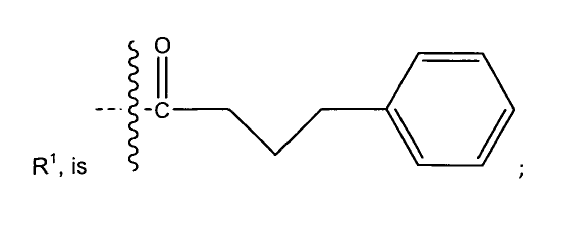 R² and R³ are independently of one another selected from
hydrogen or any of the following structures, which may optionally be substituted by one or more of F, Cl, Br, I, NH₂, OH, SH, CH₃, C₂H₅, C₃H₇, C₄H₉, CH₂Cl, CHCl₂, CF₃, CHF₂, CH₂F: 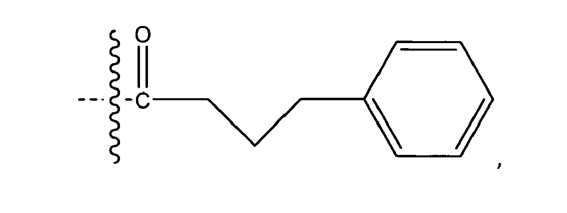 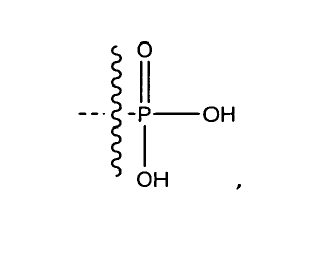 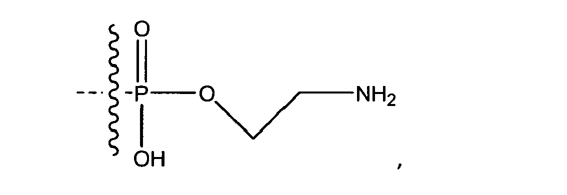 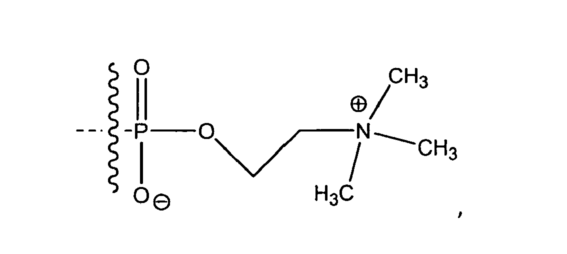 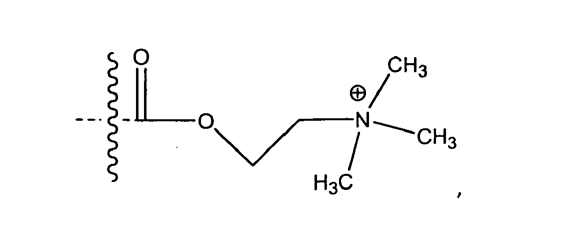 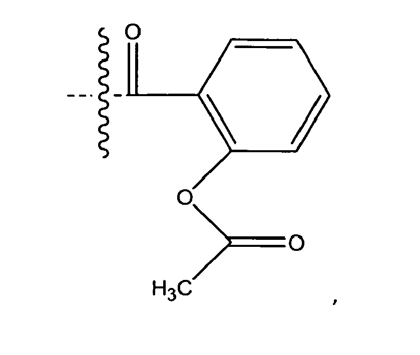 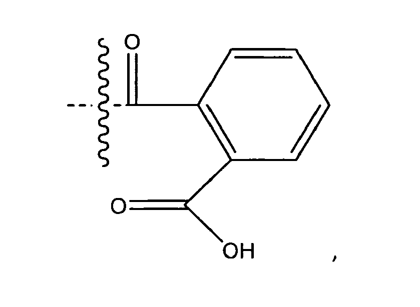 a radical of an amino acid, 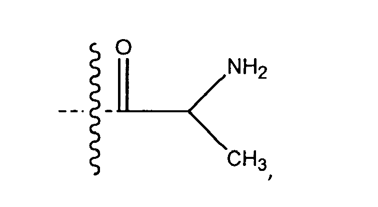 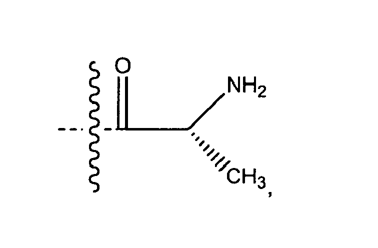 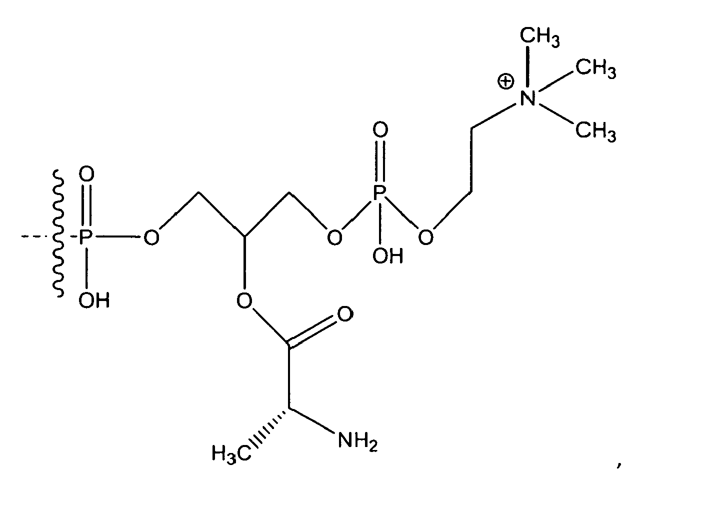 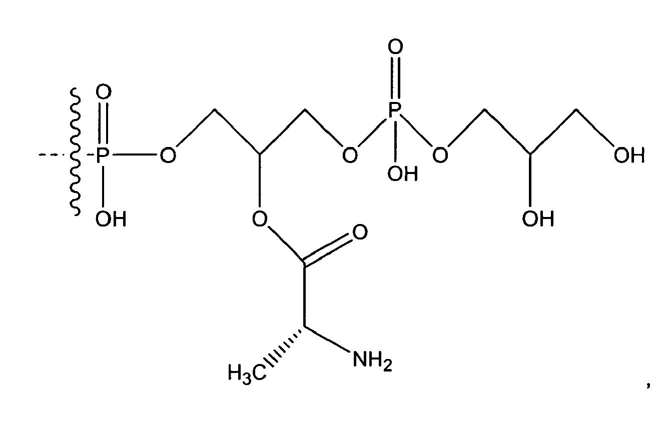 or 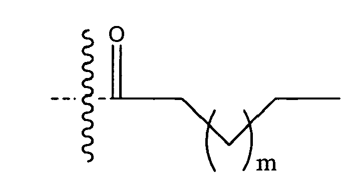 with m being 6, 7, 8, 9, 10, 11, 12, 13, 14, or 15, optionally unsaturated.

5. Glycerol Phenyl Butyrate Ester of General Formula I according to any of claims 1 to 4, wherein
two of R¹, R² and R³ are 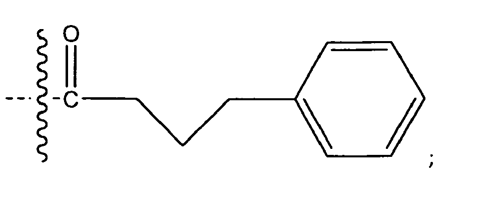 and the remaining is selected from
hydrogen or any of the following structures, which may optionally be substituted by one or more of F, Cl, Br, I, NH₂, OH, SH, CH₃, C₂H₅, C₃H₇, C₄H₉, CH₂Cl, CHCl₂, CF₃, CHF₂, CH₂F: 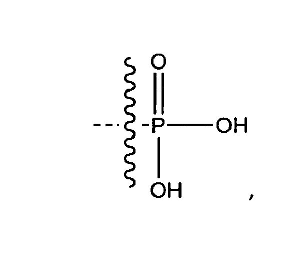 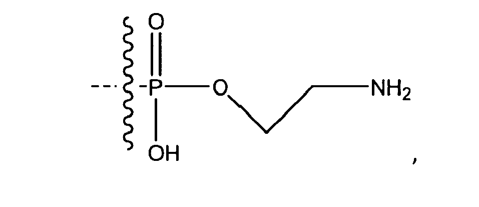 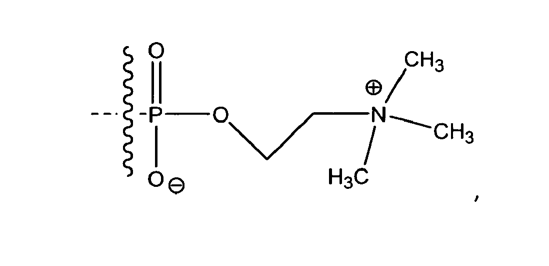 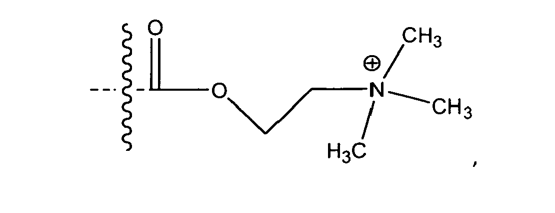 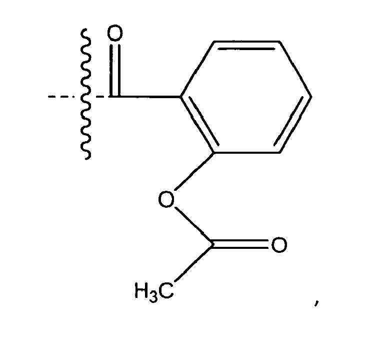 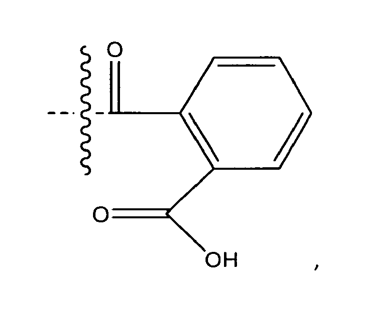 a radical of an amino acid, 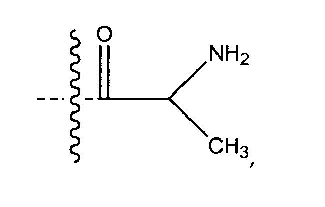 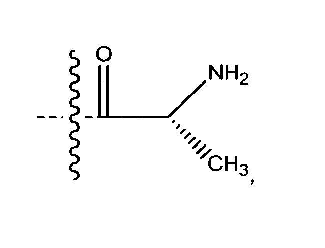 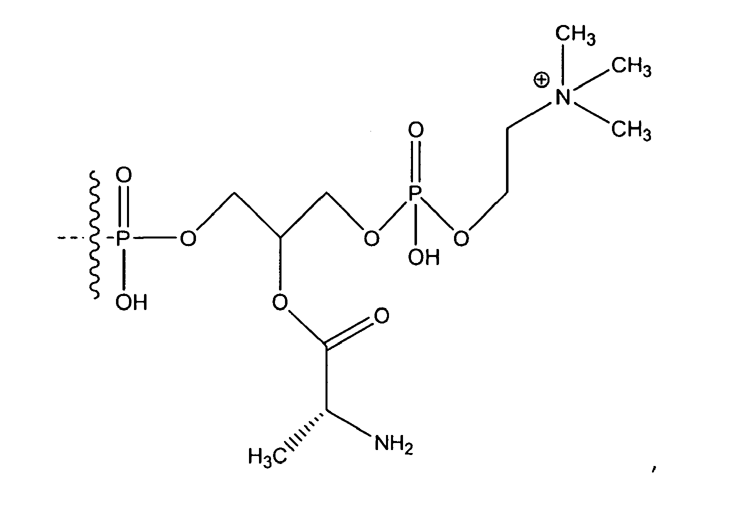 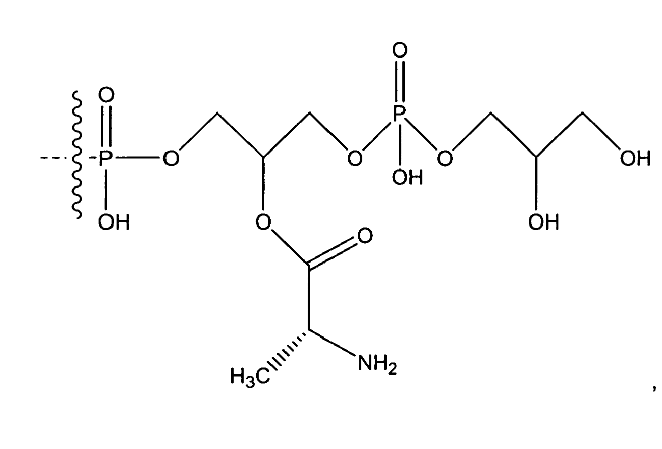 or 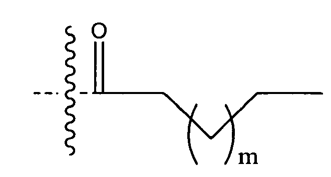 with m being 6, 7, 8, 9, 10, 11, 12, 13, 14, or 15, optionally unsaturated.

6. Glycerol Phenyl Butyrate Ester of General Formula I according to any of claims 1 to 4, wherein
R¹, is 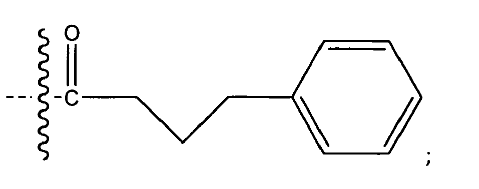 R² and R³ are independently of one another selected from
hydrogen or any of the following structures, which may optionally be substituted by one or more of F, Cl, Br, I, NH₂, OH, SH, CH₃, C₂H₅, C₃H₇, C₄H₉, CH₂Cl, CHCl₂, CF₃, CHF₂, CH₂F: 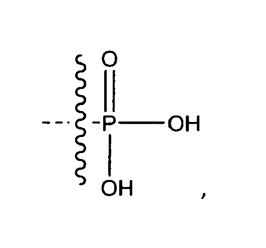 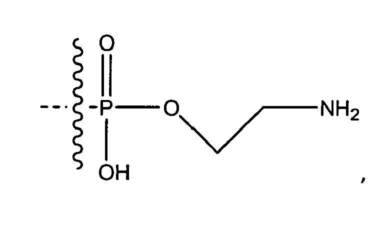 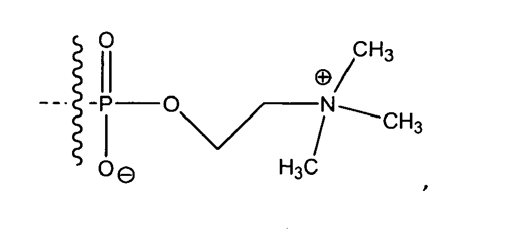 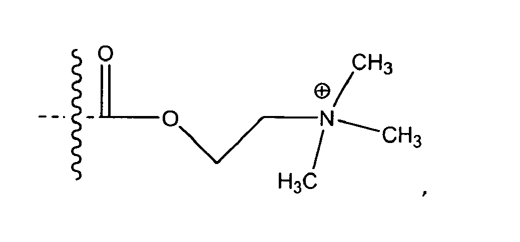 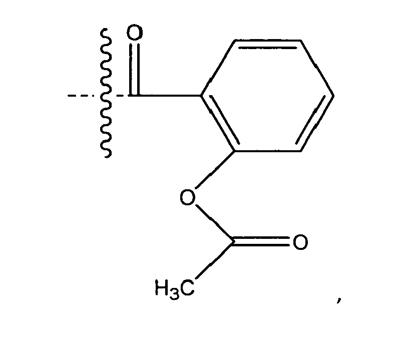 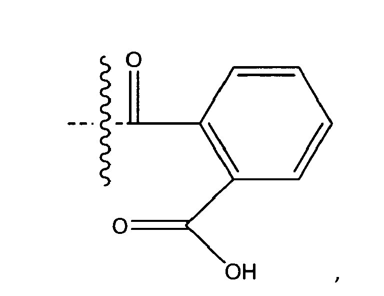 a radical of an amino acid, 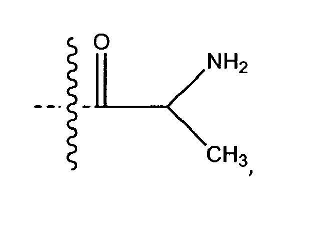 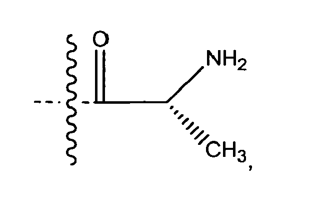 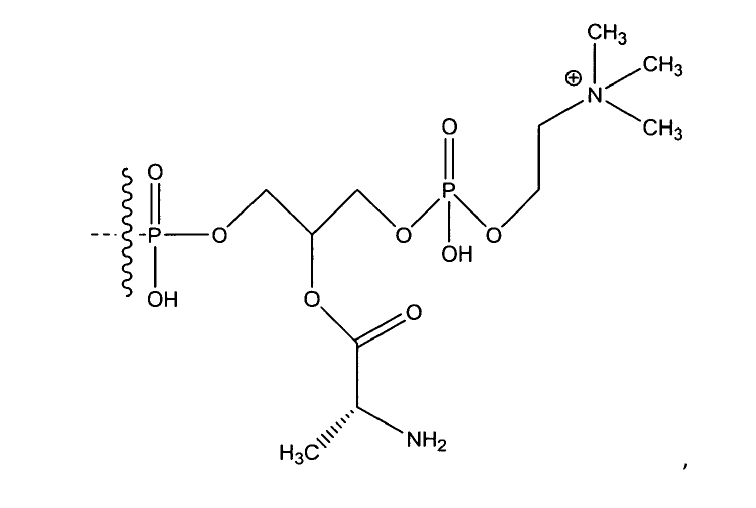 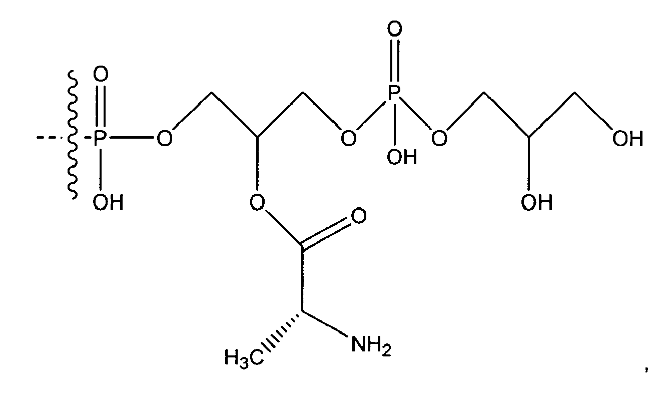 or 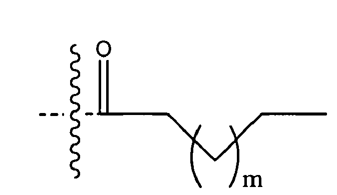 with m being 6, 7, 8, 9, 10, 11, 12, 13, 14, or 15, optionally unsaturated.

7. Glycerol Phenyl Butyrate Ester of General Formula I according to claim 6, wherein
R² is selected from
hydrogen or any of the following structures: 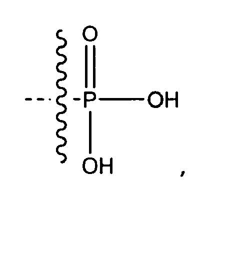 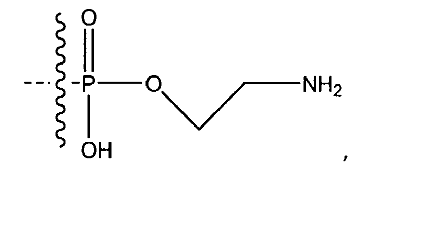 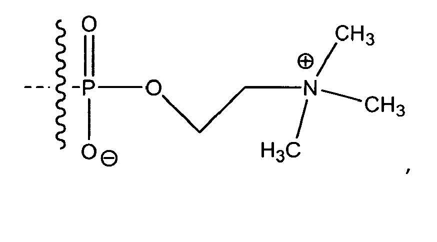 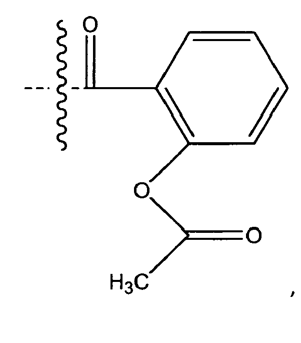 or 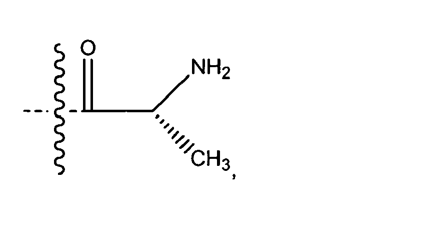 and
R³ is selected from 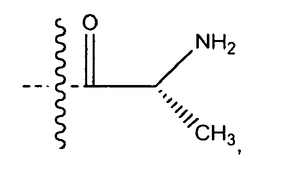 preferably wherein
R² is hydrogen
and R³ is 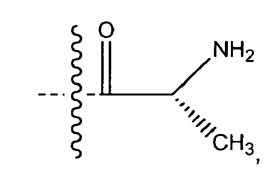 or
R² is 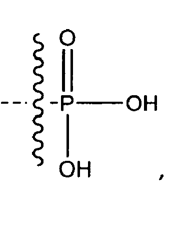 and R³ is 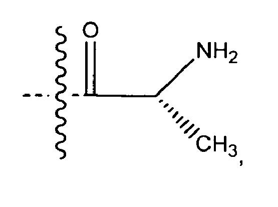 or
R² is 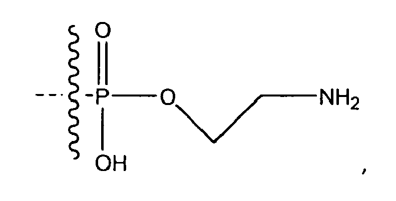 and R³ is... 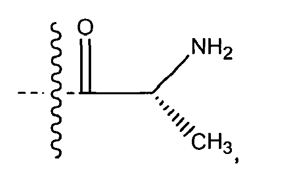 or
R² is 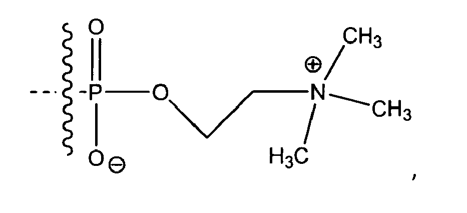 and R³ is... 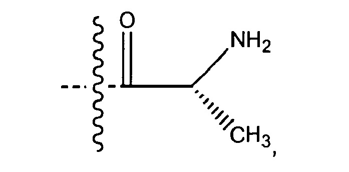 or
R² is 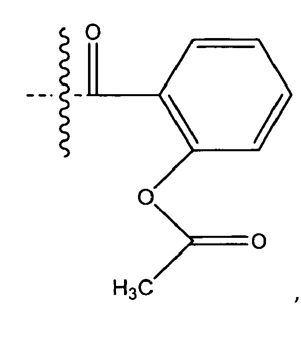 or
and R³ is... 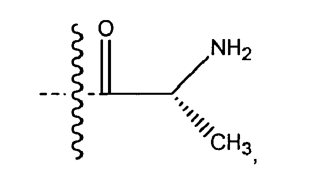 or
R² is 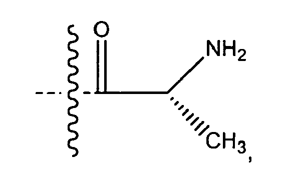 and R³ is...

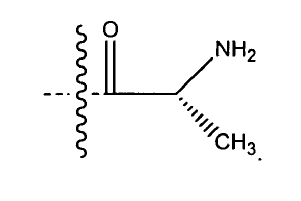

8. Glycerol Phenyl Butyrate Ester of General Formula I according to any of claims 1 to 5, wherein
R¹ and R³ are 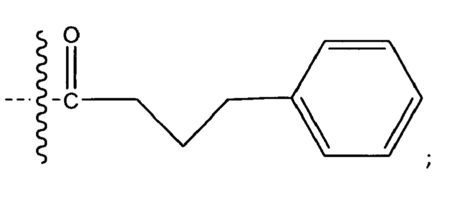 and R² is selected from
hydrogen or any of the following structures, which may optionally be substituted by one or more of F, Cl, Br, I, NH₂, OH, SH, CH₃, C₂H₅, C₃H₇, C₄H₉, CH₂Cl, CHCl₂, CF₃, CHF₂, CH₂F: 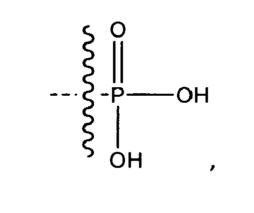 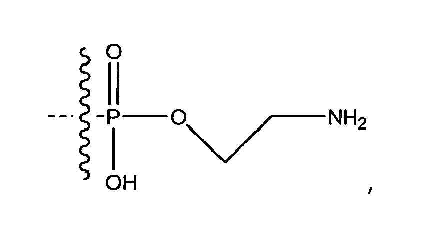 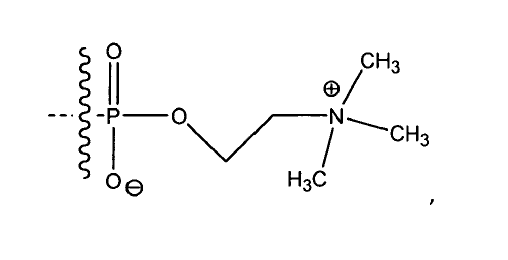 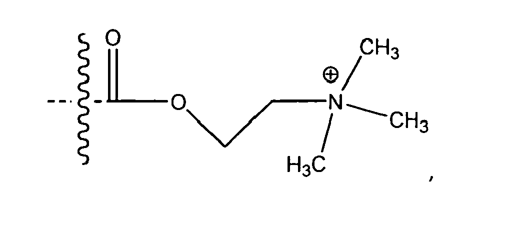 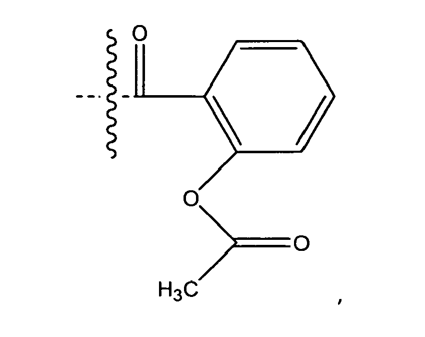 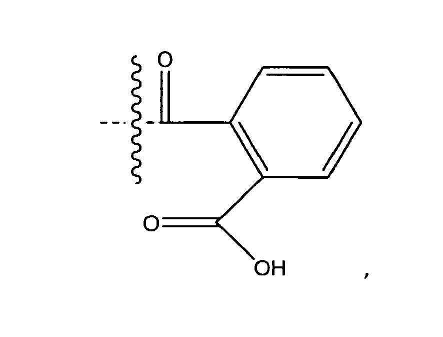 a radical of an amino acid, 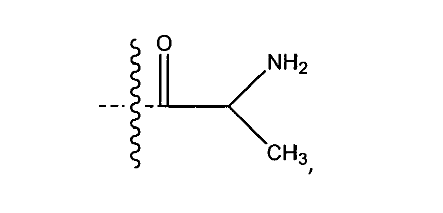 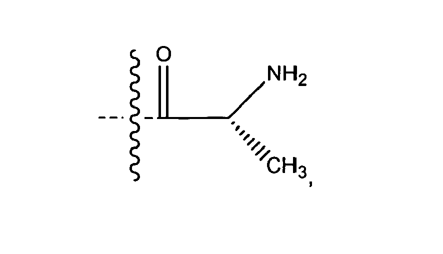 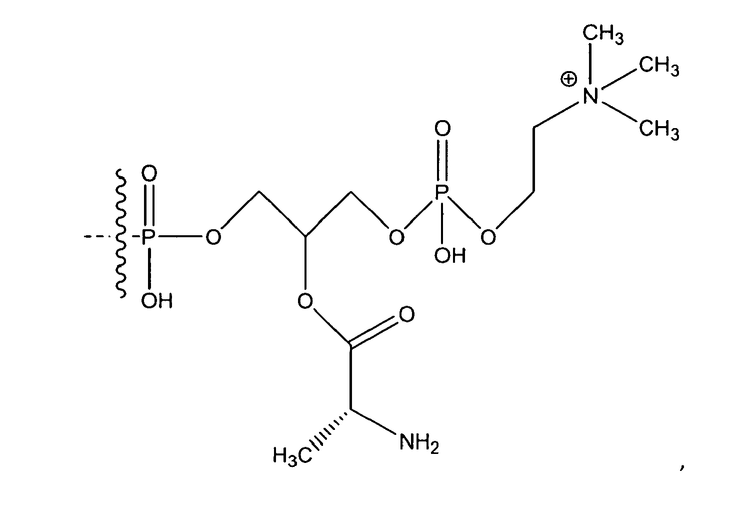 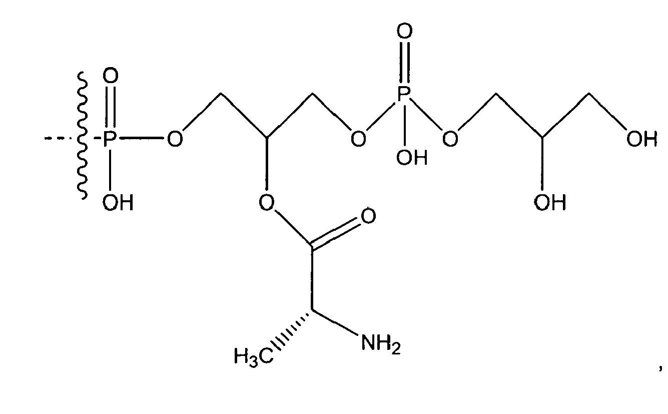 or 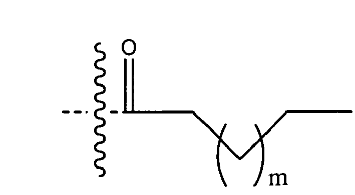 with m being 6, 7, 8, 9, 10, 11, 12, 13, 14, or 15, optionally unsaturated.

9. Glycerol Phenyl Butyrate Ester of General Formula I according to claim 8, wherein R² is selected from
hydrogen or any of the following structures, which may optionally be substituted by one or more of F, Cl, Br, 1, NH₂, OH, SH, CH₃, C₂H₅, C₃H₇, C₄H₉, CH₂Cl, CHCl₂, CF₃, CHF₂, CH₂F: 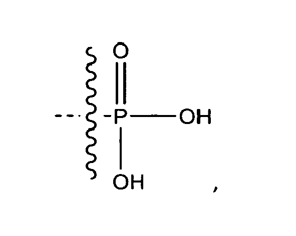 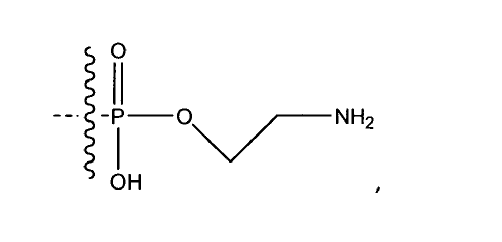 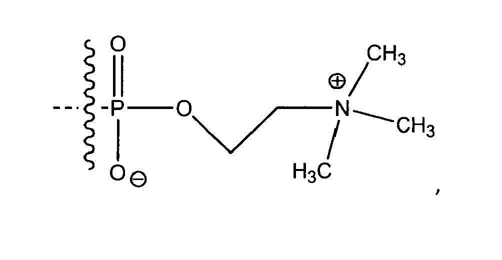 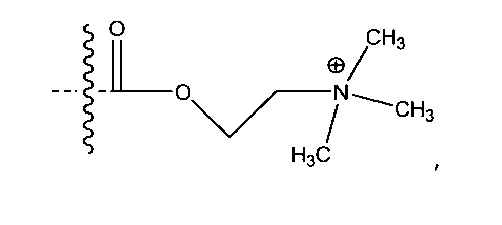 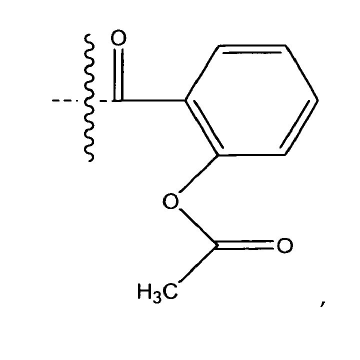 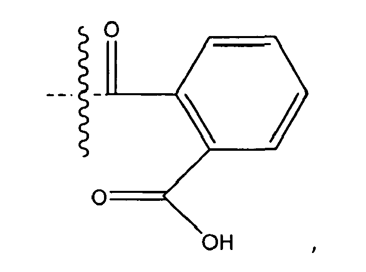 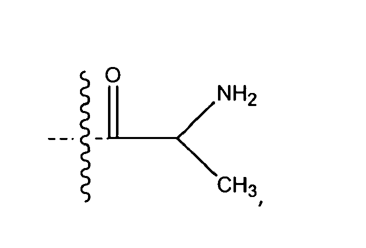 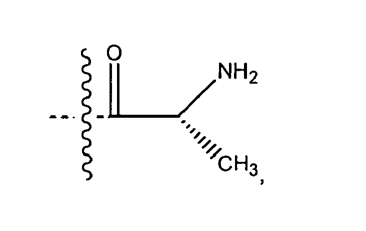 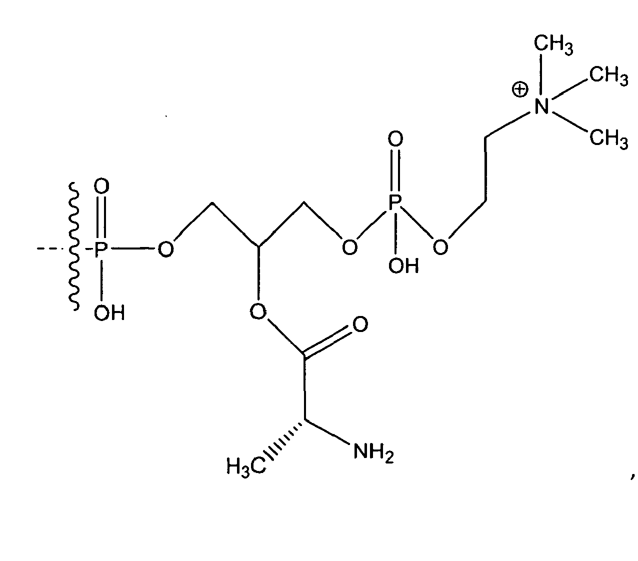 or

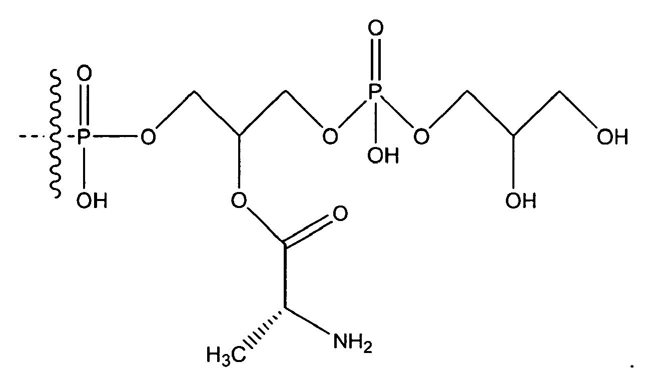

10. Glycerol Phenyl Butyrate Ester of General Formula I according to any of claims 1 to 3, wherein
R² is 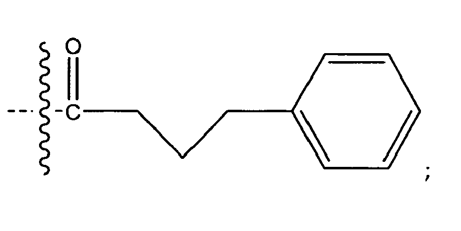 and R¹ and R³ independently from one another are selected from
hydrogen or any of the following structures, which may optionally be substituted by one or more of F, Cl, Br, I, NH₂, OH, SH, CH₃, C₂H₅, C₃H₇, C₄H₉, CH₂Cl, CHCl₂, CF₃, CHF₂, CH₂F: 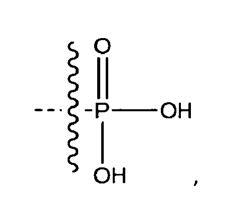 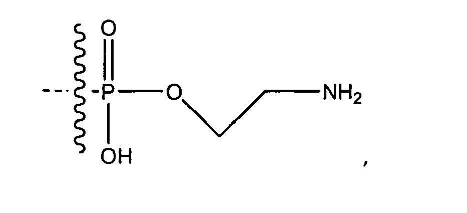 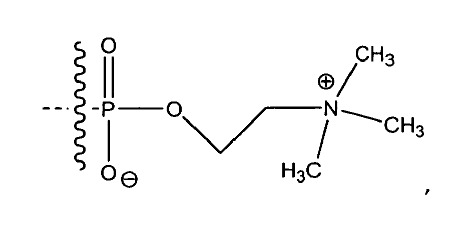 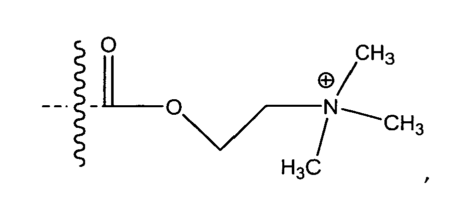 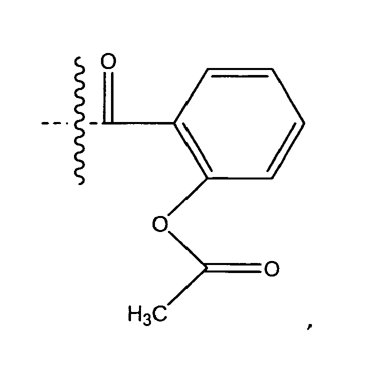 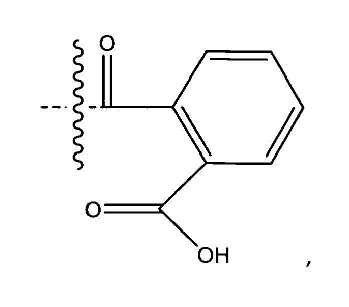 a radical of an amino acid, 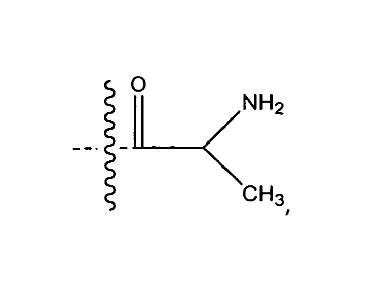 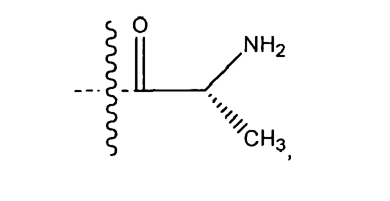 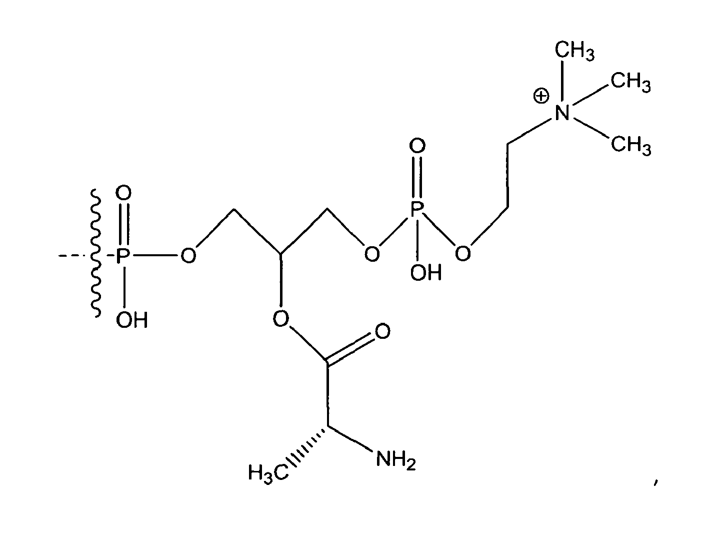 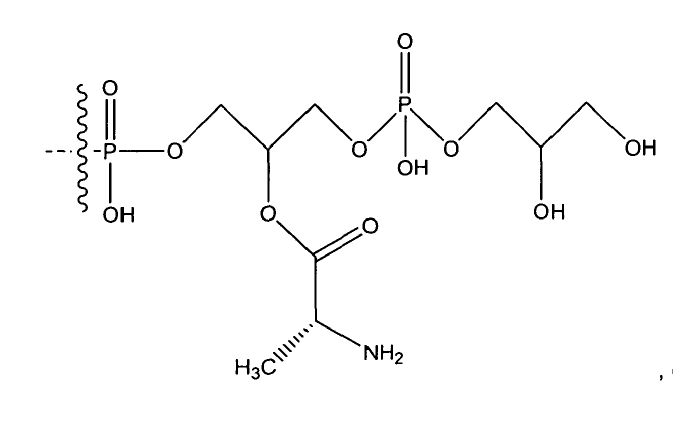 or 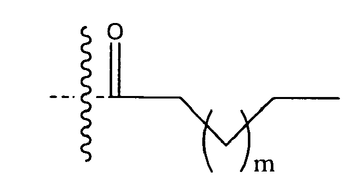 with m being 6, 7, 8, 9, 10, 11, 12, 13, 14, or 15, optionally unsaturated.

11. Glycerol Phenyl Butyrate Ester of General Formula I according to claim 10, wherein R¹ and R³ independently from one another are selected from
hydrogen or any of the following structures, which may optionally be substituted by one or more of F, Cl, Br, I, NH₂, OH, SH, CH₃, C₂H₅, C₃H₇, C₄H₉, CH₂Cl, CHCl₂, CF₃, CHF₂, CH₂F: 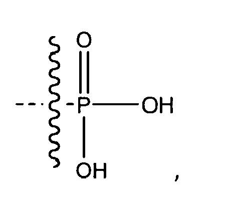 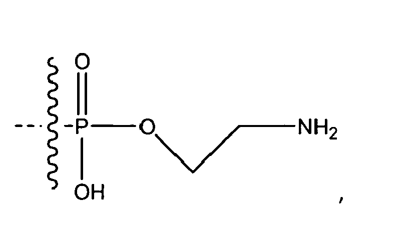 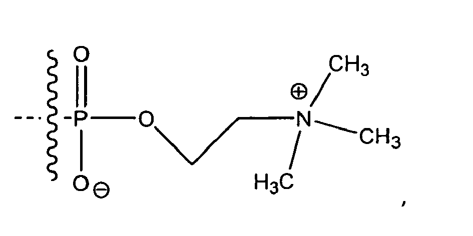 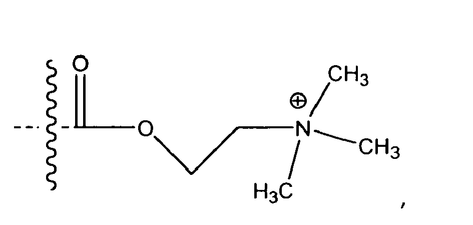 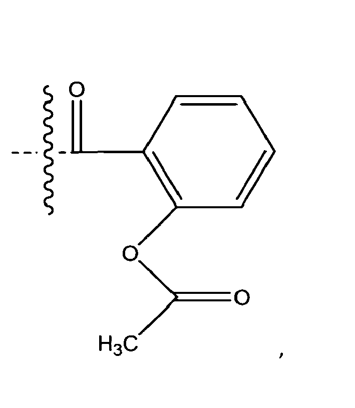 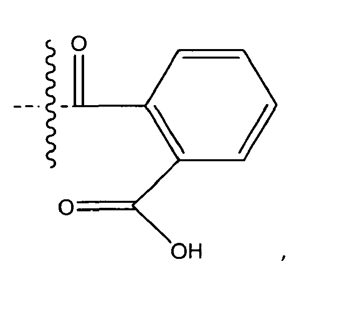 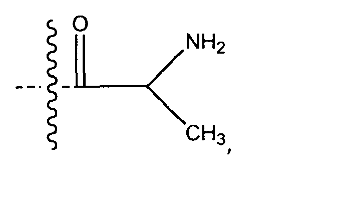 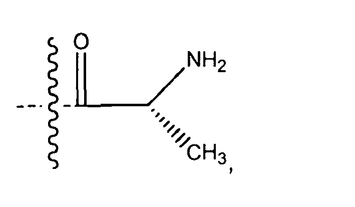 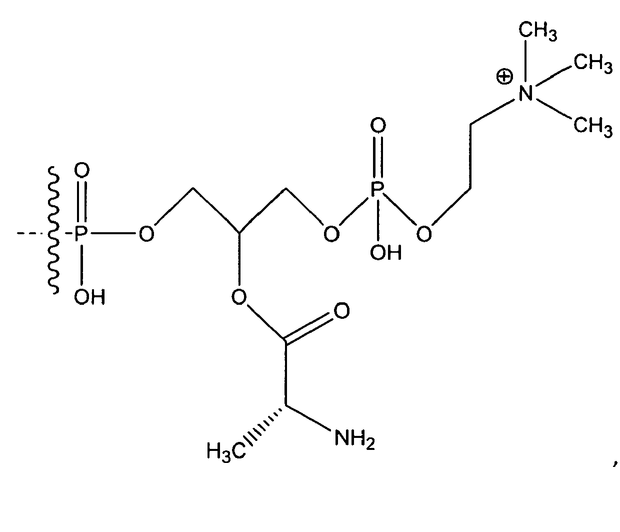 or

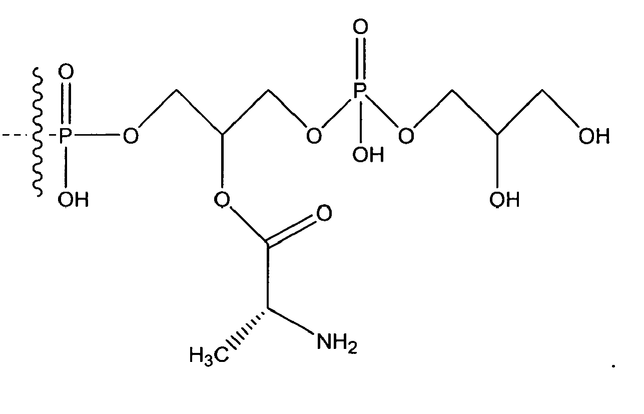

12. Glycerol Phenyl Butyrate Ester according to claim 1 selected from 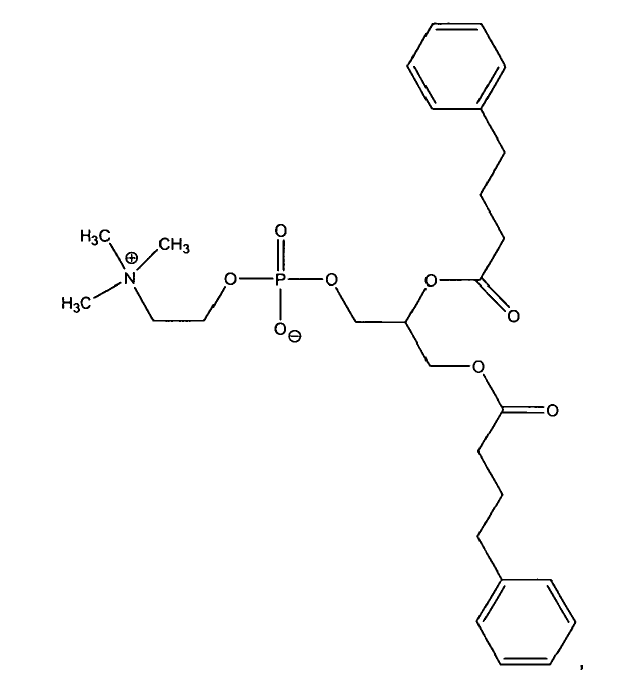 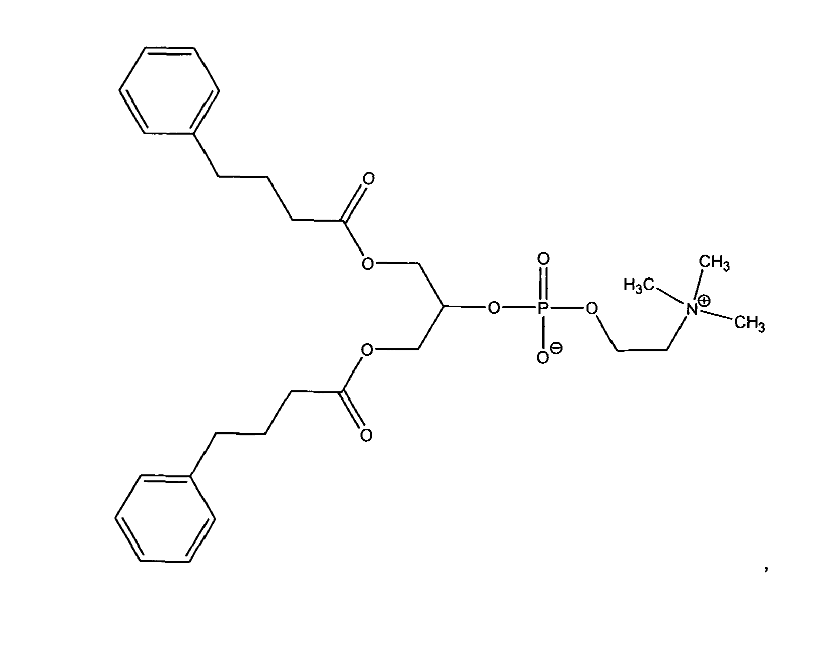 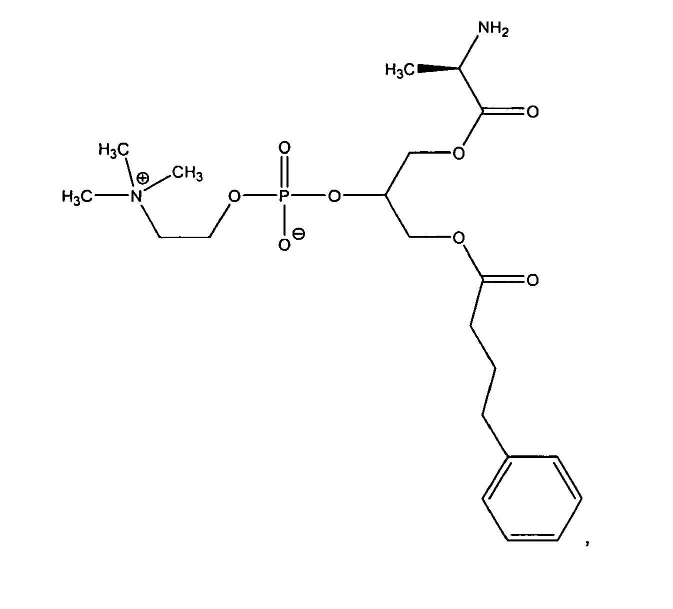 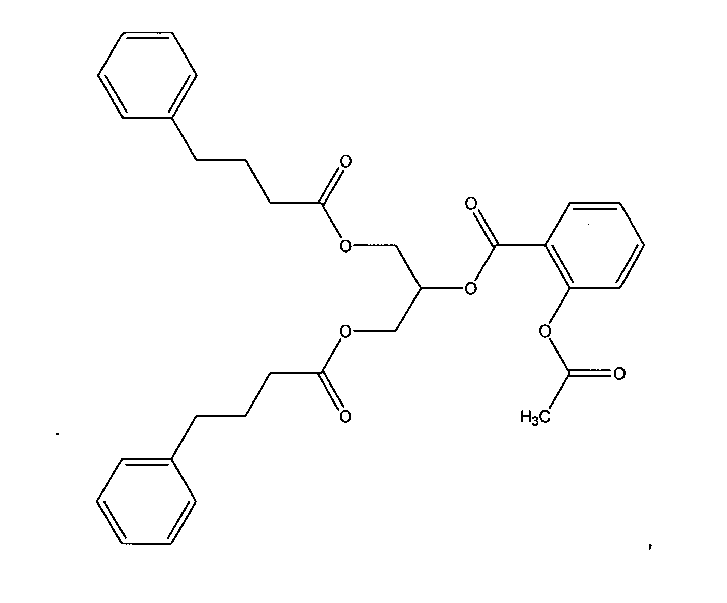 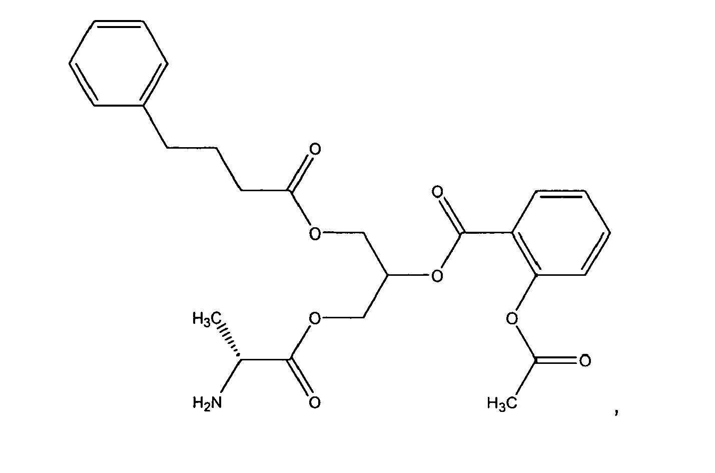 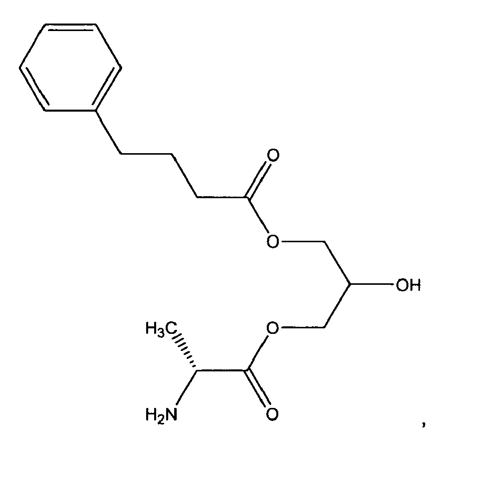 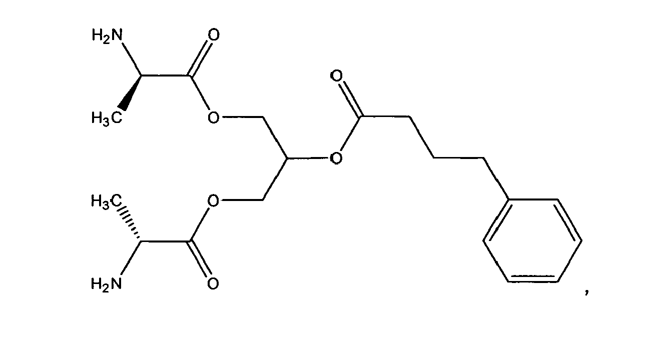 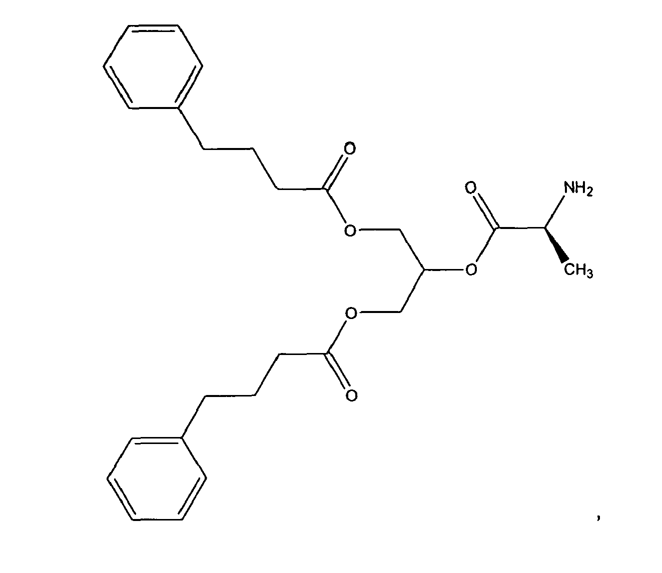 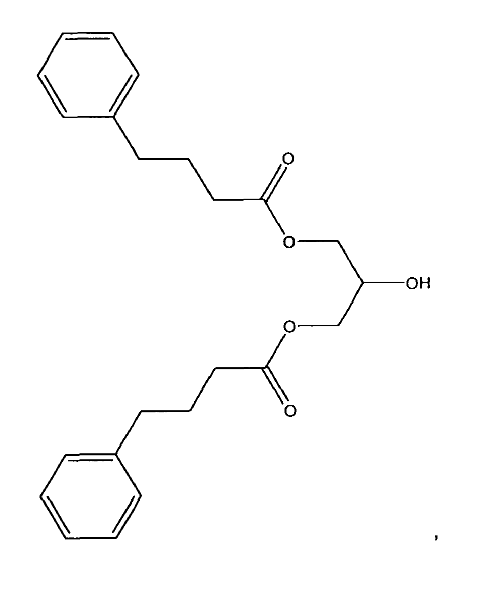 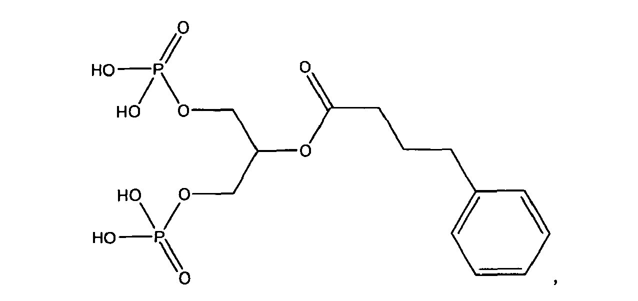 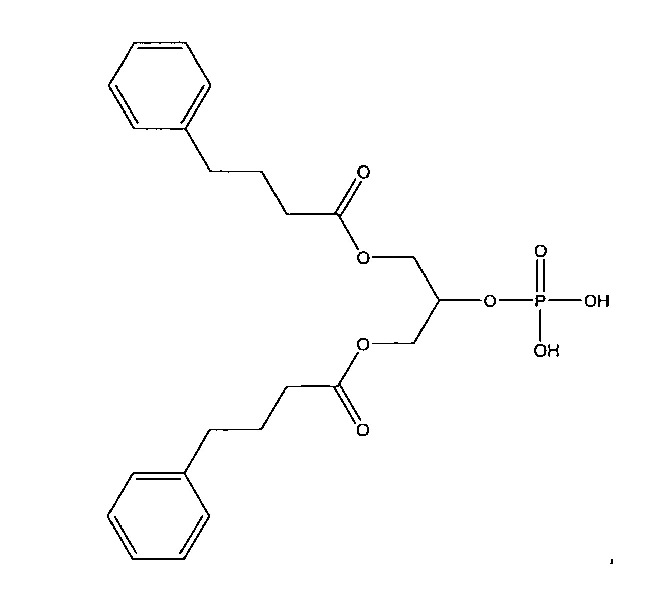 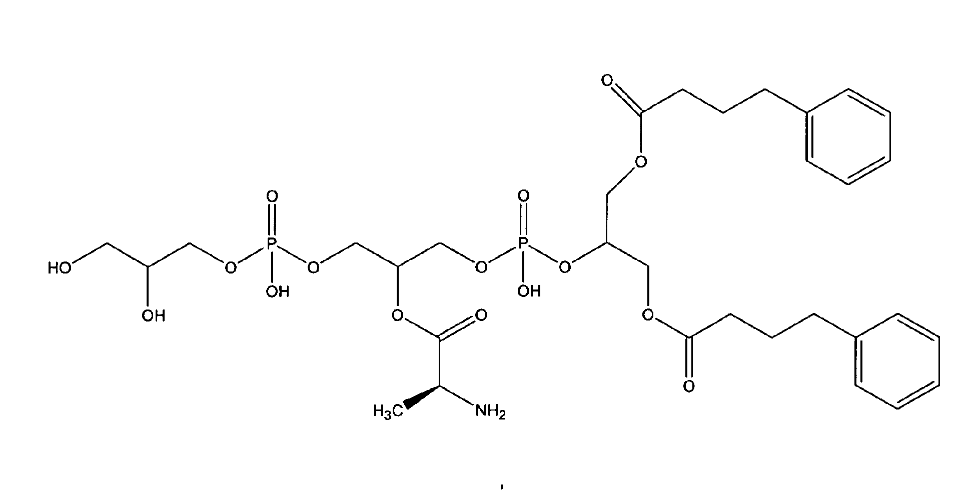 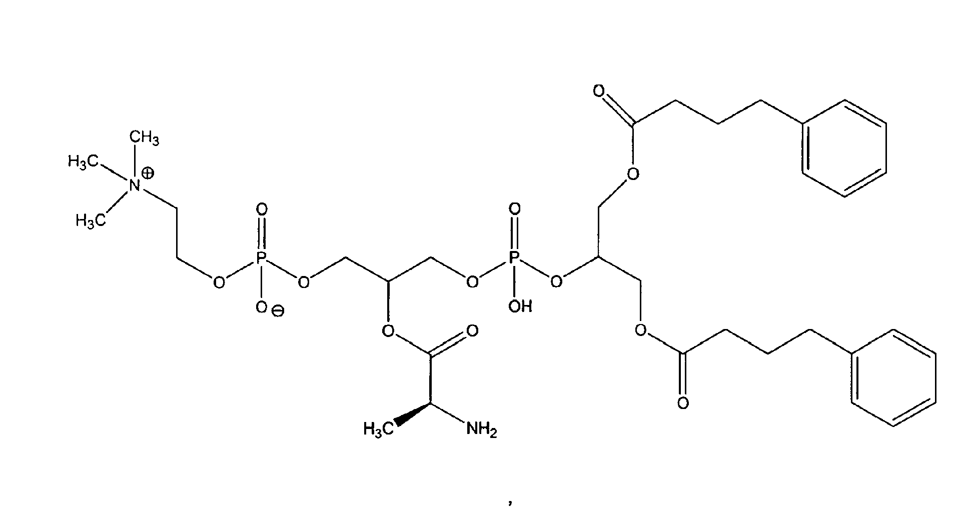 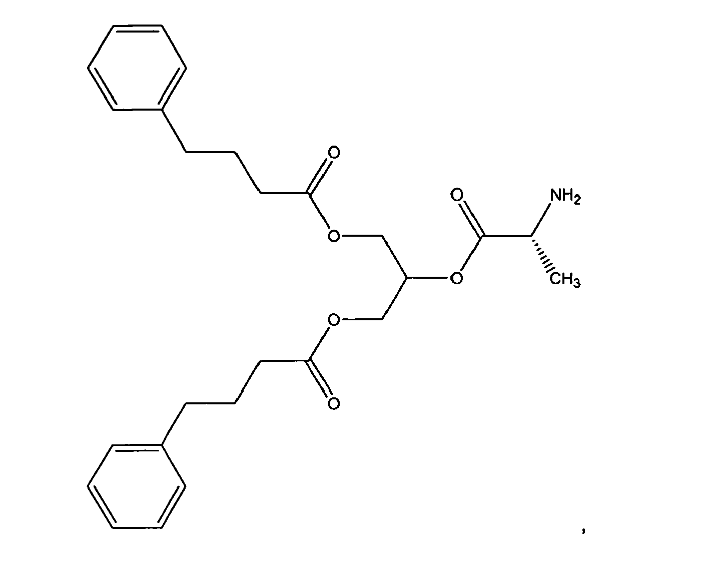 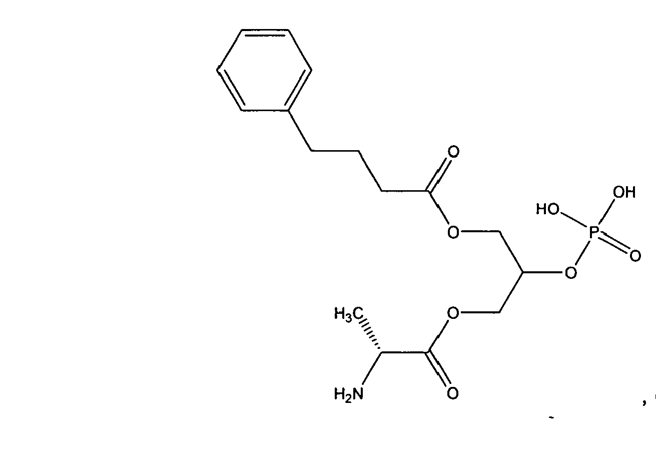 or 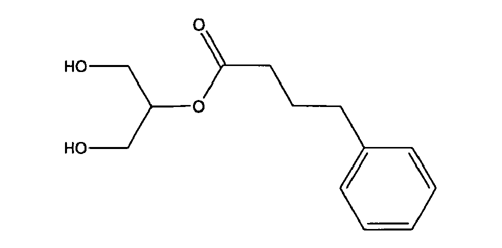 wherein the compound may optionally be substituted by one or more of F, Cl, Br, I, NH₂, OH, SH, CH₃, C₂H₅, C₃H₇, C₄H₉, CH₂Cl, CHCl₂, CF₃, CHF₂, CH₂F, preferably is unsubstituted;
optionally in form of one of its stereoisomers, enantiomers or diastereomers, its racemate or in form of a mixture of at least two of its stereoisomers in any mixing ratio;
in form of the free acid, free base or salt.

13. Process for the production of a Glycerol Phenyl Butyrate Ester according to any of claims 1 to 12, wherein in a first step a compound according to formula II 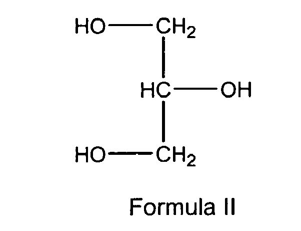 is reacted with at least one of R¹', R²', or R³' being compounds identical in structure to the substituents R¹, R² and R³ respectively as defined in any of claims 1 to 12 except for being either the free acid, or base or salt thereof, or comprising one or more leaving groups LG like a halogen, like Br or Cl, and optionally one or more protecting groups.

14. Pharmaceutical formulation comprising at least one Glycerol Phenyl Butyrate Ester according to any of claims 1 to 12 and optionally at least one physiologically acceptable excipient.

15. Glycerol Phenyl Butyrate Ester according to any of claims 1 to 12 for use in the treatment of cancer, mucositis, amyotropic lateral sclerosis (ALS), skin/mucosal itching, degenerative diseases of the eye, neurodegenerative disorders, Huntington's disease, eating disorders, obesity, drug induced weight gain, pruritis, alcoholism, promyelocytic leukemia, especially acute promyelocytic leukemia, cardiac injury, especially adriamycin induced cardiac injury, epithelial damage, connective tissue damage, desmosis, Parkinson Disease, myelodysplastic syndrome, urea cycle disorder, fibrotic lung diseases, hepatic encephalopathies, thioredoxin mediated diseases, human papilloma virus (HPV) infections, autoimmune diseases, thalassemia, genetic disorders; or in the regulation of HDAC6 activity, the modulation of stem cells, the prevention of grey hair, the promotion of neuronal growth, the prevention of bone and joint diseases, or as adjuvant in cancer therapy.
